# EUROPÄISCHE PATENTSCHRIFT

(11) **EP 0 739 898 B1**
(45) Veröffentlichungstag und Bekanntmachung des Hinweises auf die Patenterteilung: **26.09.2001**
(21) Anmeldenummer: 96103533.4
(22) Anmeldetag: 07.03.1996
(51) Int. Cl.: C07H 21/00, C07F 9/40, A61K 31/70, A61K 31/66, C12Q 1/68

(54) **Phosphonomonoesternnukleinsäuren, Verfahren zu ihrer Herstellung und ihre Verwendung**
Phosphonomonoester nucleic acids, methods for their preparation and their use
Phosphonomonoester acides nucléiques, leur préparation et leur utilisation

(30) Priorität: 13.03.1995 DE 19508923; 24.11.1995 DE 19543865
(43) Veröffentlichungstag der Anmeldung: 30.10.1996
(73) Patentinhaber: Aventis Pharma Deutschland GmbH, 65929 Frankfurt am Main (DE)
(72) Erfinder: Peyman, Anuschirwan, Dr., D-65779 Kelkheim (DE); Uhlmann, Eugen, Dr., D-61479 Glashütten (DE); Breipohl, Gerhard, Dr., D-60529 Frankfurt (DE); Wallmeier, Holger, Dr., D-65843 Sulzbach (DE)

(56) Entgegenhaltungen:
- EP-A- 0 552 766
- WO-A-94/22864
- WO-A-94/22891
- WO-A-95/11911
- DE-A- 4 321 946
- US-A- 5 264 562

## Beschreibung

Die vorliegende Erfindung betrifft neue Oligonucleotidanaloga mit wertvollen physikalischen, biologischen und pharmakologischen Eigenschaften sowie ein Verfahren zu deren Herstellung. Ihre Anwendung bezieht sich auf die Verwendung als Inhibitoren der Genexpression (Antisense Oligonucleotide, Ribozyme, Sense Oligonucleotide und Triplex Forming Oligonucleotide), als Sonden zum Nachweis von Nucleinsäuren und als Hilfsmittel in der Molekularbiologie.

Oligonucleotide finden in wachsendem Maße Anwendung als Inhibitoren der Genexpression (J. F. Milligan, M. D. Matteucci und J. C. Martin, J. Med. Chem. 36 (1993) 1923; E. Uhlmann and A. Peyman, Chemical Reviews 90 (1990) 543; S. T. Crooke, Annu. Rev. Pharmacol. Toxicol. 32 (1992) 329).

Antisense Oligonucleotide sind Nucleinsäure-Fragmente, deren Basensequenz komplementär ist zu einer zu inhibierenden mRNA. Diese Target-mRNA kann zellulären, viralen oder sonstigen pathogenen Ursprungs sein. Als zelluläre Target-Sequenzen kommen beispielsweise die von Rezeptoren, Enzymen, Wachstumsfaktoren, Immunmodulatoren, lonenkanälen oder Onkogenen in Frage. Die Inhibition der Virus Vermehrung mit Hilfe von Antisense Oligonucleotiden wurde beispielsweise für RSV (Rous Sarcoma Virus), HSV-1 und -2 (Herpes Simplex Virus Typ I und II), HIV (Human Immunodeficiency Virus) und Influenza-Viren beschrieben. Dabei setzt man Oligonucleotide ein, die zur viralen Nucleinsäure komplementär sind.

Sense Oligonucleotide sind dagegen in ihrer Sequenz so konzipiert, daß sie beispielsweise Nucleinsäure-bindende Proteine oder Nucleinsäure-prozessierende Enzyme binden ("einfangen") und so deren biologische Aktivität inhibieren (C. Hélène and J. J. Toulmé, Biochim. Biophys. Acta 1049 (1990) 99). Als virale Targets sind hier beispielsweise die Reverse Transkriptase, DNA-Polymerase und Transaktivator-Proteine zu nennen. Triplex Forming Oligonucleotide haben im allgemeinen die DNA als Target und bilden nach Bindung an diese eine tripelhelicale Struktur aus.

Während mit Hilfe der Antisense Oligonucleotide im allgemeinen die Prozessierung (Splicing etc.) der mRNA oder deren Translation in das Protein gehemmt werden, hemmen Triplex Forming Oligonucleotide die Transkription oder Replikation der DNA (N. T. Thuong, und C. Hélène, Angew. Chem. 105 (1993) 697; Uhlmann und Peyman, Chemical Reviews 90 (1990) 543). Es ist aber auch möglich, einzelsträngige Nucleinsäuren in einer ersten Hybridisierung mit einem Antisense Oligonucleotid unter Ausbildung eines Doppelstranges zu binden, der dann in einer zweiten Hybridisierung mit einem Triplex Forming Oligonucleotid eine Triplex-Struktur ausbildet. Die Antisense und Triplex Bindungsregionen können dabei entweder in zwei separaten Oligonucleotiden oder aber in einem Oligonucleotid beherbergt sein.

Eine weitere Anwendung synthetischer Oligonucleotide sind die sogenannten Ribozyme, welche die Target-RNA infolge ihrer Ribonuclease-Aktivität zerstören (D. Castanotto, J. J. Rossi, J. O. Deshler, Critical Rev. Eukar. Gene Expr. 2 (1992) 331).

In der DNA-Diagnostik werden Nucleinsäure-Fragmente mit geeigneter Markierung als sogenannte DNA-Sonden oder DNA-Probes für die spezifische Hybridisierung an eine nachzuweisende Nucleinsäure eingesetzt. Die spezifische Ausbildung des neuen Doppelstranges wird dabei mit Hilfe der Markierung, die vorzugsweise nicht radioaktiv ist, verfolgt. Auf diese Weise lassen sich genetische, maligne, virale oder durch andere Pathogene verursachte Krankheiten nachweisen.

Für die meisten genannten Anwendungen sind Oligonucleotide in ihrer natürlich vorkommenden Form wenig oder völlig ungeeignet. Sie müssen chemisch so modifiziert werden, daß sie den speziellen Anforderungen gerecht werden. Damit Oligonucleotide in biologischen Systemen, beispielsweise zur Inhibition der Virus-Vermehrung eingesetzt werden können, müssen sie folgende Voraussetzungen erfüllen:
1. Sie müssen unter in vivo Bedingungen, also sowohl im Serum als auch intrazellulär, eine ausreichend große Stabilität aufweisen.
2. Sie müssen so beschaffen sein, das sie die Zell- und Nucleus-Membran passieren können.
3. Sie müssen unter physiologischen Bedingungen in Basen-spezifischer Weise an ihre Target-Nucleinsäure binden, um den inhibitorischen Effekt zu entfalten.

Für DNA-Sonden sind diese Voraussetzungen nicht unabdingbar; jedoch müssen diese Oligonucleotide so derivatisiert sein, daß ein Nachweis, beispielsweise mittels Fluoreszenz, Chemilumineszenz, Kolorimetrie oder spezifischer Färbung, möglich ist (Beck und Köster, Anal. Chem. 62 (1990) 2258).

Es sind eine Vielzahl chemischer Variationen von Oligonucleotiden bekannt, die synthetisiert wurden mit dem Ziel, die oben genannten Anforderungen besser zu erfüllen als die nicht modifizierten Oligonucleotide. Die chemische Veränderung der Oligonucleotide erfolgt meistens in der Weise, daß Phosphatrückgrat, Ribose-Einheit oder die Nucleobasen entsprechend verändert werden (Uhlmann und Peyman, Chemical Review 90 (1990) 543). Unter den Modifikationen finden sich auch solche, in denen sowohl die Phosphat-Brücke, wie auch die Zuckereinheit durch andere Gruppierungen ersetzt wurden, beispielsweise durch "Morpholinonucleosid"-Oligomere (E. P. Stirchak et al., Nucleic Acids Res. 17 (1989) 6129) oder "PNAs" (P. E. Nielsen et al, Bioconj. Chem. 5 (1994) 3). Insbesondere PNA's zeichnen sich durch ungewöhnlich hohe Affinitäten zur Target-RNA aus, leiden aber an anderen ungünstigen Eigenschaften wie mangelnde Löslichkeit oder mangelnde Zellpenetration (W. Wang et al., Tetrahedron Letters 36 (1995) 1181; M. Egholm et al., in "Innovation and Perspectives in Solid Phase Synthesis, Peptides, Proteins, Nucleic Acids", Roger Epton, Ed. Mayflower Worldwide Limited, Birminghan, 1994, 145-148).

WO-A-94 22864 ,beschreibt Antisense Oligonucleotide, die durch eine 2-Methyl-1,3-dihydroxypropyl Gruppe modifiziert sind. Die 2-Methyl-Gruppe ist durch Q-X substituiert, worin X für eine O, S oder NR6-Gruppe und Q für eine Oligonucleotid-Base stehen. Die Oligonucleotide werden aus einem R1-OCH2CR2(CH2-X-Q)-(CH2)nOR3-Zwischenprodukt hergestellt (worin R1 für H oder eine Schutzgruppe wie Trityl, R3 für H oder P(R4)OR5, R4 für eine Amin-Gruppe und R5 vorzugsweise für 2-Cyanoethyl stehen).

Aufgabe ist es daher, neue Oligonucleotid-Analoga mit günstigen Eigenschaften zu finden.

Gegenstand der Erfindung sind daher Verbindungen der Formel I 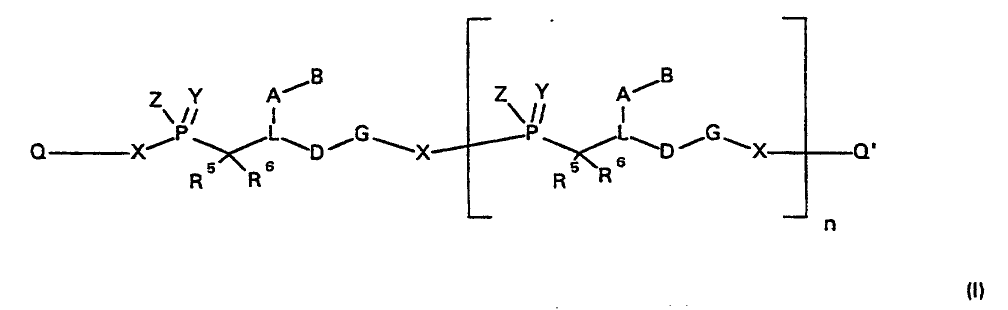 worin
- n: eine Zahl von Null bis 100 bedeutet;
- B: unabhängig voneinander Wasserstoff, Hydroxy, (C₁-C₂₀)-Alkyl, (C₁-C₂₀)-Alkoxy, (C₁-C₂₀)-Alkylthio, (C₆-C₂₀)-Aryl, (C₆-C₂₀)-Aryl-(C₁-C₆)-alkyl, (C₆-C₂₀)-Aryl-(C₁-C₆)-alkoxy, (C₆-C₂₀)-Aryl-(C₁-C₆)-alkylthio, eine aromatische Gruppe oder eine heterocyclische Gruppe bedeutet, wobei Alkyl, Aryl und/oder die aromatische oder heterocyclische Gruppe gegebenenfalls ein oder mehrfach durch Hydroxy, (C₁-C₄)-Alkoxy,-NR⁹R¹⁰, -C(O)OH, Oxo, -C(O)OR⁸, -C(O)NR⁹R¹⁰, -CN, -F, -Cl, -Br, -NO₂, (C₂-C₆)-Alkoxyalkyl, -S(O)ₘR⁸, -(C₁-C₆)-Alkyl-S(O)ₘR⁸, -NHC(=NH)NHR⁸, -C(=NH)NHR⁸, -NR⁹C(=O)R⁸, =NOR⁸, NR⁹C(=O)OR¹⁰,-OC(=O)NR⁹R¹⁰ und -NR⁹C(=O)NR⁹R¹⁰ substituiert sein können, oder
- B: unabhängig voneinander für eine natürliche Nucleobase, eine unnatürliche Nucleobase oder einen Reporter Liganden steht;
- A-B: kann auch für eine über die Carboxylgruppe aufkondensierte D- oder L-Aminosäure oder für Peptide bestehend aus diesen Aminosäuren mit bis zu einer Länge von 5 Aminosäureresten stehen,
- L: unabhängig voneinander N oder R¹N⁺, und
- **R**^{**1**}: für Wasserstoff oder (C₁-C₆)-Alkyl steht, das mit Hydroxy, (C₁-C₆)-Alkoxy, (C₁-C₆)-Alkylthio oder Amino substituiert sein kann, bevorzugt Wasserstoff oder Methyl bedeutet;
- A: unabhängig voneinander eine Einfachbindung, eine Methylengruppe oder eine Gruppe der Formel IIa oder IIb bedeutet; 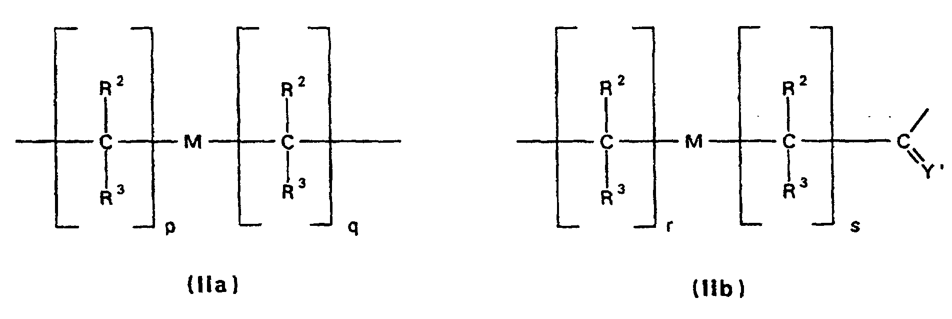
- Y': für =O, =S, =CH₂, =C(CH₃)₂ oder =NR¹ steht, wobei R¹ wie oben definiert ist;
- M: für eine Einfachbindung, -O-, -S- oder -NR¹- steht, wobei R¹ wie oben definiert ist;
- R² und R³: unabhängig voneinander für Wasserstoff, Hydroxy, (C₁-C₆)-Alkoxy, (C₁-C₆)-Alkylthio, Amino, Halogen, wie F, Cl, Br oder (C₁-C₆)-Alkyl steht, welches gegebenenfalls mit Hydroxy, (C₁-C₆)-Alkoxy oder (C₁-C₆)-Alkylthio substituiert sein kann, bevorzugt jedoch Wasserstoff bedeutet;
- p und q: unabhängig voneinander für Null bis 5 stehen;
- r und s: unabhängig voneinander für Null bis 5 stehen;
- D und G: unabhängig voneinander für CR⁵R⁶ stehen;
- R⁵ und R⁶: unabhängig voneinander Wasserstoff, (C₁-C₆)-Alkyl, (C₆-C₂₀)-Aryl, (C₆-C₂₀)-Aryl-(C₁-C₆)-alkyl, Hydroxy, (C₁-C₆)-Alkoxy, (C₁-C₆)-Alkylthio bedeuten, und Alkyl und Aryl gegebenenfalls mit SR¹ oder NR¹R^{1'} substituiert sein kann, wobei R¹ wie oben definiert ist und R^{1'} unabhängig von R¹ die gleiche Bedeutung wie R¹ hat, R⁵ und R⁶ jedoch bevorzugt Wasserstoff bedeutet;
- X: für -O-, -S- oder -NR¹-, worin R¹ wie oben definiert ist, steht;
- Y: für =O oder =S steht;
- z: für -OR⁸, -NR⁹R¹⁰ oder X'Q" steht, wobei X' wie X und Q" wie Q definiert sind;
- R⁸: Wasserstoff, (C₁-C₁₈)-Alkyl, (C₂-C₁₈)-Alkenyl, (C₃-C₁₈)-Alkinyl, (C₆-C₁₂)-Aryl, (C₆-C₁₂)-Aryl-(C₁-C₆)-alkyl bedeutet, wobei Alkyl ein oder mehrfach mit Hydroxy, (C₁-C₄)-Alkoxy, F, Cl, Br substituiert sein kann und Aryl 13-fach mit Hydroxy, (C₁-C₄)-Alkoxy, (C₁-C₄)-Alkyl, F, Cl, Br, NO₂,NR⁹R¹⁰, -C(O)OH, -C(O)O-(C₁-C₆)-Alkyl, -C(O)NR⁹R¹⁰, substituiert sein kann, bevorzugt jedoch für Wasserstoff, (C₁-C₆)-Alkyl, (C₆-C₁₂)-Aryl oder (C₆-C₁₂)-Aryl-(C₁-C₆)-alkyl steht, wobei Aryl einfach mit (C₁-C₄)-Alkoxy, (C₁-C₄)-Alkyl, F, Cl, Br, NO₂, substituiert sein kann, besonders bevorzugt Wasserstoff, (C₁-C₆)-Alkyl, Phenyl oder 2-(4-Nitrophenyl)ethyl bedeutet;
- R⁹ und R¹⁰: unabhängig voneinander für Wasserstoff, (C₁-C₁₈)-Alkyl, (C₁-C₁₈)Alkenyl, (C₁-C₁₈)-Alkinyl, (C₆-C₁₂)-Aryl, (C₆-C₁₂)-Aryl-(C₁-C₆)-alkyl stehen, wobei Alkyl ein oder mehrfach mit Hydroxy, (C₁-C₄)-Alkoxy, F, Cl, Br substituiert sein kann, oder R⁹ und R¹⁰ können zusammen mit dem sie tragenden N-Atom einen 4-7-gliedrigen Ring bilden;
- Q und Q': unabhängig voneinander Wasserstoff oder R⁸ bedeuten, für Konjugate stehen, welche die Eigenschaften von Antisense-Oligonucleotiden oder von Tripelhelix bildenden Oligonucleotiden günstig beeinflußen oder als Markierung einer DNA Sonde dienen oder bei der Hybridisierung des Oligonucleotidanalogons an die Target-Nucleinsäure diese unter Bindung oder Quervernetzung angreift, oder Oligonucleotide bedeuten, die unmodifiziert oder modifiziert sein können, wobei folgende Varianten beispielhaft für einige Modifikationen stehen sollen (z.B. beschrieben in E. Uhlmann and A. Peyman, Chemical Reviews 90 (1990) 543; "Protocols for Oligonucleotides and Analogs", Synthesis and Properties & Synthesis and Analytical Techniques, S. Agrawal, Ed, Humana Press, Totowa, USA 1993):

a) vollständiger oder teilweiser Ersatz der 3'- und/oder der 5'Phosphorsäurediesterbrücken, beispielsweise durch Phosphorothioat-, Phoshorodithioat-, NR⁴R^{4'-} Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester, 2,2,2-Trichlorodimethylethylphosphonat-, (C₁-C₈)Alkylphosphonat- oder (C₆-C₁₂)-Arylphosphonat-Brücken, wobei R⁴ und R^{4'} unabhängig voneinander für Wasserstoff, (C₁-C₁₈)-Alkyl, (C₆-C₂₀)-Aryl, (C₆-C₁₄)-Aryl-(C₁-C₈)-alkyl oder -(CH₂)_{c}-[NH(CH₂)_{c}]_{d}-NR⁷R⁷ steht, worin c eine ganze Zahl von 2 bis 6 und d eine ganze Zahl von 0 bis 6 ist, und R⁷ unabhängig voneinander Wasserstoff, (C₁-C₆)-Alkyl oder (C₁-C₄)-Alkoxy-(C₁-C₆)-alkyl ist, bevorzugt R⁴ und R^{4'} für Wasserstoff, (C₁-C₈)-Alkyl oder Methoxyethyl, besonders bevorzugt für Wasserstoff, (C₁-C₄)-Alkyl oder Methoxyethyl steht oder R⁴ und R^{4'} können auch zusammen mit dem sie tragenden Stickstoffatom einen 5-6-gliedrigen heterocyclischen Ring bilden, der zusätzlich ein weiteres Heteroatom aus der Reihe O, S, N enthalten kann;
b) vollständiger oder teilweiser Ersatz der 3'- oder 5'-Phosphorsäurediesterbrücken durch "Dephospho"-Brücken (s. z.B. Uhlmann und Peyman in "Methods in Molecular Biology", Vol. 20, "Protocols for Oligonucleotides and Analogs", S. Agrawal, Ed., Humana Press, Totowa 1993, Chapter 16, 355ff), beispielsweise durch Formacetal, 3'-Thioformacetal, Methylhydroxylamin, Oxim, Methylendimethylhydrazo, Dimethylensulfon oder Silylgruppen;
c) vollständiger oder teilweiser Ersatz des Zuckerphosphat-Rückgrats, beispielsweise durch "Morpholinonucleosid"-Oligomere (E. P. Stirchak et al., Nucleic Acids Res. 17 (1989) 6129) oder "PNAs" (P. E. Nielsen et al, Bioconj. Chem. 5 (1994) 3), oder PNA-DNA-Hybride wie beispielsweise in DE-P 44 08 528.1 bzw. EP-A 0 672 677 (HOE 94/F 057) beschrieben;
d) vollständiger oder teilweiser Ersatz der β-D-2'-Desoxyriboseeinheiten, beispielsweise durch α-D-2'-Desoxyribose, L-2'-Desoxyribose, 2'-F-2'-Desoxyribose, 2'-O-(C₁-C₆)Alkyl-Ribose, 2'-O-(C₂-C₆)Alkenyl-Ribose, 2'-NH₂-2'-desoxyribose, β-D-Xylofuranose, α-Arabinofuranose, 2,4-Dideoxy-β-D-erythrohexo-pyranose, und carbocyclische (z.B. Froehler, J.Am.Chem.Soc. 114 (1992) 8320) und offenkettige Zuckeranaloga (z.B. Vandendriessche et al., Tetrahedron 49 (1993) 7223) oder Bicyclo-Zuckeranaloga (z.B. M. Tarkov et al., Helv. Chim. Acta 76 (1993) 481);
e) vollständiger oder teilweiser Ersatz der natürlichen Nucleosid-Basen, beispielsweise durch 5-(Hydroxymethyl)uracil, 5-Aminouracil, Pseudouracil, Dihydrouracil, 5-(C₁-C₆)-Alkyl-uracil, 5-(C₂-C₆)-Alkenyl-uracil, 5-(C₂-C₆)-Alkinyluracil (beispielsweise beschrieben in Gutierrez et al., J. Am. Chem. Soc. 116 (1994) 540 oder Sagi et al., Tetrahedron Lett. 34 (1993) 2191), 5-(C₁-C₆)-Alkyl-cytosin, 5-(C₂-C₆)-Alkenyl-cytosin, 5-(C₂-C₆)-Alkinyl-cytosin (Gutierrez et al., J. Am. Chem. Soc. 116 (1994) 540 oder Sagi et al., Tetrahedron Lett. 34 (1993) 2191), 5-Fluoruracil, 5-Fluorcytosin, 5-Chloruracil, 5-Chlorcytosin, 5-Bromuracil, 5-Bromcytosin oder 7-Deaza-7-substituierte Purine (beispielsweise beschrieben in Seela, Nucl. Acids Res. 20 (1992) 2297; Heterocycles 34 (1992) 229).
Q und Q' können auch für Konjugate stehen, welche die Eigenschaften von Antisense-Oligonucleotiden oder von Tripelhelix bildenden Oligonucleotiden (wie beispielsweise Zellpenetration, Nucleaseabbau, Affinität zur Target-RNA/DNA, Pharmakokinetik) günstig beeinflußen oder als Markierung einer DNA Sonde dienen oder bei der Hybridisierung des Oligonucleotidanalogons an die Target-Nucleinsäure diese unter Bindung oder Quervernetzung angreift. Beispiele dafür sind Konjugate mit Poly-Lysin, mit Interkalatoren wie Pyren, Acridin, Phenazin, Phenanthridin, mit fluoreszierenden Verbindungen wie Fluorescein, mit Cross-Linkern wie Psoralen, Azidoproflavin, mit lipophilen Molekülen wie (C₁₂-C₂₀)-Alkyl, mit Lipiden wie 1,2-Di-hexadecyl-rac-glycerin, mit Steroiden wie Cholesterin oder Testosteron, mit Vitaminen wie Vitamin E, mit Poly- bzw. Oligo-ethylengylcol, mit (C₁₂-C₁₈)-Alkyl-Phosphatdiestern, mit -O-CH₂-CH(OH)-O-(C₁₂-C₁₈)-Alkyl. Bevorzugt sind Konjugate mit lipophilen Molekülen wie (C₁₂-C₂₀)-Alkyl, mit Steroiden wie Cholesterin oder Testosteron, mit Poly- oder Oligoethylenglykol, mit Vitamin E, mit Interkalatoren wie Pyren, mit (C₁₄-C₁₈)-Alkyl-Phosphatdiestern, mit -O-CH₂-CH(OH)-O-(C₁₂-C₁₆)-Alkyl. Die Darstellung solcher Oligonucleotid-Konjugate ist dem Fachmann bekannt (s. z.B. Uhlmann & Peyman, Chem. Rev. 90 (1990) 543; M. Manoharan in "Antisense Research and Applications", Crooke and Lebleu, Eds., CRC Press, Boca Raton, 1993, Chapter 17, S.303ff. und EP-A 0 552 766). Weiterhin können die Oligonucleotide am 3' oder am 5'-Ende 3'-3'- und 5'-5'-Inversionen (beschrieben beispielsweise in M. Koga et al., J. Org. Chem. 56 (1991) 3757) tragen.

Aromatische Gruppen sind beispielsweise Phenyl, Naphthyl, Pyrenyl, Anthracenyl, Phenanthryl, Biphenyl, Binaphtyl, Tetracenyl, Pentacenyl, Hexacenyl, Triphenylenyl, Chrysenyl oder Benzopyrenyl.

Unter heterocyclische Gruppen sind beispielsweise Chromanyl, Chromenylium-1-yl, Furanyl, Isochromanyl, Isochromenyl, Isoquinolyl, Piperazinyl, Quinolinyl, Pyridinyl, Pyrrolidinyl, Imidazyl, Tetrahydrofuranyl, Aziranyl, Oxiranyl, Thiophenyl, Pyrimidinyl, Thielanyl, Thiazolyl, Azepinyl, Pyrrolyl, Tetrahydropyrrolyl, Benzofuranyl, Indolyl, Isoindolyl, Isatinyl, Dioxindolyl, Indoxylyl, Coumarinyl, Coumaronyl, Carbazolyl, Pyrazolyl, Pyrrolyl, Indazolyl, Oxazolyl, Isoxazolyl, Thiazolyl, 1,2,4-Triazolyl, 1,2,3-Triazolyl, Tetrazolyl, Pentazolyl, Piperidinyl, Pyradizinyl, Phenazinyl, Phenoxazinyl, Phenothiazinyl, Morpholinyl, Thiazinyl, Benzodiazepinyl, Purinyl, Xanthinyl, Hypoxanthinyl, Theophyllinyl, Theobrominyl, Coffeinyl, Pteridinyl, Pterinyl, Pteridinyl, Alloxazinyl und Nortropinyl zu verstehen.

Unter natürlichen Nucleobasen werden z.B. Uracil, Cytosin, 5-Methyluracil, Adenin und Guanin und unter unnatürliche Nucleobasen werden z.B. 5-Nitroindol, 5-(Hydroxymethyl)-uracil, 5-Aminouracil, Pseudouracil, Dihydrouracil, 5-(C₁-C₆)-Alkyl-uracil, 5-(C₂-C₆)-Alkenyl-uracil, 5-(C₃-C₆)-Alkinyluracil, 5-(C₁-C₆)-Alkyl-cytosin, 5-(C₂-C₆)-Alkenyl-cytosin, 5-(C₃-C₆)-Alkinyl-cytosin, 5-Fluoruracil, 5-Fluorcytosin, 5-Chloruracil, 5-Chlorcytosin, 5-Bromuracil, 5-Bromcytosin und 7-Deaza-7-substituierte Purine, wie 7-Deaza-7-(C₃-C₇)-alkinylguanin, 7-Deaza-7-(C₃-C₇)-alkinyladenin, 7-Deaza-7-(C₂-C₇)-alkenylguanin, 7-Deaza-7-(C₂-C₇)-alkenyladenin, 7-Deaza-7-(C₁₋C₇)-alkylguanin, 7-Deaza-7-(C₁-C₇)-alkyladenin, 7-Deaza-7-bromguanin und 7-Deaza-7-bromadenin verstanden.

Bevorzugt stehen unnatürliche Nucleobasen für 5-(C₁-C₆)-Alkyl-uracil, 5-(C₂-C₆)-Alkenyl-uracil, 5-(C₃-C₆)-Alkinyl-uracil, 5-(C₁-C₆)-Alkyl-cytosin, 5-(C₂-C₆)-Alkenyl-cytosin, 5-(C₃-C₆)-Alkinyl-cytosin, 5-Fluoruracil, 5-Fluorcytosin, 5-Chloruracil, 5-Chlorcytosin, 5-Bromuracil, 5-Bromcytosin, oder 7-Deaza-7-substituierte Purine wie 7-Deaza-7-(C₃-C₇)-alkinylguanin, 7-Deaza-7-(C₃-C₇)-alkinyladenin, 7-Deaza-7-(C₂-C₇)-alkenylguanin, 7-Deaza-7-(C₂-C₇)-alkenyladenin, 7-Deaza-7-(C₁-C₇)-alkylguanin, 7-Deaza-7-(C₁-C₇)-alkyladenin, 7-Deaza-7-bromguanin und 7-Deaza-7-bromadenin, besonders bevorzugt für 5-(C₃-C₆)-Alkyl-uracil, 5-(C₂-C₆)-Alkenyl-uracil, 5-(C₃-C₆)-Alkinyl-uracil, 5-(C₁-C₆)-Alkyl-cytosin, 5-(C₂-C₆)-Alkenyl-cytosin, 5-(C₃-C₆)-Alkinyl-cytosin oder 7-Deaza-7-substituierte Purine, und ganz besonders bevorzugt für 5-Pentinylcytosin, 5-Hexinyluracil, 5-Hexinylcytosin, 7-Deaza-7-propinylguanin, 7-Deaza-7-propinyladenin, 7-Deaza-7-methylguanin, 7-Deaza-7-methyladenin, 7-Deaza-7-propinyladenin, 7-Deaza-7-bromguanin, 7-Deaza-7-bromadenin. Reporter Liganden sind beispielsweise Fluorescein, Biotin, Acridin, Phenanthrolin, Phenanthridin und Eosin.

Unter D- oder L-Aminosäuren seien, falls nicht anders angegeben, besonders folgende genannt (vgl. Schröder, Lübke, Peptides, Band 1, New York 1965, Seiten XXII-XXIII; Houben-Weyl, Methoden der Organischen Chemie, Band XV/1 und 2, Stuttgart 1974):
Aad, Abu, yAbu, ABz, 2ABz, eAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, Cyta, Daad, Dab, Dadd, Dap, Dapm, Dasu, Djen, Dpa, Dtc, Fel, GIn, Glu, Gly, Guv, hAla, hArg, hCys, hGln, hGlu, His, hlle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, hArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val etc, deren Abkürzung ohne einen Stereodeskriptor für den Rest in der L-Form steht,
oder auch cyclische Aminosäuren, wie z.B.
Pyrrolidin-2-carbonsäure; Piperidin-2-carbonsäure; 1,2,3,4-Tetrahydroisochinolin-3-carbonsäure;
Decahydroisochinolin-3-carbonsäure;
Octahydroindol-2-carbonsäure; Decahydrochinolin-2-carbonsäure;
Octahydrocyclopenta[b]pyrrol-2-carbonsäure;
2-Azabicyclo[2.2.2]octan-3-carbonsäure;
2-Azabicyclo[2.2.1]heptan-3-carbonsäure;
2-Azabicyclo[3.1.0]hexan-3-carbonsäure;
2-Azaspiro[4.4]nonan-3-carbonsäure;
2-Azaspiro[4.5]decan-3-carbonsäure;
Spiro[(bicyclo[2.2.1]heptan)-2,3-pyrrolidin-5-carbonsäurel;
Spiro[(bicyclo[2.2.2]octan)-2,3-pyrrolidin-5-carbonsäure];
2-Azatricyclo[4.3.0.1^{6,9}]decan-3-carbonsäure;
Decahydrocyclohepta[b]pyrrol-2-carbonsäure;
Decahydrocycloocta[b]pyrrol-2-carbonsäure;
Octahydrocyclopenta[c]pyrrol-2-carbonsäure;
Octahydroisoindol-1-carbonsäure;
2,3,3a,4,6a-Hexahydrocyclopenta[b]pyrrol-2-carbonsäure;
2,3,3a,4,5,7a-Hexahydroindol-2-carbonsäure;
Tetrahydrothiazol-4-carbonsäure;
Isoxazolidin-3-carbonsäure; Pyrazolidin-3-carbonsäure;
Hydroxypyrrolidin-2-carbonsäure; die alle gegebenenfalls substituiert sein können:

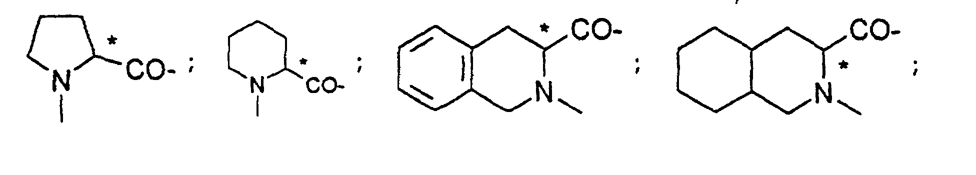

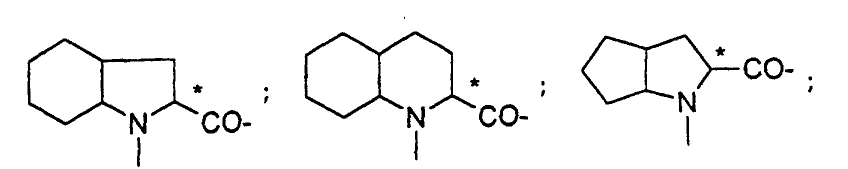

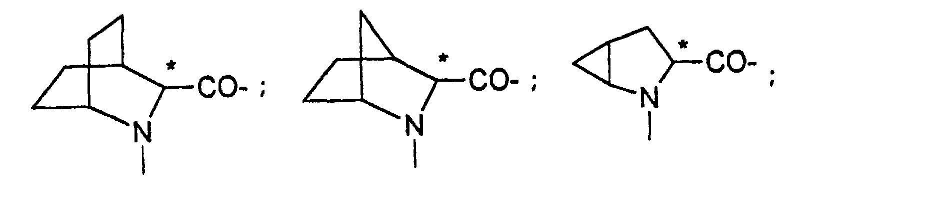

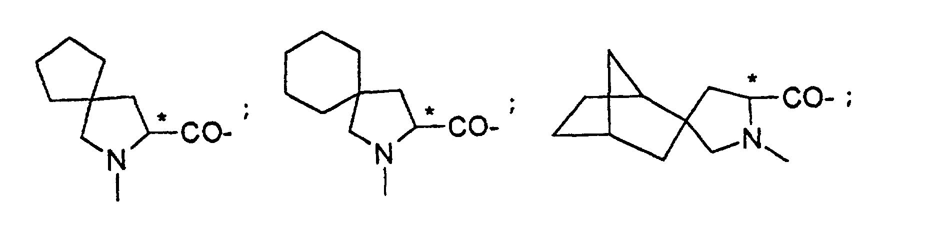

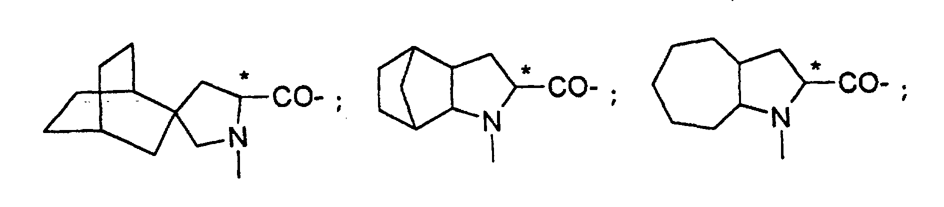

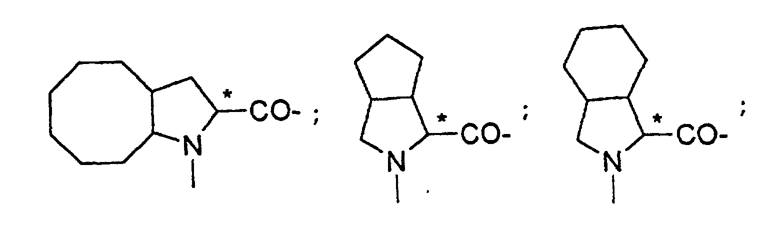

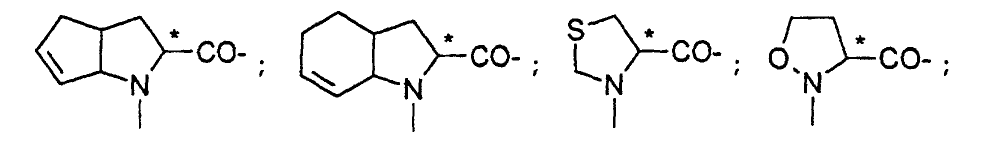

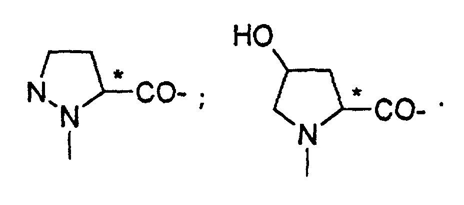

US-A 4,344,949, US-A 4,374,847, US-A 4,350,704, EP-A 29 488, EP-A 31 741, EP-A 46 953, EP-A 49 605, EP-A 49 658, EP-A 50 800, EP-A 51 020, EP-A 52 870, EP-A 79 022, EP-A 84 164, EP-A 89 637, EP-A 90 341, EP-A 90 362, EP-A 105 102, EP-A 109 020, EP-A 111 873, EP-A 271 865 und EP-A 344 682.

Alkyl und davon abgeleitete Reste wie beispielsweise Alkoxy und Alkylthio können verzweigt, unverzweigt oder cyclisch, gesättigt oder ein oder mehrfach ungesättigt sein.

Bevorzugt sind Verbindungen der Formel I, worin
- n: eine Zahl von Null bis 50 bedeutet;
- B: unabhängig voneinander für eine natürliche Nucleobase oder eine unnatürliche Nucleobase steht;
- L: N bedeutet;
- A: eine Gruppe der Formel IIb bedeutet, worin r = 1 und s Null, und R², R³ = H und Y' =O und M eine Einfachbindung bedeuten;
- D und G: unabhängig voneinander CHR⁵ bedeuten;
- R⁵: für Wasserstoff steht;
- x: -O- bedeutet;
- Y: =O bedeutet;
- Z: für Hydroxy, Methoxy, Ethoxy, (4-Nitrophenol)ethoxy, Propoxy, isoPropoxy, Butoxy, Pentoxy, Phenoxy oder Allyloxy steht;
- Q und Q': unabhängig voneinander für Wasserstoff, R⁸ oder Oligonucleotide stehen, die unmodifiziert und modifiziert sein können, wobei

a) die 3'- und/oder 5'-Phosphorsäurediesterbrücken vollständig cder teilweise durch Phosphorothioat-, Phoshorodithioat-, NR⁴R^{4'-}Phosphoramidat-, N3'-P5'-Phosphoramidat (beispielsweise beschrieben in Gryaznov et al., J. Am. Chem. Soc. 116 (1994) 3143), Phosphat-O-Methylester-, Phosphat-O-ethylester-, Phosphat-O-isopropylester-, Methylphosphonat- oder Phenylphosphonat-Brücken ersetzt sind;
b) ein, zwei oder drei 3'- oder 5-Phosphorsäurediesterbrücken an den Pyrimidin-Positionen und am 5'-Ende und/oder am 3'-Ende durch Formacetale und/oder 3'-Thioformacetale ersetzt sind;
c) das Zuckerphosphat-Rückgrats vollständiger oder teilweise durch "PNAs" oder PNA-DNA-Hybride ersetzt ist;
d) die β-D-2'-Desoxyriboseeinheiten vollständig oder teilweise durch 2'-F-2'-Desoxyribose, 2'-O-(C₁-C₆)Alkyl-Ribose, 2'-O-(C₂-C₆)Alkenyl-Ribose, 2'-NH₂-2'-desoxyribose ersetzt sind;
e) die natürlichen Nucleosid-Basen vollständig oder teilweise durch 5-(C₁-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5-Bromcytosin, 7-Deaza-7-(C₂-C₇)-alkinylguanin, 7-Deaza-7-(C₂-C₇)-alkinyladenin, 7-Deaza-7-(C₂-C₇)-alkenylguanin, 7-Deaza-7-(C₂-C₇)-alkenyladenin, 7-Deaza-7-(C₁₋C₇)-alkylguanin, 7-Deaza-7-(C₁-C₇)-alkyladenin, 7-Deaza-7-bromguanin, 7-Deaza-7-bromadenin ersetzt sind.

Besonders bevorzugt sind Verbindungen der Formel I, worin
- n: eine Zahl von 0 bis 30 bedeutet;
- Q und Q': unabhängig voneinander für Wasserstoff, R⁸, worin R⁸ für H, (C₁-C6)- Alkyl, Phenyl oder 2-(4-Nitrophenylethyl) steht, oder für Oligonucleotide stehen, die unmodifiziert und modifiziert sein können, wobei

a) die 3'- und/oder 5'-Phosphorsäurediesterbrücken vollständig oder teilweise durch Phosphorothioat-, Phosphorodithioat- oder Methylphosphonat-Brücken ersetzt sind;
b) ein, zwei oder drei 3'- oder 5-Phosphorsäurediesterbrücken am 5'- und am 3'-Ende ersetzt sind;
c) das Zuckerphosphat-Rückgrats vollständiger oder teilweise durch "PNAs" oder PNA-DNA-Hybride ersetzt ist;
d) die β-D-2'-Desoxyriboseeinheiten vollständig oder teilweise durch 2'-F-2'-Desoxyribose, 2'-O-(C₁-C₄)Alkyl-Ribose, 2'-O-(C₂-C₄)Alkenyl-Ribose, 2'-NH₂-2'-desoxyribose ersetzt sind;
e) die natürlichen Nucleosid-Basen vollständig oder teilweise durch 5-(C₃-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 7-Deaza-7-(C₂-C₇)-alkinylguanin, 7-Deaza-7-(C₂-C₇)-alkinyladenin, 7-Deaza-7-(C₂-C₇)-alkenylguanin, 7-Deaza-7-(C₂-C₇)-alkenyladenin, 7-Deaza-7-(C₁₋C₇)-alkylguanin, 7-Deaza-7-(C₁-C₇)-alkyladenin, 7-Deaza-7-bromguanin, 7-Deaza-7-bromadenin ersetzt sind.

Ganz besonders bevorzugt sind Verbindungen der Formel I, worin
- n: eine Zahl von 0 bis 25 bedeutet;
- B: unabhängig voneinander für eine natürliche Nucleobase steht;
- Z: für Hydroxy, Ethoxy, (4-Nitrophenol)ethoxy oder Phenoxy steht;
- Q und Q': unabhängig voneinander für Wasserstoff, R⁸, worin R⁸ für H, (C₁-C₆)- Alkyl, Phenyl oder 2-(4-Nitrophenylethyl) steht, oder für Oligonucleotide stehen, die unmodifiziert und modifiziert sein können, wobei

a) die 3'- und/oder 5'-Phosphorsäurediesterbrücken vollständig oder teilweise durch Phosphorothioat-Brücken ersetzt sind;
c) das Zuckerphosphat-Rückgrats vollständiger oder teilweise durch "PNAs" oder PNA-DNA-Hybride ersetzt ist;
d) die β-D-2'-Desoxyriboseeinheiten vollständig oder teilweise durch 2'-O-Methyl-, 2'-O-Allyl-, 2'-O-Butylribose ersetzt sind;
e) die natürlichen Nucleosid-Basen vollständig oder teilweise durch 5-Hexinylcytosin, 5-Hexinyluracil, 5-Hexinylcytosin, 7-Deaza-7-propinylguanin, 7-Deaza-7-propinyladenin, 7-Deaza-7-methylguanin, 7-Deaza-7-methyladenin, Deaza-7-bromguanin, 7-Deaza-7-bromadenin ersetzt sind.

Ein weiterer Gegenstand der Erfindung sind Verbindungen der Formel I, in denen Q und Q' verknüpft sind, d.h. ein cyclisches Molekül bilden, wobei auch die Möglichkeit gelten soll, daß Q und Q' zusammen eine Einfachbindung ergeben. Die Synthese von solchen Verbindungen kann analog beschriebenen Verfahren erfolgen wie beispielsweise Gao et al., Nucl. Acids Res. 23 (1995) 2025 oder Wang and Kool, Nucl. Acids Res. 22 (1994) 2326.

Ein weiterer Gegenstand der Erfindung sind Oligonucleotide bzw. modifizierte Oligonucleotide, beispielsweise PNA's, in welche Verbindungen der Formel I am 3'-Ende oder am 5'-Ende oder am 5'- und am 3'-Ende eingebaut sind.

Die Verknüpfung der Oligonucleotide mit den Verbindungen der Formel I erfolgt bevorzugt über die 5'- oder 3'-Hydroxygruppe der Nucleotidbausteine, ebenfalls über eine Phosphonsäuremonoesterbindung. In den Formeln XVIII und XIX wird die Verknüpfung mit Oligonucleotiden beispielhaft verdeutlicht.

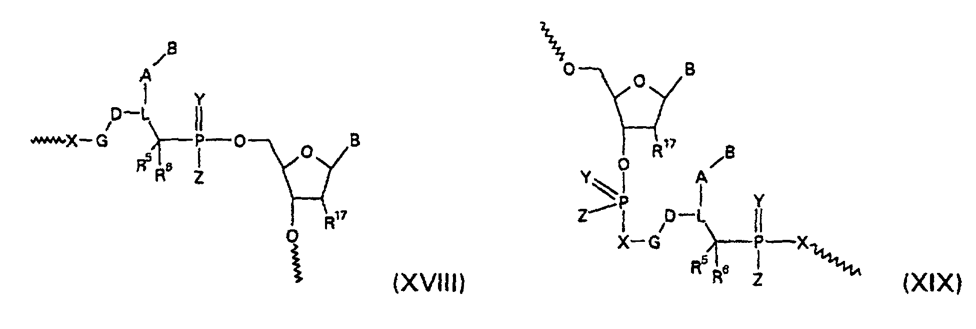

R¹⁷ steht hier für H, OH, F, 2'-O-(C₁-C₆)Alkyl, 2'-O-(C₂-C₆)Alkenyl, bevorzugt für H oder Methoxy oder O-Allyl, besonders bevorzugt für H steht. Alle anderen Variablen sind oben erläutert.

Formel XX und Formel XXI, worin die Variablen obige Bedeutung haben, verdeutlichen beispielhaft die Verknüpfung mit PNA's.

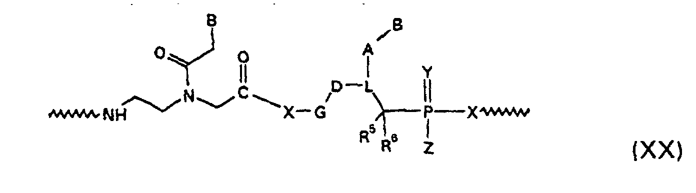

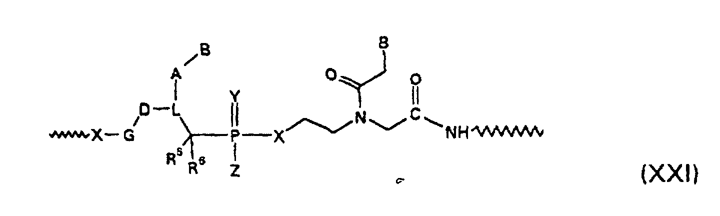

Die Kombinationen der erfindungsgemäßen Verbindungen der Formel I (abgekürzt PMENA falls Q und Q' = H) mit Oligonucleotiden oder modifizierten Oligonucleotiden, wie beispielsweise PNA's oder anderen Modifikationen, wie sie oben beschrieben sind, sollen schematisch nochmals verdeutlicht werden (OLIGO steht für unmodifizierte oder modifizierte Oligonucleotide):
Beispiele für solche Kombinationen sind:
   5'- OLIGO - PMENA
   5'- PMENA - OLIGO
   5'- OLIGO - PMENA - OLIGO
Ferner seien genannt:
   5'- OLIGO - (PMENA - OLIGO)ₐ (a = 1-20)
   5'- PMENA - OLIGO - PMENA
   5'- PMENA - (OLIGO - PMENA)ₐ (a = 1-20)

Die Synthese dieser kombinierten Verbindungen erfolgt so, daß je nach Molekül zunächst mit der Synthese der PMENA-Bausteine, die im folgenden beschrieben wird, begonnen wird, die dann mit den Oligonucleotidbausteinen gekoppelt werden. Dabei werden die Oligonucleotide nach dem Fachmann bekannten Methoden (Sonveaux, Bioorganic Chemistry 14 (1986) 274ff) durch Festphasensynthese oder durch Lösungssynthese als Monomerbausteine oder durch Blockkondensation aufgekoppelt. Die Kondensationen erfolgen wahlweise nach der Amidit-Methode, der H-Phosphonat-Methode oder dem Phosphortriester-Verfahren (Sonveaux, Bioorganic Chemistry 14 (1986) 274ff). Werden umgekehrt PMENA-Bausteine auf OLIGO-Bausteine gekoppelt, so erfolgt dies bevorzugt nach der in f₁) beschriebenen Methode. Die Konjugation mit PNA-Bausteinen erfolgt in der gleichen Weise oder, wenn (monomere oder oligomere) PNA-Bausteine auf PMENA-Bausteine aufgekoppelt werden unter den dem Fachmann bekannten Methoden der Peptid-Synthese oder der Ester-Synthese.

Gegenstand der Erfindung ist weiterhin ein Verfahren zur Herstellung von Verbindungen der Formel I, das dadurch gekennzeichnet ist, daß man
a₁) Verbindungen der Formel III 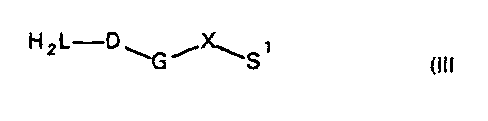 worin
   - D, G, L und X: oben genannte Bedeutungen haben und
   - S¹: für eine geeignete Schutzgruppe steht, wie beispielsweise Dimethoxytrityl, Monomethoxytrityl, Trityl, Pixyl, tert.-Butoxycarbonyl oder Fluorenylmethyloxycarbonyl, bevorzugt Monomethoxytrityl oder tert.-Butoxycarbonyl,
   mit Verbindungen der Formel IV 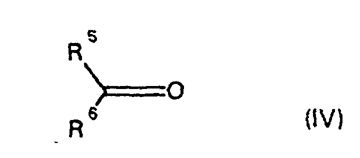 worin
   - R⁵ und R⁶: oben genannte Bedeutungen haben,
   in einem geeigneten organischen Lösungsmittel, beispielsweise in Methanol, Ethanol, iso-Propanol, Butanol, Acetonitril, Dichlormethan (DCM), Chloroform, Benzol, Dimethylformamid (DMF), Dimethylsulfoxid (DMSO), Diethylether, Essigsäureethylester (EE),Tetrahydrofuran (THF), N-Methylpyrrolidon, Petrolether, Xylol oder Toluol oder Mischungen geeigneter Lösungsmittel, bevorzugt in Methanol oder Ethanol, bei Temperaturen von 0 °C bis 100 °C , bevorzugt bei 10 bis 50 °C, umsetzt zu Verbindungen der Formel Va oder Vb 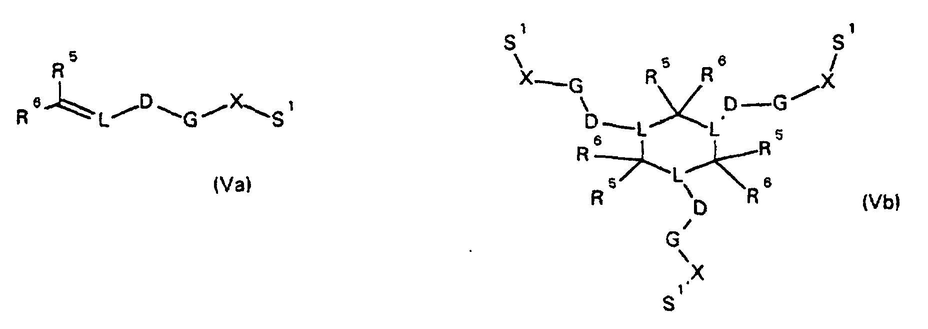 wobei bei der Wahl der Reaktionsbedingungen, die dem Fachmann bekannt sind (z.B. in S.R. Sandler, W. Karo "Organic Functional Group Preparations", Vol. II, Second Edition, Academic Press, London, 1986, Chapter 12 ("Imines")) darauf zu achten ist, daß sie mit der Schutzgruppe S¹ kompatibel sind, d.h. wird beispielsweise eine säurelabile Schutzgruppe wie die Monomethoxytritylschutzgruppe gewählt, so sollte auf Säurezusatz bei der Reaktion verzichtet werden,
b₁) Verbindungen der Formel Va oder Vb mit Verbindungen der Formel Vla oder Vlb, vorzugsweise mit Verbindungen der Formel Vla 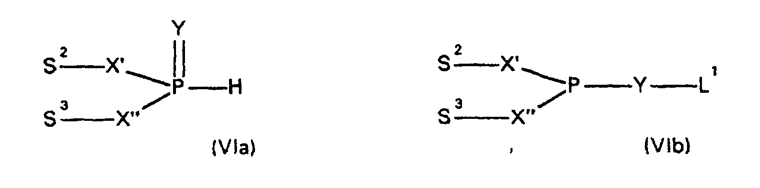 worin
   - Y: wie oben definiert ist,
   - X' und X": unabhängig voneinander wie X definiert sind,
   - S² und S³: unabhängig voneinander Schutzgruppen bedeuten, wie
   - beispielsweise: Methyl, Ethyl, Phenyl, 2-Chlorphenyl, 2,5-Dichlorphenyl, 2,4-Dichlorphenyl, 4-Nitrophenyl, 4-Methoxyphenyl, 2-Cyanoethyl, 2-(4-Nitrophenyl)ethyl, Allyl, Benzyl, 2,2,2-Trichlor-1,1-dimethylethyl, 4-Methoxybenzyl, 2,2,2-Trichlorethyl, 8-Hydroxychinnolin oder andere Phosphatschutzgruppen, wie sie dem Fachmann bekannt sind (Sonveaux, Bioorganic Chemistry 14 (1986) 274ff), bevorzugt jedoch für Methyl, Ethyl, Phenyl, 2-(4-Nitrophenyl)ethyl, Allyl, 2,2,2-Trichlorethyl stehen, und
   - L¹: für eine Abgangsgruppe, vorzugsweise für (C₁-C₄)-Alkyl, steht,
   in einem geeigneten organischen Lösungsmittel, beispielsweise in Methanol, Ethanol, iso-Propanol, Butanol, Acetonitril, Benzol, DMF, DMSO, DCM, EE, Chloroform, Diethylether, THF, N-Methylpyrrolidon, Petrolether, Xylol oder Toluol oder Mischungen geeigneter Lösungsmittel, bevorzugt in THF, bei Temperaturen von 0 °C bis 100 °C, bevorzugt bei 50 bis 80 °C, gegebenenfalls unter Zusatz von Basen, wie beispielsweise Tri-(C₁-C₆)-alkylamin, N-Alkylmorpholin, Pyridin, N,N-Dimethylaminopyridin, Butyllithium, Lithiumdiisopropylamid (LDA), Natriumhydrid, Nariumamid, Kaliumcarbonat, Caesiumcarbonat, Kalium-tert.-butylat oder komplexen Basen wie Natriumamid-R¹¹ONa, wobei R¹¹ für (C₂-C₆)-Alkyl oder CH₃CH₂-O-CH₂CH₃ steht, oder ungeladenen, peralkylierten Polyamino-Phosphazen-Basen (Schwesinger, Nachr. Chem. Techn. Lab. 38 (1990) 1214; Angew. Chem. 99 (1987) 1212), bevorzugt jedoch ohne Basenzusatz,
   umsetzt zu Verbindungen der Formel VII 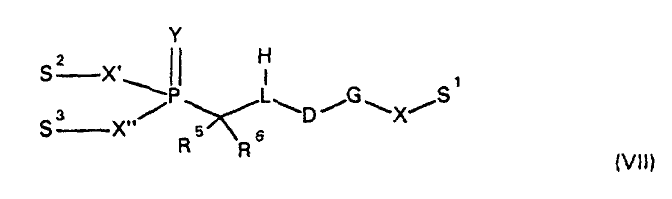 worin
   D, G, L, R⁵, R⁶, S¹, S², S³, X, X', X" und Y wie oben definiert sind;
c₁) Verbindungen der Formel VII mit Verbindungen der Formel VIII, 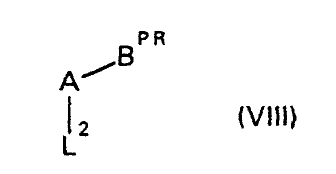 deren Synthese z.B. in Dueholm et al., J. Org. Chem. 59 (1994) 5767 beschrieben ist und
   worin
   - A: die oben genannte Bedeutung hat,
   - B^{PR}: die gleiche Bedeutung wie B hat, gegebenenfalls jedoch in geschützter Form vorliegt, d.h. falls B für eine natürliche oder unnatürliche Nucleobase steht, so steht B^{PR} für die Nucleobasen, deren Amino- bzw. Hydroxygruppen durch geeignete bekannte Schutzgruppen geschützt sind, wie beispielsweise die para-Nitrophenylethyl-Gruppe, die Benzoyl-Gruppe, die Allyl-Gruppe und die para-(t-Butyl)benzoylgruppe für die Hydroxygruppe und die Acetyl-, Benzoyl-, para-(t-Butyl)benzoyl-, para-(Methoxy)benzoyl-Gruppe, para-Nitrophenylethyloxycarbonyl-Guppe, Isobutyryl-Gruppe, para-(t-Butyl)phenylacetyl-Gruppe, N,N-Dimethylformamidino, Fluorenylmethyloxycarbonyl-Gruppe, Benzyloxycarbonyl-Gruppe, Phenoxyacetyl-Gruppe für die Aminogruppe oder andere in der Oligonucleotid-Chemie für Nucleobasen üblichen Schutzgruppen (Sonveaux, Bioorganic Chemistry 14 (1986) 274ff; Beaucage, Tetrahedron 49 (1993) 2223ff), bevorzugt für B^{PR} seien genannt: 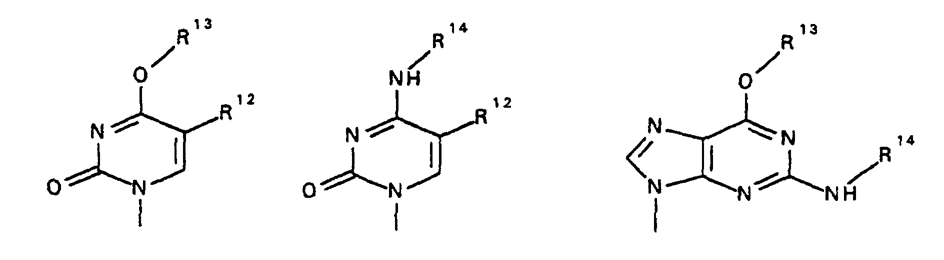 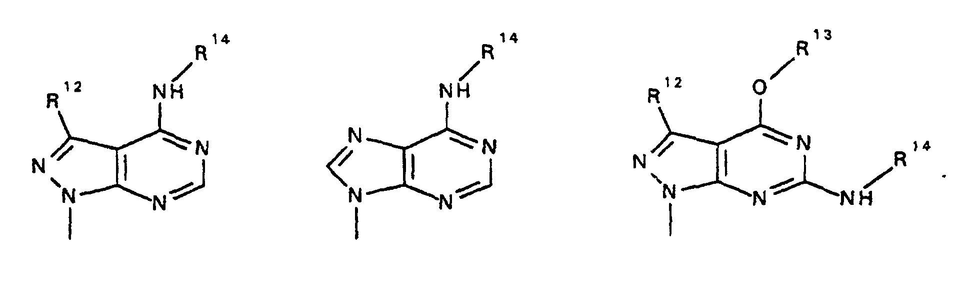 worin
   R¹² Wasserstoff, 1-Propinyl, 1-Butinyl, 1-Pentinyl oder 1-Hexinyl, insbesondere Wasserstoff, 1-Propinyl oder 1-Hexinyl; und
   R¹³ Wasserstoff, Diphenylcarbamoyl oder 2-(4-Nitrophenyl)ethyl steht und
   R¹⁴ Acetyl, Benzoyl, para-(t-Butyl)benzoyl, para-(Methoxy)benzoyl, para-Nitrophenylethyloxycarbonyl, Isobutyryl, para-(t-Butyl)phenylacetyl, Benzyloxycarbonyl oder Phenoxyacetyl bedeuten, und
   L² für eine dem Fachmann bekannte Abgangsgruppe wie beispielsweise Cl, Br, O-SO₂Methyl, O-SO₂Trifluormethyl, OTosylat, O-C₆F₅ steht oder, falls A die Bedeutung von Formel IIb hat auch für OH stehen kann;
   in einem geeigneten organischen Lösungsmittel, beispielsweise in Acetonitril, Benzol, DMF, DMSO, DCM, EE, Chloroform, Diethylether, Tetramethylharnstoff, THF, N-Methylpyrrolidon, Petrolether, Xylol, oder Toluol oder Mischungen geeigneter Lösungsmittel, bevorzugt in DMF, bei Temperaturen von -20°C bis 100 °C, bevorzugt bei 0 bis 50 °C, gegebenenfalls unter Zusatz von Basen, wie beispielsweise Tri-(C₁-C₆)-alkylamin, N-Alkylmorpholin, Pyridin, N,N-Dimethylaminopyridin, Butyllithium, Lithiumdiisopropylamid (LDA), Natriumhydrid, Natriumamid, Kaliumcarbonat, Caesiumcarbonat, Kalium-tert.-butylat oder komplexen Basen wie Natriumamid-R¹¹ONa, wobei R¹¹ für (C₂-C₆)-Alkyl oder CH₃CH₂-O-CH₂CH₃ steht oder ungeladenen, peralkylierten Polyamino-Phosphazen-Basen (Schwesinger, Nachr. Chem. Techn. Lab. 38 (1990) 1214; Angew. Chem. 99 (1987) 1212), falls A für Formel IIb und L² für OH steht, bevorzugt unter Zusatz von Triethylamin, Diisopropylethylamin oder N-Ethylmorpholin oder ohne Basenzusatz und unter Zusatz eines zur Knüpfung von Peptid-Bindungen üblichen Kopplungsreagenzes,
   umsetzt zu Verbindungen der Formel IX 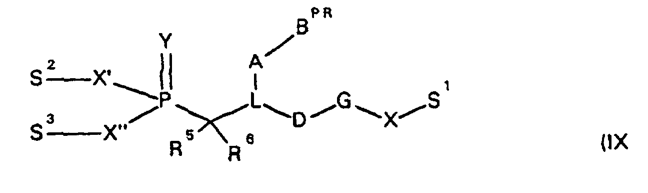 worin
   A, B^{PR}, D, G, L, R⁵, R⁶, S¹, S², S³, X, X', X" und Y wie oben definiert sind;
d₁) aus Verbindungen der Formel IX die Schutzgruppe S³ nach bekannten Verfahren (z.B. Greene, Wuts, Protective Groups in Organic Synthesis, J. Wiley & Sons, New York 1991) abspaltet, so z.B. für Verbindungen der Formel IX, in denen S² und S³ für 2-(4-Nitrophenyl)ethyl stehen durch Behandlung mit 0.1M 1,8-Diazabicyclo[5,4,0]undec-7-en (DBU) in Pyridin oder Acetonitril bei Raumtemperatur oder für Verbindungen der Formel IX, in denen S² und S³ für Phenyl oder Ethyl stehen durch Behandlung mit wässrigem Ammoniak oder für Verbindungen der Formel IX, in denen S² für 2-(4-Nitrophenyl)ethyl und S³ für Allyl steht durch Behandlung mit Pd[P(C₆H₅)₃]₄ und Triphenylphosphin in DCM (Hayakawa et al., J. Org. Chem. 58 (1993) 5551), oder für Verbindungen der Formel IX, in denen S² für 2-(4-Nitrophenyl)ethyl und S³ für Allyl steht durch Behandlung mit 0,5M DBU in Pyridin oder Acetonitril oder für Verbindungen der Formel IX in denen S² für 2-Cyanoethyl und S³ für Allyl steht durch Behandlung mit Triethylamin in Pyridin, oder für Verbindungen der Formel IX in denen S² für 2-(4-Nitrophenyl)ethyl und S³ für 2,2,2-Trichlor-1,1-dimethylethyl steht durch Behandlung mit Tributylphosphin,
   wobei man Verbindungen der Formel X erhält 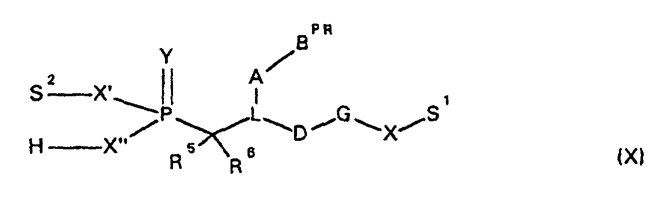 worin
   A, B^{PR}, D, G, L, R⁵, R⁶, S¹, S², X', X" und Y wie oben definiert sind;
e₁) aus Verbindungen der Formel IX die Schutzgruppe S¹ nach bekannten Verfahren (z.B. Greene, Wuts, Protective Groups in Organic Synthesis, J. Wiley & Sons, New York 1991, Sonveaux, Bioorganic Chemistry 14 (1986) 274ff) abspaltet, so wird beispielsweise die Monomethoxytritylschutzgruppe durch Behandlung mit Säure abgespalten, beispielsweise durch Behandlung mit 80% Essigsäure, mit 1-4% Dichloressigsäure in Methylenchlorid oder Chloroform, mit 2% p-Toluolsulfonsäure in DCM/Methanol oder durch Behandlung mit 1% Trifluoressigsäure in Chloroform,
   wobei man Verbindungen der Formel XI erhält 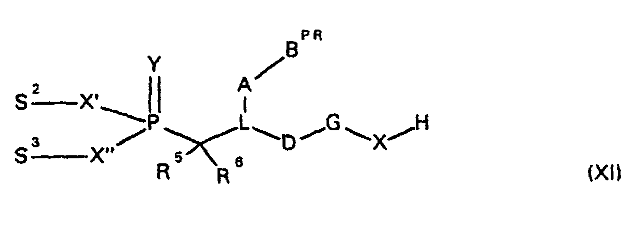 worin
   A, B^{PR}, D, G, L, R⁵, R⁶, S², S³, X, X', X" und Y wie oben definiert sind;
f₁) Verbindungen der Formel XI mit Verbindungen der Formel X gemäß dem aus der Oligonucleotid-Chemie (Sonveaux, Bioorganic Chemistry 14 (1986) 274ff, Reese, J. Chem. Soc. Perkin Trans. 1993, 2291ff) bekannten "Phosphotriester-Verfahren" in einem geeigneten organischen Lösungsmittel, wie Acetonitril, Benzol, DMF, DMSO, DCM, EE, Chloroform, Diethylether, Tetramethylharnstoff, THF, N-Methylpyrrolidon, Petrolether, Xylol oder Toluol oder Mischungen geeigneter Lösungsmittel, bevorzugt in Pyridin, bei Temperaturen von -20°C bis 100 °C, bevorzugt bei 0 bis 50 °C, unter Zusatz eines Kopplungsreagenzes, wie beispielsweise 6-Nitrobenzotriazol-1-yloxytris(dimethylamino)-phosphonium Hexafluorophosphat (NBOP, Hashmi, Nucleosides,& Nucleotides 13 (1994) 1059), Benzotriazol-1-yloxy-tris-(dimethylamino)phosphoniumhexafluorophosphat (BOP, B. Castro, J.R. Dormoy, G. Evin and C. Selve, Tetrahedron Lett. 1975, 1219-1222), Benzotriazol-1-yloxy-tripyrrolidino-phosphoniumhexafluorophosphat (PyBOP, J. Coste, D. Le-Nguyen und B. Castro, Tetrahedron Lett. 1990, 205-208), O-(7-aza)benzotriazol-1-yltetramethyluroniumhexafluorophosphat (HATU, L. Carpino, J. Am. Chem. Soc. 1993, 115, 4397), N,N-Bis[2-oxo-3-oxazolidinyl]phosphordiamidchlorid (Katti, Tetrahedron Lett. 26 (1985) 2547), 2-Chloro-5,5-dimethyl-2-oxo-1,3,2-dioxaphosphorinan (Stawinski, Nucl. Acids Res., Symp. Ser. 24, 1991, 229) oder einer Verbindung der Formel XII 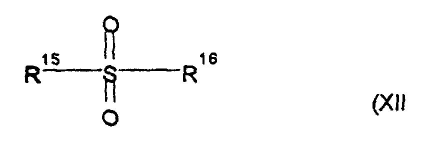 worin
   - R¹⁵: für (C₆-C₁₂)-Aryl, gegebenenfalls ein bis vierfach substituiert durch (C₁-C₆)-Alkyl, (C₁-C₆)-Alkoxy, Nitro, Chlor, Brom und wobei gegebenenfalls ein bis 3 C-Atome durch Heteroatome, bevorzugt Stickstoff substituiert sind, d.h. beispielsweise für Phenyl, Tolyl, 2,4,6-Trimethylphenyl, 2,4,6-Triisopropylphenyl, 2,3,5,6-Tetramethylbenzol (Losse, Liebigs Ann. Chem. 1989, 19ff), 4-Brombenzol, 2-Nitrobenzol, 4-Nitrobenzol, 8-Chinolyl steht, bevorzugt für 2,4,6-Trimethylphenyl oder 2,4,6-Triisopropylphenyl, und
   - R¹⁶: für eine Abgangsgruppe wie beispielsweise Chlor, Brom, Imidazol, Triazol, 4-Nitroimidazol, 1,2,3,4-Tetrazol, 3-Nitro-1,2,4-Triazol steht,
   bevorzugt unter Zusatz eines Kopplungsreagenzes der Verbindung der Formel XII oder BOP, PyBOP oder HATU,
   gegebenenfalls unter Zusatz eines Katalysators (Reese, J. Chem. Soc. Perkin Trans. 1993, 2291ff), wie beispielsweise N-Methylimidazol, Pyridin-N-oxiden wie etwa 4-Methoxy-pyridin-N-oxid oder 4-Ethoxy-pyridin-N-oxid, 4,6-Dinitro-1-hydroxybenzotriazol; 1-Hydroxy-5-phenyltetrazol, 1-Hydroxy-(5-(4-nitrophenyl)tetrazol, 3-Nitro-1H-1,2,4-triazol, 5-(3-Nitrophenyl)-1H-tetrazol, 5-(3,5-Dinitrophenyl)-1H-tetrazol, 5-(1-methylimidazol-2-yl)-1H-tetrazol, 5-[(1-methylimidazol-2-yl)methyl]-1H-tetrazol oder 1 -Hydroxy-4-nitro-6-(trifluormethyl)benzotriazol, bevorzugt mit 4-Ethoxypyridin-N-oxid oder 4-Methoxy-pyridin-N-oxid als Katalysator,
   wobei die Herstellung der Kopplungsreagenzien in situ erfolgen kann, oder aber separat erfolgen und die Lösung der aktivierten Spezies (Verbindung der Formel X + Kopplungsreagenz) in einem geeigneten Lösungsmittel zugegeben werden kann,
   zu Verbindungen der Formel XIII 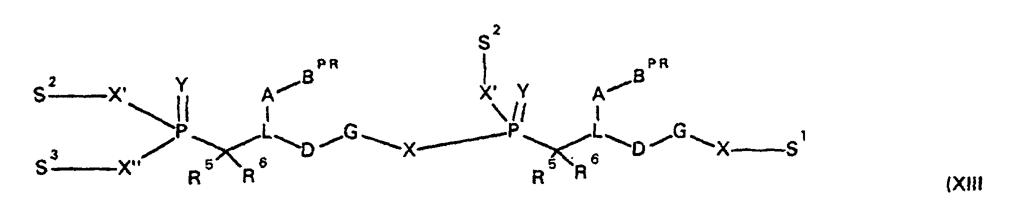 worin
   A, B^{PR}, D, G, L, R⁵, R⁶, S¹, S², S³, X, X', X" und Y wie oben definiert sind;
g₁) ausgehend von Verbindungen der Formel XIII die Schritte e₁) und f₁) bis zur gewünschten Kettenlänge wiederholt wobei Verbindungen der Formel XIV resultieren, 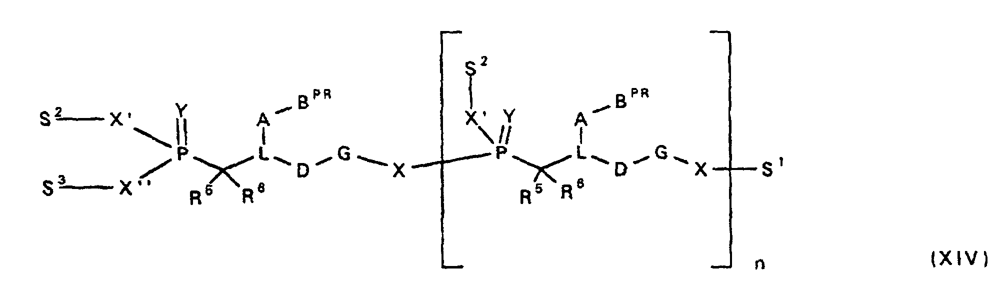 worin
   A, B^{PR}, D, G, L, R⁵, R⁶, S¹, S², S³, X, X', X", Y und n wie oben definiert sind;
h₁) die Schutzgruppen S¹, S², und S³ und die Schutzgruppen an B^{PR} nach bekannten Verfahren abspaltet (z.B. Greene, Wuts, Protective Groups in Organic Synthesis, J. Wiley & Sons, New York 1991), d.h. beispielsweise die Schutzgruppe S¹ wie in Schritt e₁) beschrieben, die Schutzgruppen S² oder S³, falls sie für 2-(4-Nitrophenyl)ethyl stehen, durch Behandlung mit 0.5M 1,8-Diazabicyclo[5,4,0]undec-7-en (DBU) in Pyridin oder Acetonitril bei Raumtemperatur, falls S² oder S³ für Phenyl stehen, durch Behandlung mit wässrigem Ammoniak, falls S² oder S³ für Allyl stehen, durch Behandlung mit Pd[P(C₆H₅)₃]₄ und Triphenylphosphin in DCM (Hayakawa et al., J. Org. Chem. 58 (1993) 5551), falls S² oder S³ für 2-Cyanoethyl stehen, durch Behandlung mit Triethylamin in Pyridin, oder falls S² oder S³ für 2,2,2-Trichlor-1,1-dimethylethyl stehen, durch Behandlung mit Tributylphosphin, und die Schutzgruppen an B^{PR}, beispielsweise falls R¹⁴ für para-Nitrophenylethyloxycarbonyl steht, mit 0.5M DBU in Pyridin, falls R¹⁴ für Isobutyryl oder Benzoyl oder para-(Methoxy)benzoyl steht, mit konz. NH₄OH bei 20 bis 60 °C oder, falls R¹³ für 2-(4-Nitrophenyl)ethyl steht, durch Behandlung mit 0.5M DBU in Pyridin oder Acetonitril, bevorzugt wird, wenn S¹ gleich Monomethoxytrityl und S² gleich 2-(para-Nitrophenyl)ethyl wird bevorzugt zuerst die Monomethoxytritylgruppe wie in e₁) beschrieben abgespalten, danach S² wie beschrieben abgespalten, danach die restlichen Schutzgruppen, beispielsweise an den Nucleobasen, entfernt;
und gegebenenfalls die Gruppen Q und Q' nach dem Fachmann bekannten Verfahren einführt (s. z.B. Uhlmann & Peyman, Chem. Rev. 90 (1990) 543; M. Manoharan in "Antisense Research and Applications", Crooke and Lebleu, Eds., CRC Press, Boca Raton, 1993, Chapter 17, S.303ff; EP-A 0 552 766; S. Agrawal in Methods in Molecular Biology, Vol. 26, S. 93ff, Humana Press, Totowa 1994), und gegebenenfalls die erhaltenen Verbindungen gemäß Wang, Nucl. Acids Res. 22 (1994) 2326 cyclisiert, wodurch Verbindungen der Formel I resultieren.

Alternativ lassen sich Konjugate Q' auch nach dem Fachmann bekannten Verfahren (J. March, "Advanced Organic Chemistry", Fourth Ed., J. Wiley & Sons, 1992) in die Monomerbausteine der Formel XXII einbauen, die dann entsprechend den genannten Verfahren in die Verbindungen der Formel 1 eingebaut werden.

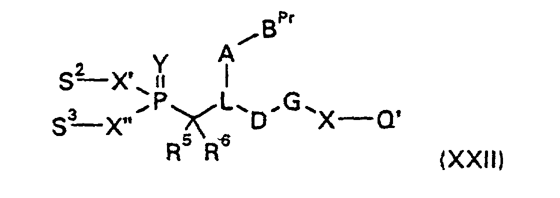

Verbindungen der Formel XXII lassen sich beispielsweise für Q' = Alkyl herstellen durch Umsetzung von Verbindungen der Formel XXIII mit Verbindungen der Formeln Vla oder Vlb und weitere Umsetzungen analog wie es für die Formeln Va bzw. Vb beschrieben wurde.

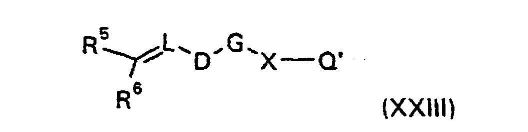

Verbindungen der Formel XXII lassen sich auch herstellen aus Verbindungen der Formel IX durch Abspaltung der Schutzgruppe S¹ und Einführung der Gruppe Q' nach bekannten Verfahren (J. March, "Advanced Organic Chemistry", Fourth Ed., J. Wiley & Sons, 1992).

Alternativ lassen sich Konjugate Q und Q" auch nach dem Fachmann bekannten Verfahren (J. March, "Advanced Organic Chemistry", Fourth Ed., J. Wiley & Sons, 1992) in die Monomerbausteine Der Formel XXIV einbauen, die dann entsprechend den genannten Verfahren in die Verbindungen der Formel I eingebaut werden. 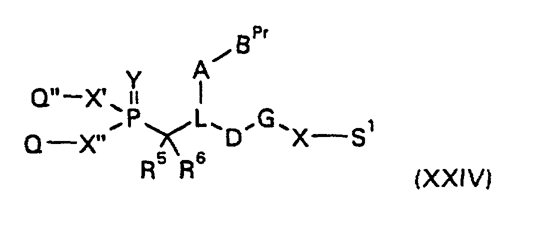 Kopplungsreagenzien zur Knüpfung von Peptid-Bindungen (siehe c₁)) sind beispielsweise beschrieben in Houben-Weyl, Methoden der organischen Chemie, Band 15/2, Georg Thieme Verlag Stuttgart 1974 und weitere Reagenzien wie z.B. BOP (B. Castro, J.R. Dormoy, G. Evin and C. Selve, Tetrahedron Lett. 1975, 1219-1222), PyBOP (J. Coste, D. Le-Nguyen und B. Castro, Tetrahedron Lett. 1990, 205-208), BroP (J. Coste, M.-N. Dufour, A. Pantaloni und B. Castro, Tetrahedron Lett. 1990, 669-672), PyBroP (J. Coste, E. Frerot, P. Jouin und B. Castro, Tetrahedron Lett. 1991, 1967-1970) und Uronium-Reagenzien, wie z.B. HBTU (V. Dourtoglou, B. Gross, V. Lambropoulou, C. Zioudrou, Synthesis 1984, 572-574), TBTU, TPTU, TSTU, TNTU (R. Knorr, A. Trzeciak, W. Bannwarth and D. Gillessen, Tetrahedron Letters 1989, 1927-1930), TOTU (EP-A-0 460 446), HATU (L.A. Carpino, J. Am. Chem. Soc. 1993, 115, 4397-4398), HAPyU, TAPipU (A. Ehrlich, S. Rothemund, M. Brudel, M. Beyermann, L.A. Carpino und M. Bienert, Tetrahedron Lett. 1993, 4781-4784), BOI (K. Akaji, N. Kuriyama, T. Kimura, Y. Fujiwara und Y. Kiso, Tetrahedron Lett. 1992, 3177-3180) oder Säurechloride bzw. Säurefluoride (L. A. Carpino, H. G. Chao, M. Beyermann and M. Bienert, J. Org. Chem., 56 (1991), 2635; J.-N. Bertho, A. Loffet, C. Pinel, F. Reuther and G. Sennyey in E. Giralt and D. Andreu (Eds.) Peptides 1990, Escom Science Publishers B. V.1991, pp. 53-54; J. Green und K. Bradley, Tetrahedron 1993, 4141-4146), 2,4,6-Mesitylensulfonyl-3-nitro-1,2,4-triazolid (MSNT) (B. Blankemeyer-Menge, M. Nimitz und R. Frank, Tetrahedron Lett. 1990, 1701-1704), 2,5-Diphenyl-2,3-dihydro-3-oxo-4-hydroxythiophendioxid (TDO) (R. Kirstgen, R.C. Sheppard, W. Steglich, J. Chem. Soc. Chem. Commun. 1987, 1870-1871) oder aktivierte Ester (D. Hudson, Peptide Res. 1990, 51-55) in den jeweiligen Literaturstellen. Bevorzugt ist ferner die Verwendung von Carbodiimiden, z.B. Dicyclohexylcarbodiimid oder Diisopropylcarbodiimid. Bevorzugt verwendet werden ebenfalls Phosphonium-Reagenzien, wie z.B. PyBOP oder PyBroP, Uronium-Reagenzien, wie z.B. HBTU, TBTU, TPTU, TSTU, TNTU, TOTU oder HATU, BOI.

Dabei kann die Kopplung direkt durch Addition von Verbindungen der Formel VIII mit dem Aktivierungsreagenz und gegebenenfalls unter Zusatz von Additiven wie z.B. 1-Hydroxybenzotriazol (HOBt) (W. König, R. Geiger, Chem. Ber. 103, 788 (1970)) oder 3-Hydroxy-4-oxo-3,4- dihydrobenzotriazin (HOObt) (W. König, R. Geiger, Chem. Ber. 103, 2034 (1970)) durchgeführt werden oder aber die Voraktivierung des Bausteines als aktivierter Ester kann separat erfolgen und die Lösung der aktivierten Spezies in einem geeigneten Lösungsmittel zugegeben werden.

Gegenstand der Erfindung ist weiterhin ein Verfahren zur Herstellung der Verbindungen der Formel I, worin n 1 bis 100 bedeutet, das dadurch gekennzeichnet ist, daß man in den Verbindungen der Formeln XV und XVI, 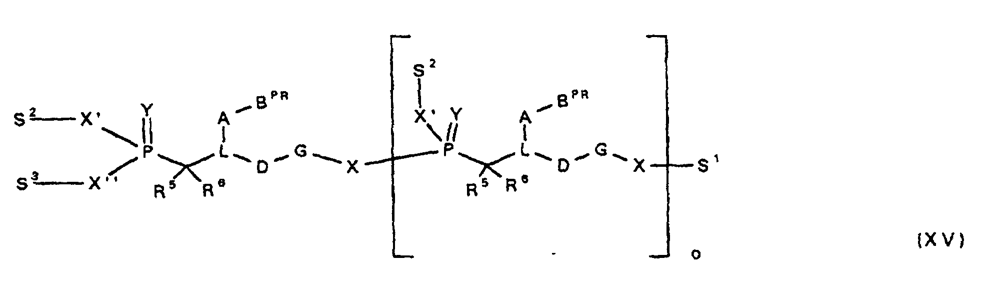 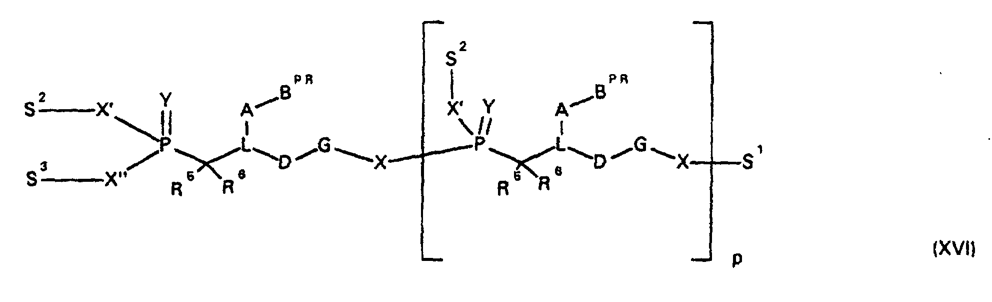 worin
A, B^{PR}, D, G, L, R⁵, R⁶, S¹, S², S³, X, X', X" und Y wie oben definiert sind, o und p unabhängig voneinander Null bis 50, bevorzugt Null bis 20 und o+p+1 =n bedeuten;
a₂) in den Verbindungen der Formel XV die Schutzgruppe S¹ wie unter e₁) beschrieben abspaltet,
b₂) in den Verbindungen der Formel XVI die Schutzgruppe S³ wie unter d₁) beschrieben abspaltet und
c₂) die entstehenden Verbindungen wie unter f₁) beschrieben miteinander koppelt, wobei Verbindungen der Formel XIV resultieren, 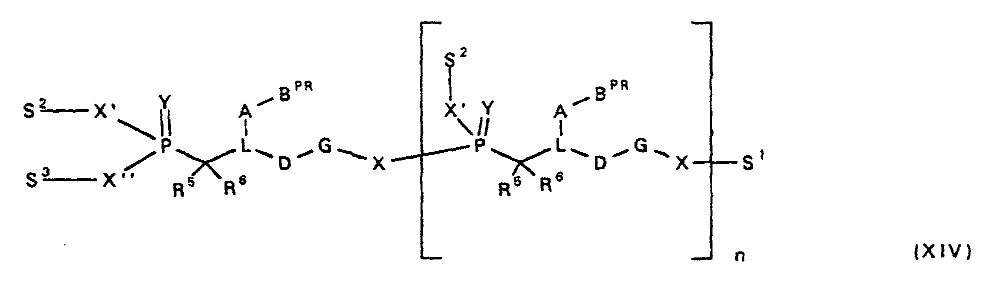 worin
   A, B^{PR}, D, G, L, R⁵, R⁶, S¹, S², S³, X, X', X", Y und n wie oben definiert sind,
d₂) und diese wie unter h₁} beschrieben zu Verbindungen der Formel I umsetzt.

Gegenstand der Erfindung ist weiterhin ein Verfahren zur Herstellung der Verbindungen der Formel I, das dadurch gekennzeichnet, daß man
a₃) Verbindungen der Formel X, 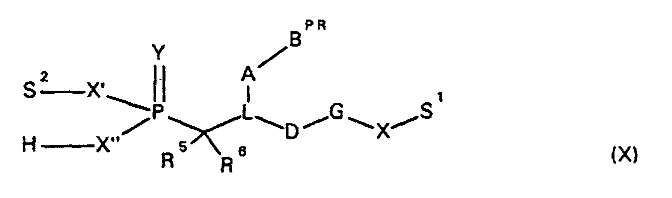 worin
   A, B^{PR}, D, G, L, R⁵, R⁶, S¹, S², X, X', X" und Y wie oben definiert sind,
   nach bekannten Verfahren über einen SPACER an einen festen Träger koppelt, zu Verbindungen der Formel XVII, 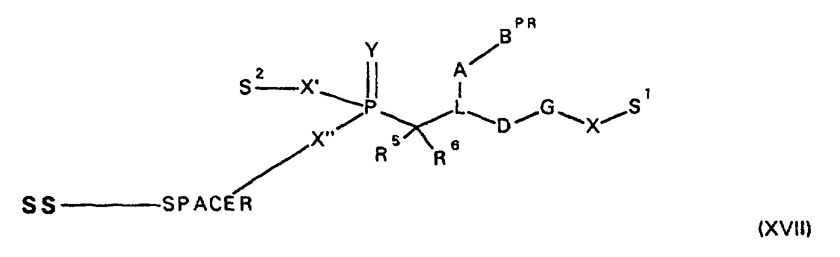 worin
   A, B^{PR}, D, G, L, R⁵, R⁶, S¹, S², X, X', X" und Y wie oben definiert sind,
   - SS: für einen zur Festphasensynthese geeigneten festen Träger steht, wie beispielsweise Aminopropyl-CPG (CPG =® Controlled Pore Glass) oder ® Tentagel, und
   SPACER für eine vom Träger nach erfolgter Synthese abspaltbare Gruppe steht, wie sie dem Fachmann bekannt sind (Sonveaux, Bioorganic Chemistry 14 (1986) 274ff), beispielsweise die Bis(hydroxyethyl)sulfonylgruppe, wie sie in EP-A 0 552 766 (HOE 92/F012) beschrieben ist, oder SPACER für bisfunktionelle Konjugatmoleküle Q, die über bekannte abspaltbare Gruppen an den festen Träger geknüpft werden, beispielsweise für Nucleotide oder Oligonucleotide, die über einen Bernsteinsäurerest an den festen Träger gebunden werden (Sonveaux, Bioorganic Chemistry 14 (1986) 274ff) oder Poly-bzw. Oligoethylenglykole, die über einen Bernsteinsäurerest an den festen Träger gebunden werden (Jäschke, Tetrahedron Lett. 34 (1993) 301) oder beispielsweise Cholesterinderivate, die über einen Bernsteinsäurerest an den festen Träger gebunden werden (MacKellar, Nucl. Acids Res. 20 (1992) 3411) steht;
b₃) aus Verbindungen der Formel XVII, 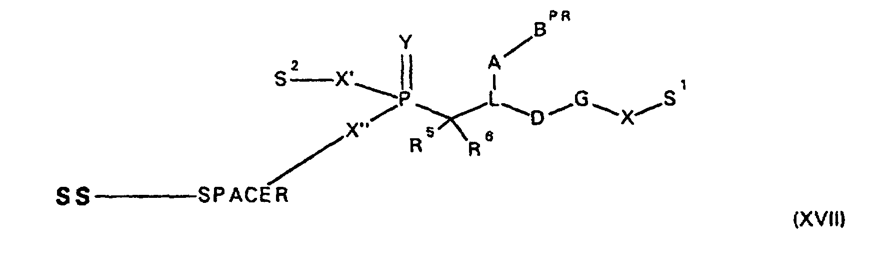 worin
   A, B^{PR}, D, G, L, R⁵, R⁶, S¹, S², SS, SPACER, X, X', X" und Y wie oben definiert sind,
   die Schutzgruppe S¹ wie unter e₁) beschrieben abspaltet;
c₃) die resultierende Verbindung mit Verbindungen der Formel X, 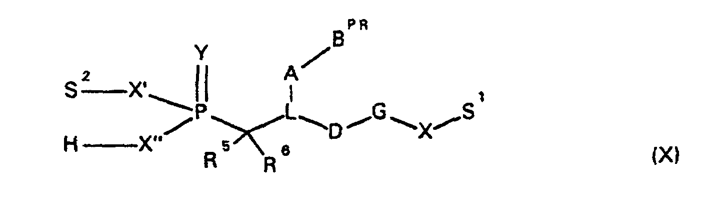 worin
   A, B^{PR}, D, G, L, R⁵, R⁶, S¹, S², X', X" und Y wie oben definiert sind, wie unter f₁) beschrieben umsetzt;
d₃) die Schritte b₃) und c₃) bis zur gewünschten Kettenlänge wiederholt;
e₃) gegebenenfalls Konjugate Q' durch bekannte Verfahren (s. z.B. Uhlmann & Peyman, Chem. Rev. 90 (1990) 543; M. Manoharan in "Antisense Research and Applications", Crooke and Lebleu, Eds., CRC Press, Boca Raton, 1993, Chapter 17, S.303ff; EP-A 0 552 766; S. Agrawal in "Methods in Molecular Biology", Vol. 26, S. 93ff, Humana Press, Totowa 1994) aufkoppelt;
f₃) die so erzeugten Verbindungen nach bekannten Verfahren vom festen Träger abspaltet, beispielsweise den Bis(hydroxyethyl)sulfonyl-Linker, wie in EP-A 0 552 766 (HOE 92/F 012) beschrieben, durch Behandlung mit DBU, den Bernsteinsäure-Linker durch Behandlung mit wäßrigem Ammoniak und die Schutzgruppen wie in Schritt h₁) beschrieben, wobei die Abspaltung der Schutzgruppen auch vor der Spaltung vom Träger erfolgen kann, und gegebenenfalls Konjugate Q durch bekannte Verfahren (s.o.) aufkoppelt, wobei die Reihenfolge der Aufkopplung von Q bzw. Q' (e₃, f₃) auch geändert werden kann, und gegebenenfalls die erhaltenen Verbindungen cyclisiert.

Die Verbindungen der Formel I finden als Inhibitoren der Genexpression Verwendung. Gegenstand der Erfindung sind daher die Verwendung von therapeutisch wirksamen erfindungsgemäßen Verbindungen zur Herstellung eines Arzneimittels sowie ein Verfahren zur Herstellung eines Arzneimittels, das dadurch gekennzeichnet ist, daß man die erfindungsgemäßen Verbindungen mit einem physiologisch annehmbaren Träger sowie gegebenenfalls geeigneten Zusatz- und/oder Hilfsstoffen vermischt.

Als therapeutisch wirksame Verbindungen versteht man im allgemeinen solche, die aufgrund der Abfolge der Bausteine B, die den Nucleobasen entsprechen eine Funktion ausüben als Analoge von Antisense Oligonucleotiden, Tripelhelixbiidende-Oligonucleotiden, Aptameren (RNA oder DNA-Moleküle die an spezifische Zielmoleküle, z. B. Proteine oder Rezeptoren, binden können (z.B. L.C. Bock et al., Nature 1992, 355, 564) oder Ribozyme (katalytische RNA, s. z.B. Castanetto et al., Critical Rev. Eukar. Gene Expr. 1992, 2, 331), insbesondere als Analoge von Antisense-Oligonucleotiden und Tripelhelixbildende-Oligonucleotiden.

Darüberhinaus ist ein weiterer Gegenstand der vorliegenden Erfindung die Verwendung der erfindungsgemäßen Verbindungen als Diagnostikum, beispielsweise zur Detektion der Präsenz oder Absenz oder der Menge eines spezifischen doppelsträngigen oder einzelsträngigen Nucleinsäuremoleküls in einer biologischen Probe.

Die erfindungsgemäßen Verbindungen haben für die erfindungsgemäße Verwendung eine Länge (n-1) von ca. 6 - 100, vorzugsweise von ca. 10 - 40, insbesondere von ca. 12 - 31 Nucleotiden. Ansonsten gelten auch hier die oben beschriebenen Vorzugsbereiche, Modifikationen bzw. Konjugationen.

Die Arzneimittel der vorliegenden Erfindung können beispielsweise zur Behandlung von Erkrankungen, die durch Viren hervorgerufen werden, beispielsweise durch HIV, HSV-1, HSV-2, Influenza, VSV, Hepatitis B oder Papilloma Viren, verwendet werden.

Erfindungsgemäße Sequenzen (Basenabfolgen), die gegen solche Targets wirksam sind, sind beispielsweise:
a) gegen HIV, z. B. 
ACACCCAATTCTGAAAATGG | SEQ ID NO:1
AGGTCCCTGTTCGGGCGCCA | SEQ ID NO:2
GGTCCCTGTTCGGGCGCCA | SEQ ID NO:26
GTCGACACCCAATTCTGAAAATGGATAA | SEQ ID NO:3
GCTATGTCGACACCCAATTCTGAAA | SEQ ID NO:4
GTCGCTGTCTCCGCTTCTTCTTCCTG | SEQ ID NO:5
GTCTCCGCTTCTTCTTCCTGCCATAGG | SEQ ID NO:6
b) gegen HSV-1, z.B.

GCGGGGCTCCATGGGGGTCG SEQ ID NO:7
GGAGGATGCTGAGGAGG SEQ ID NO:28
GGAGGATGCTGAGG SEQ ID NO:29
CAGGAGGATGCTGAGGAGG SEQ ID NO:30

Die Arzneimittel der vorliegenden Erfindung eignen sich beispielsweise auch zur Behandlung von Krebs oder der Restenose. Beispielsweise können dabei Sequenzen (Basenabfolgen) zum Einsatz kommen, die gegen Targets gerichtet sind, die für Krebsentstehung bzw. Krebswachstum verantwortlich sind. Solche Targets sind beispielsweise:
1) Nucleare Onkoproteine wie beispielsweise c-myc, N-myc, c-myb, c-fos, c-fos/jun, PCNA, p120
2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl
3) Zelluläre Rezeptoren wie beispielsweise EGF-Rezeptor, c-erbA, Retinoid-Rezeptoren, Protein-Kinase regulatorische Untereinheit, c-fms
4)Cytokine, Wachstumsfaktoren, Extrazelluläre Matrix wie beispielsweise CSF-1, IL-6, IL-1a, IL-1b, IL-2, IL-4, bFGF, Myeloblastin, Fibronectin,

Erfindungsgemäße Sequenzen (Basenabfolgen), die gegen solche Targets wirksam sind, sind beispielsweise
a) gegen c-Ha-ras, z. B. CAGCTGCAACCCAGC SEQ ID NO:8
c) c-myc, z.B. 
GGCTGCTGGAGCGGGGCACAC | SEQ ID NO:9
AACGTTGAGGGGCAT | SEQ ID NO:10
d) c-myb, z.B. 
GTGCCGGGGTCTTCGGGC SEQ ID NO:11
GTGCCGGGGTCTTCGGG SEQ ID NO:27
e) c-fos, z.B. 
GGAGAACATCATGGTCGAAAG SEQ ID NO:12
CCCGAGAACATCATGGTCGAAG SEQ ID NO:13
GGGGAAAGCCCGGCAAGGGG SEQ ID NO:14
f) p120, z.B. CACCCGCCTTGGCCTCCCAC SEQ ID NO:15
g) EGF-Rezeptor, z.B. 
GGGACTCCGGCGCAGCGC SEQ ID NO:16
GGCAAACTTTCTTTTCCTCC SEQ ID NO:17
h) p53 Tumorsuppressor, z.B. 
GGGAAGGAGGAGGATGAGG SEQ ID NO:18
GGCAGTCATCCAGCTTCGGAG SEQ ID NO:19
i) bFGF, z.B.

GGCTGCCATGGTCCC SEQ ID NO:31

Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM oder ELAM.

Erfindungsgemäße Sequenzen (Basenabfolgen), die gegen solche Targets wirksam sind, sind beispielsweise
a) VLA-4, z.B. GCAGTAAGCATCCATATC SEQ ID NO:20
b) ICAM, z.B. 
CCCCCACCACTTCCCCTCTC SEQ ID NO:21
CTCCCCCACCACTTCCCCTC SEQ ID NO:22
GCTGGGAGCCATAGCGAGG SEQ ID NO:23
c) ELAM-1, z. B.

ACTGCTGCCTCTTGTCTCAGG SEQ ID NO:24
CAATCAATGACTTCAAGAGTTC SEQ ID NO:25

Die Arzneimittel der vorliegenden Erfindung eignen sich beispielsweise ferner zur Behandlung von Erkrankungen, die durch Faktoren wie TNF alpha ausgelöst werden. Erfindungsgemäße Sequenzen (Basenabfolgen), die gegen solche Targets wirksam sind, sind beispielsweise
a) TNF-alpha, z.B.

TCATGGTGTCCTTTGCAGCC SEQ ID NO:32
TCATGGTGTCCTTTGCAG SEQ ID NO:33

Die Arzneimittel können z.B. in Form von pharmazeutischen Präparaten, die man beispielsweise topisch oder oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talk und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzlich und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.
Eine bevorzugte Applikation ist die orale Applikation. Eine weitere bevorzugte Form der Verabreichung ist die Injektion. Hierzu werden die Antisense-Oligonucleotide in einer flüssigen Lösung, vorzugsweise in einem physiologisch annehmbaren Puffer, wie z.B. Hank's Lösung oder Ringer's Lösung, formuliert. Die erfindungsgemäßen therapeutisch wirksamen Verbindungen können aber auch in fester Form formuliert werden und vor dem Gebrauch gelöst oder suspendiert werden. Die für die systematische Verabreichung bevorzugten Dosierungen betragen ca. 0,01 mg/kg bis ca. 50 mg/kg Körpergewicht und Tag.

### Sequenzliste:

ACACCCAATTCTGAAAATGG | SEQ ID NO:1
AGGTCCCTGTTCGGGCGCCA | SEQ ID NO:2
GTCGACACCCAATTCTGAAAATGGATAA | SEQ ID NO:3
GCTATGTCGACACCCAATTCTGAAA | SEQ ID NO:4
GTCGCTGTCTCCGCTTCTTCTTCCTG | SEQ ID NO:5
GTCTCCGCTTCTTCTTCCTGCCATAGG | SEQ ID NO:6
GCGGGGCTCCATGGGGGTCG | SEQ ID NO:7
CAGCTGCAACCCAGC | SEQ ID NO:8

GGCTGCTGGAGCGGGGCACAC | SEQ ID NO:9
AACGTTGAGGGGCAT | SEQ ID NO:10
GTGCCGGGGTCTTCGGGC | SEQ ID NO:11
GGAGAACATCATGGTCGAAAG | SEQ ID NO:12
CCCGAGAACATCATGGTCGAAG | SEQ ID NO:13
GGGGAAAGCCCGGCAAGGGG | SEQ ID NO:14
CACCCGCCTTGGCCTCCCAC | SEQ ID NO:15
GGGACTCCGGCGCAGCGC | SEQ ID NO:16
GGCAAACTTTCTTTTCCTCC | SEQ ID NO:17
GGGAAGGAGGAGGATGAGG | SEQ ID NO:18
GGCAGTCATCCAGCTTCGGAG | SEQ ID NO:19
GCAGTAAGCATCCATATC | SEQ ID NO:20
CCCCCACCACTTCCCCTCTC | SEQ ID NO:21
CTCCCCCACCACTTCCCCTC | SEQ ID NO:22
GCTGGGAGCCATAGCGAGG | SEQ ID NO:23
ACTGCTGCCTCTTGTCTCAGG | SEQ ID NO:24
CAATCAATGACTTCAAGAGTTC | SEQ ID NO:25
GGTCCCTGTTCGGGCGCCA | SEQ ID NO:26
GTGCCGGGGTCTTCGGG | SEQ ID NO:27
GGAGGATGCTGAGGAGG | SEQ ID NO:28
GGAGGATGCTGAGG | SEQ ID NO:29
CAGGAGGATGCTGAGGAGG | SEQ ID NO:30
GGCTGCCATGGTCCC | SEQ ID NO:31
TCATGGTGTCCTTTGCAGCC | SEQ ID NO:32
TCATGGTGTCCTTTGCAG | SEQ ID NO:33

### Beispiele:

1) N-(4-Methoxytriphenylmethoxy)ethylaminomethanphosphonsäuredi(2-(p-nitrophenyl)ethyl)ester
   1a) N-Fluorenylmethyloxycarbonyl-2-aminoethanol
      8.61 g (0.141 mol) 2-Aminoethanol wurden in 250 ml Dioxan und 150 ml H₂O gelöst. Bei 15-20°C wurden zunächst 17.79 g (0.212 mol) NaHCO₃, dann portionsweise 50 g (0.148 mol) Fluorenylmethyloxycarbonyl-N-succinimid zugegeben. Es wurde 1h bei Raumtemperatur gerührt, dann wurde zur Trockne eingedampft. Der Rückstand wurde zwischen Dichlormethan (DCM) und H₂O verteilt, die organische Phase über Na₂SO₄ getrocknet und das Lösungsmittel im Vakuum abgedampft. Der Rückstand wurde mit 100 ml Ether verrührt, das Produkt abgesaugt und gut mit Ether gewaschen. Die Ausbeute betrug 38.77 g (97%).
      MS (ES+) 284.2 (M+H)⁺; ¹H-NMR (200MHz, DMSO, TMS): δ = 3.05 (dd, 2H, CH₂OH); 3.39 (dd, 2H, N-CH₂); 4,25 (m, 3H, Ar-CH-CH₂); 4.61 (t, 1H, OH); 7.14-7.98 (m, 15H, Ar-H, NH).
   1 b) N-Fluorenylmethyloxycarbonyl-2-amino-1-(4-methoxytriphenylmethoxy)ethan
      10 g (35.3 mmol) N-Fluorenylmethyloxycarbonyl-2-aminoethanol (aus Beispiel 1a), gelöst in 100 ml absol. N,N-Dimethylformamid (DMF) wurden bei 0°C mit 5.93 g (45.93 mmol) Diisopropylethylamin (DIPEA) und 10.91 g (35.3 mmol) 4-Methoxytriphenylmethylchlorid versetzt und zunächst 1h bei 0°C, dann 1h bei Raumtemperatur gerührt. Das Reaktionsgemisch wurde eingedampft und zwischen DCM und einer gesättigten wässrigen NaHCO₃-Lösung verteilt. Die organische Phase wurde mit H₂O gewaschen über Na₂SO₄ getrocknet und das Lösungsmittel im Vakuum abgedampft. Zur Reinigung wurde über Kieselgel chromatographiert (zuerst n-Heptan/Essigsäureethylester (EE)/Triethylamin (TEA) 70/29/1; dann EE/TEA 99/1). Die Ausbeute betrug 14,4 g (73%).
      MS (FAB): 562.3 (M+Li)⁺; ¹H-NMR (200MHz, DMSO, TMS): δ = 2.95 (t, 2H, CH₂O-MMTr); 3.21 (dd, 2H, N-CH₂); 3.75 (s, 3H, OCH3); 4,25 (m, 3H, Ar-CH-CH₂); 4.61 (t, 1H, OH); 6.80-7.96 (m, 23H, Ar-H, NH).
   1c) 2-Amino-1-(4-methoxytriphenylmethoxy)ethan
      5.0 g (9 mmol) N-Fluorenylmethyloxycarbonyl-2-amino-1-(4-methoxytriphenylmethoxy)ethan (aus Beispiel 1b), gelöst in 50 ml absol. DMF wurden bei Raumtemperatur mit 6.55 g (90 mmol) Diethylamin versetzt und 2h gerührt. Zur Reinigung wurde an Kieselgel chromatographiert (zuerst n-Heptan/ EE/ TEA 50/49/1; dann EE/Methanol/TEA 79/20/1). Die Ausbeute betrug 2.96 g (98.7%).
      MS (ES+): 340.3 (M+Li)⁺; ¹H-NMR (200MHz, DMSO, TMS): δ = 2.75 (t, 2H, CH₂O-MMTr); 2.93 (dd, 2H, N-CH₂); 3.75 (s, 3H, OCH3); 6.83-7.47 (m, 14H, Ar-H).
   1d) 2-Methylimino-1-(4-methoxytriphenylmethoxy)ethan (Trimer)
      2.96g (8.9 mmol) 2-Amino-1-(4-methoxytriphenylmethoxy)ethan (aus Beispiel 1c), gelöst in 10ml Methanol wurden unter Eiskühlung mit 1.08g (13.22 mmol) 37% Formaldehyd versetzt und 4h bei Raumtemperatur gerührt, wobei sich ein zäher Niederschlag bildete. Das Reaktionsgemisch wurde eingedampft und zur Reinigung über Kieselgel chromatographiert (n-Heptan/EE/TEA 50/49/1). Die Ausbeute betrug 1.7g (55%).
      MS (FAB) 1042.8 (M+Li)⁺; 1034.8 (M-H)⁺. ¹H-NMR (200MHz, DMSO, TMS): δ = 2.60 (t, 6H, O-CH₂); 2.99(t, 6H, N-CH₂); 3.69 (s, 9H, OCH₃); 6.78-7.42 (m, 42H, Ar-H).
   1e) Di(2-(4-nitrophenyl)ethyl)phosphit
      23.42g (0.1 mol) Diphenylphosphit wurden zusammen mit 33.43g (0.2 mol) p-Nitrophenylethanol unter Argon für 14h auf 100°C erhitzt, zur Reinigung wurde über Kieselgel chromatographiert (n-Heptan/EE 50/50; dann EE/Methanol 80/20). Ausbeute: 55%.
      MS (FAB) 403.1 (M+Na)⁺; 381.1 (M+H)⁺. ¹H-NMR (200MHz, DMSO, TMS): δ = 3.03 (t, 4H, Ar-CH₂); 4.20(4H, dt, O-CH₂); 6.71 (d, J=140Hz; 1H, PH); 7.52 (d, 4H, Ar-H); 8.17 (d, 4H, Ar-H).
   1f) N-(4-methoxytriphenylmethoxy)ethylaminomethanphosphonsäuredi(2-(p-nitrophenyl)ethyl)ester
      Zu 500mg (1.32 mmol) Di(2-(4-nitrophenyl)ethyl)phosphit (aus Beispiel 1e), gelöst in 2 ml absol. Tetrahydrofuran (THF) wurden 341 mg (0.329 mmol) 2-Methylimino-1-(4-methoxytriphenylmethoxy)ethan (Trimer) (aus Beispiel 1d) gegeben und das Gemisch wurde 3h bei 80°C gerührt. Das Lösungsmittel wurde abgedampft und es wurde noch 30 min bei 100°C gerührt. Zur Reinigung wurde über Kieselgel chromatographiert (zuerst EE/TEA 99/1; dann EE/Methanol/TEA 90/9/1). Ausbeute: 83%.
      MS(FAB) 732.3 (M+Li)⁺. ¹H-NMR (200MHz, DMSO, TMS): δ = 2.64-3.06 (m, 10H, Ar-CH₂ + P-CH₂ + CH₂-OMMTr + N-CH₂); 3.73 (s, 3H, OCH₃); 4.16 (dt, 4H, PO-CH₂); 6.78-8.08 (m, 22H, Ar-H).
2) N-(N⁶-Anisoyl)cytosin-1-yl-acetyl-N-(4-methoxytriphenylmethoxy)ethylaminomethanphosphonsäuredi(2-(p-nitrophenyl)ethyl)ester
   2a) Zu 2.00 g (2.76 mmol) N-(4-methoxytriphenylmethoxy)ethylaminomethanphosphonsäuredi(2-(p-nitrophenyl)ethyl)ester (aus Beispiel 1f), gelöst in 60 ml absol. DMF, wurden 0.952g (8.27 mmol) N-Ethylmorpholin (NEM), 0.834g (2.76 mmol) (N⁶-Anisoyl)cytosin-1-yl-essigsäure und 1.153g (3.03 mmol) O-(7-aza)benzotriazol-1-yltetramethyluroniumhexafluorophosphat (HATU, L. Carpino, J. Am. Chem. Soc. 1993, 115, 4397) zugegeben und 12h bei Raumtemperatur gerührt. Danach wurde nocheinmal die gleiche Menge HATU zugegeben und weitere 3h bei Raumtemp. gerührt. Zur Reinigung wurde an Kieselgel chromatographiert (DCM/Methanol/TEA 95/4/1). Die Ausbeute betrug 2.7g (97%).
      MS(ES+): 1012.0 (M+H)⁺. ¹H-NMR (200MHz, DMSO, TMS): δ = 2.94 (t, 4H, P-O-CH₂-CH₂-Ar); 3.06 (t, 2H, MMTr-O-CH₂); 3.23-3.63 (m, 4H, P-CH₂ + N-CH₂); 3.75 (s, 3H, OCH₃); 3.83 (s, 3H, OCH₃); 4.10 (dt, 4H, P-O-CH₂); 4.79 (s, breit, 2H, CO-CH₂); 6.80-8.18 (m, 28H, Ar-H, Cytosinyl-H); 11.03 (s breit, 1H, NH).
   2b) Ansatz wie in Beispiel 2a, jedoch unter Verwendung von O-(Cyan(ethoxycarbonyl)methylenamino)-1,1,3,3-tetramethyluroniumtetrafluoroborat (TOTU, EP 0460446) anstelle von HATU. Die Ausbeute betrug 57%. Spektroskopische Daten: s. Beispiel 2a.
3) N-(N⁶-Anisoyl)cytosin-1-yl-acetyl-N-(4-methoxytriphenylmethoxy)ethylaminomethanphosphonsäure(2-(p-nitrophenyl)ethyl)monoester (Triethylammoniumsalz)
   1g (0.99 mmol) N-(N⁶-Anisoyl)cytosin-1-yl-acetyl-N-(4-methoxytriphenylmethoxy)ethylaminomethanphosphonsäuredi(2-(p-nitrophenyl)ethyl)ester (aus Beispiel 2) wurden in 20 ml einer 0.1M Lösung 1,8-Diazabicyclo[5.4.0]undec-7-en (DBU) in absol. Acetonitril gelöst und 4h bei Raumtemp. gerührt. Das Reaktionsgemisch wurde zwischen DCM und einer wässrigen KH₂PO₄-Lösung (pH 7) verteilt, die organische Phase wurde über Na₂SO₄ getrocknet und das Lösungsmittel im Vakuum abgedampft. Zur Reinigung wurde über Kieselgel chromatographiert (EE/Methanol/TEA 70/29/1). Die Ausbeute betrug 540 mg (57%).
   MS (FAB): 906.5 (M-H+2Na)⁺; 884.6 (M+Na)⁺; 862.5 (M+H)⁺. ¹H-NMR (200MHz, DMSO, TMS): δ = 3.00 (m, 4H, P-O-CH₂-CH₂-Ar + MMTr-O-CH₂); 3.38-3.60 (m, 4H, P-CH₂ + N-CH₂); 3.73 (s, 3H, OCH₃); 3.82 (s, 3H, OCH₃); 4.01 (dt, 2H, P-O-CH₂); 4.79& 5.03 (jeweils s, breit, 2H, CO-CH₂); 6.78-8.20 (m, 24H, Ar-H, Cytosinyl-H); 11.00 (s breit, 1H, NH).
4) N-(N⁶-Anisoyl)cytosin-1-yl-acetyl-N-(2-hydroxy)ethylaminomethanphosphonsäuredi(2-(p-nitrophenyl)ethyl)ester
   1.00g (0.99 mmol) N-(N⁶-Anisoyl)cytosin-1-yl-acetyl-N-(4-methoxytriphenylmethoxy)ethylaminomethanphosphonsäuredi(2-(p-nitrophenyl)ethyl)ester (aus Beispiel 2) wurden in 80 ml 80% wässriger Essigsäure gelöst und 4h bei Raumtemperatur gerührt. Das Lösungsmittel wurde abgedampft und es wurde zweimal mit Toluol koevaporiert. Zur Reinigung wurde über Kieselgel chromatographiert (EE/Methanol/TEA 85/14/1). Die Ausbeute betrug 522 mg (71%).
   MS (FAB): 761.2 (M+Na)⁺ ; 739.3 (M+H)⁺. ¹H-NMR (200MHz, DMSO, TMS): δ = 2.98 (t, 4H, P-O-CH₂-CH₂-Ar); 3.38-3.67 (m, 4H, N-CH₂CH₂-OH); 3.80-3.89 (m, 2H, P-CH₂); 3.91 (s, 3H, OCH₃); 4.12 (dt, 4H, P-O-CH₂); 4.78 & 4.87 (jew.s, breit, 2H, CO-CH₂); 6.98-8.19 Im, 14H, Ar-H, Cytosinyl-H); 11.02 (s breit, 1H, NH).
5) 5'-MMTr-C^{An}-P(ONPE)-C^{An}-P(ONPE)₂
   Die Synthese erfolgte analog Beispiel 17 aus N-(N⁶-Anisoyl)cytosin-1-yl-acetyl-N-(4-methoxytriphenylmethoxy)ethylaminomethanphosphonsäure(2-(p-nitrophenyl)ethyl)monoester (Triethylammoniumsalz) (Beispiel 3) und N-(N⁶-Anisoyl)cytosin-1-yl-acetyl-N-(2-hydroxy)ethylaminomethanphosphonsäuredi(2-(p-nitrophenyl)ethyl)ester (Beispiel 4). Zur Reinigung wurde über Kieselgel chromatographiert (EE/Methanol/TEA 85/14/1). Ausbeute: 73%.
   MS(FAB) 1605 (M+Na)⁺; 1583 (M+H)⁺; ¹H-NMR (200MHz, DMSO, TMS): δ = 2.94-3.18 (m, 6H, P-O-CH₂-CH₂-Ar); 3.26-3.95 (m, 10H); 3.75 (s, 3H, OCH₃); 3.85 (s, 6H, OCH₃); 3.99-4.36 (m, 8H, P-O-CH₂); 4.75-4.92 Im, breit, 4H, CO-CH₂); 6.83-8.18 (m, 38H, Ar-H, Cytosinyl-H); 10.98 & 11.03 (jew s breit, 2H, NH).
6) 5'-HO-C^{An}-P(ONPE)-C^{An}-P(ONPE)₂
   Die Synthese erfolgte analog Beispiel 4 aus "5'-MMTr-C^{An}-P(ONPE)-C^{An}-P(ONPE)₂" (Beispiel 5). Zur Reinigung wurde über Kieselgel chromatographiert (EE/Methanol/TEA 85/14/1). Die Ausbeute betrug 74%.
   MS (FAB) 1332.4 (M+Na)⁺; 1310.3 (M+H)⁺;
7) N-(4-methoxytriphenylmethoxy)ethylaminomethanphosphonsäurediethylester
   Die Synthese erfolgte analog Beispiel 1f, jedoch mit Diethylphosphit. Ausbeute: 87.5%.
   MS(FAB) 490.2 (M+Li)⁺. ¹H-NMR (200MHz, DMSO, TMS): δ = 1.22 (t, 6H, CH₂-CH₃); 2.80 (t, 2H, N-CH₂); 2.91 (d, J= 12.5 Hz, 2H, P-CH₂);3.02 (t, 2H, CH₂-OMMTr); 3.75 (s, 3H, OCH₃); 4.01 (dq, 4H, PO-CH₂); 6.84-7.45 (m, 14H, Ar-H).
8) N-Thymin-1-yl-acetyl-N-(4-methoxytriphenylmethoxy)ethylaminomethanphosphonsäurediethylester
   Zu 2.04g (4.22 mMol) N-(4-methoxytriphenylmethoxy)ethylaminomethanphosphonsäurediethylester (Beispiel 7), gelöst in 50 ml absol. DMF wurden zugegeben 570.3 mg (4.22 mMol) Hydroxybenzotriazol (HOBT), 972.1 mg (8.44 mMol) NEM, 777 mg (4.22 mMol) Tymidin-1-yl-essigsäure und 639 mg (5.06 mMol) Diisopropylcarbodiimid. Es wurde 16 h bei Raumtemperatur gerührt, das Lösungsmittel abgedampft, der Rückstand wurde in DCM gelöst und mit gesättigter wässriger NaHCO₃-Lösung, dann mit gesättigter wässriger NaCl-Lösung estrahiert. Es wurde über Natriumsulfat getrocknet, das Lösungsmittel abgedampft. Zur Reinigung wurde über Kieselgel chromatographiert (EE/Methanol/TEA 98/2/1).Die Ausbeute betrug 2.47g (90%).
   MS (FAB): 662.3 (M+Na)⁺; 656.3 (M+Li)⁺. ¹H-NMR (200MHz, DMSO, TMS): δ = 1.12-1.32 (m, 6H, CH₂-CH₃); 1.68 & 1.75 (je. s, 3H, T-CH₃); 3.10-3.40 (m, 2H, CH₂-OMMTr); 3.53-3.70 (m, 4H, P-CH₂ + N-CH₂); 3.75 (s, 3H, OCH₃); 3.83-4.16 (m, 4H, PO-CH₂); 4.62 & 4.72 (jew. s, 2H, CO-CH₂); 6.83-7.42 (m, 15H, Ar-H, T-H); 11.28 (s, 1H, NH).
9) N-Thymin-1-yl-acetyl-N-(4-methoxytriphenylmethoxy)ethylaminomethanphosphonsäuremonoethylester (Triethylammoniumsalz)
   811mg (1.25 mMol) N-Thymin-1-yl-acetyl-N-(4-methoxytriphenylmethoxy)ethylaminomethanphosphonsäurediethylester (Beispiel 8) wurden in 3.75 ml 1N NaOH suspendiert. Es wurde 3h bei Raumtemperatur, dann 6h bei 50°C gerührt. Das Reaktionsgemisch wurde im Vakuum eingeengt, der Rückstand über Kieselgel chromatographiert (EE/Methanol/TEA 100/10/10, dann 100/40/10). Die Ausbeute betrug 897 mg (99.5%).
   MS (ES-): 620.4 (M-H)⁻. ¹H-NMR (200MHz, DMSO, TMS): δ = 1.18 (t,9H,N-CH₂-CH₃); 1.68 & 1.74 (je. s, 3H, T-CH₃); 2.96-3.08 (q,6H, N-CH₂-CH₃); 3,35(m, 2H, N-CH₂); 3.43-3.70 (d, J = 11Hz, 2H, P-CH₂); 3.63 (t, 2H, CH₂-OMMTr); 3.75 (s, 3H, OCH₃); 3.78 (dq, 2H, PO-CH₂); 4.60 & 4.86 (jew. s, 2H, CO-CH₂); 6.82-7.41 (m, 15H, Ar-H, T-H); 11.24 (s, 1H, NH).
10) N-Thymin-1-yl-acetyl-N-(2-hydroxy)ethylaminomethanphosphonsäurediethylester
   Die Synthese erfolgte analog Beispiel 4 aus N-Thymin-1-yl-acetyl-N-(4-methoxytriphenylmethoxy)ethylaminomethanphosphonsäurediethylester (Beispiel 8). Zur Reinigung wurde über Kieselgel chromatographiert (EE/Methanol 90/10). Ausbeute: 80%.
   MS (FAB): 400.1 (M+Na)⁺; 378.1 (M+H)⁺. ¹H-NMR (200MHz, DMSO, TMS): δ = 1.17-1.32 (m, 6H, CH₂-CH₃); 1.78 (s, 3H, T-CH₃); 3.40-3.69 (m, 4H, CH₂-OH + N-CH₂): 3.89 (d, J=11Hz, 2H, P-CH₂); 3.92-4.19 (m, 4H, PO-CH₂); 4.70 (s, 2H, CO-CH₂); 4.98 (t,1H,OH); 7.22 & 7.30 (jew. s., 1H, T-H); 11.25 (s, 1H, NH).
11) N-(4-methoxytriphenylmethoxy)ethylaminomethanphosphonsäurediphenylester
   Die Synthese erfolgte analog Beispiel 1f, jedoch mit Diethylphosphit. Ausbeute: 100%.
   MS(FAB) 58.2 (M+Li)⁺.
12) N-Thymin-1-yl-acetyl-N-(4-methoxytriphenylmethoxy)ethylaminomethanphosphonsäuremonophenylester (Triethylammoniumsalz)
   Die Synthese erfolgte analog Beispiel 81 aus N-(4-methoxytriphenylmethoxy)-ethylaminomethanphosphonsäurediphenylester (Beispiel 11) und Tymidin-1-yl-essigsäure. Zur Reinigung wurde über Kieselgel chromatographiert (EE/Methanol/TEA/H₂O 90/10/5/0.5). Ausbeute: 47%.
   MS (FAB): 682.3 (M+2Li-H)⁺. ¹H-NMR (200MHz, DMSO, TMS): δ = 1.16 (t,9H,N-CH₂-CH₃); 1.67 & 1.72 (je. s, 3H, T-CH₃); 2.96-3.70 (m, 12H, N-CH₂-CH₃ + N-CH₂ + P-CH₂ + CH₂-OMMTr); 3.75 (s, 3H, OCH₃);; 4.58 & 4.88 (jew. s, 2H, CO-CH₂); 6.74-7.46 (m, 20H, Ar-H, T-H); 11.23 (s, 1H, NH).
13) N-Thymin-1-yl-acetyl-N-(4-methoxytriphenylmethoxy)ethylaminomethanphosphonsäurephenyl-(4-nitrophenylethyl)diester
   385.4mg (0.5 mMol) N-Thymin-1-yl-acetyl-N-(4-methoxytriphenylmethoxy)-ethylaminomethanphosphonsäuremonophenylester (Triethylammoniumsalz) (Beispiel 12) und 92mg (0.55 mMol) (4-Nitrophenylethanol) wurden dreimal mit absol. Pyridin koevaporiert, anschließend in 15 ml absol. Pyridin gelöst. Bei 0°C wurden 403.4mg (0.15mMol) 3-Nitro-1-(p-toluolsulfonyl)-1H-1,2,4-triazol (TSNT) zugegeben, anschließend 16h bei 0-5°C gerührt, das Pyridin im Vakuum abdestilliert, der Rückstand in EE aufgenommen und nacheinander mit gesättigter wässriger NaHCO₃-Lösung, dann mit NaCI-Lösung gewaschen. Zur Reinigung wurde über Kieselgel chromatographiert (EE/TEA 100/2). Die Ausbeute betrug 162 mg.
   MS (FAB): 831.3 (M+2Li-H)⁺; (M+Li)⁺.
14) N- Thymin-1-yl-acetyl-N-(4-methoxytriphenylmethoxy)ethylaminomethanphosphonsäuredi(2-(p-nitrophenyl)ethyl)ester
   Die Synthese erfolgte analog Beispiel 8 aus N-(4-methoxytriphenylmethoxy)-ethylaminomethanphosphonsäuredi(2-(p-nitrophenyl)ethyl)ester (Beispiel 1f) und Tymidin-1-yl-essigsäure. Ausbeute: 63%
   MS (ES+): 898.4 (M+Li)⁺. ¹H-NMR (200MHz, DMSO, TMS): δ = 1.65 & 1.72 (jew. s, 3H, T-CH₃); 2.96 (t, 4H, P-O-CH₂-CH₂-Ar); 3.06 (t, 2H, N-CH₂); 3.67 (d, J = 11Hz, 2H, P-CH₂); 3.70 (m, 2H, MMTr-O-CH₂); 3.75 (s, 3H, OCH₃); 3.83 (s, 3H, OCH₃); 4.10 (dt, 4H, P-O-CH₂); 4.59 & 4.62 (jew. s, breit, 2H, CO-CH₂); 6.83-8.18 (m, 23H, Ar-H, T-H); 11.30 (s breit, 1H, NH).
15) N-Thymin-1-yl-acetyl-N-(4-methoxytriphenylmethoxy)ethylaminomethanphosphonsäure-(4-nitrophenylethyl)monoester (Triethylammoniumsalz)
   15a) Aus 30mg N-Thymin-1-yl-acetyl-N-(4-methoxytriphenylmethoxy)-ethylaminomethanphosphonsäurephenyl-(4-nitrophenylethyl)diester (Beispiel 13)
      30mg N-Thymin-1-yl-acetyl-N-(4-methoxytriphenylmethoxy)ethylaminomethanphosphonsäurephenyl-(4-nitrophenylethyl)diester (Beispiel 13) wurden in einer Mischung aus 1ml TEA, 1ml Dioxan und 80 mg p-Nitrobenzaldoxim gelöst und 3h bei Raumtemperatur gerührt. Das Lösungsmittel wurde im Vakuum abgedampft, der Rückstand dreimal mit Pyridin und zweimal mit Toluol koevaporiert. Der Rückstand wurde über Kieselgel chromatographiert (EE/TEA 100/2, dann EE/Methanol/TEA 60/40/2). Die Ausbeute betrug 23 mg.
      MS (FAB): 755.3 (M+2Li-H)⁺. ¹H-NMR (250MHz, DMSO, TMS): δ = 1.15 (t,9H,N-CH₂-CH₃); 1.60 & 1.79 (m, 3H, T-CH₃); 2.80-3.3.60 (m, 14H, N-CH₂-CH₃ + N-CH₂ + P-CH₂ + CH₂-OMMTr + Ar-CH₂); 3.73 (s, 3H, OCH₃); 4.01 (dt, 2H, P-O-CH₂); 4.58 -4.92 (m, 2H, CO-CH₂); 6.82-8.18 (m, 19H, Ar-H, T-H); 11.30 (s, 1H, NH).
   15b) Aus N- Thymin-1-yl-acetyl-N-(4-methoxytriphenylmethoxy)ethylaminomethanphosphonsäuredi(2-(p-nitrophenyl)ethyl)ester (Beispiel 14)
      Die Synthese erfolgte analog Beispiel 3 aus N- Thymin-1-yl-acetyl-N-(4-methoxytriphenylmethoxy)ethylaminomethanphosphonsäuredi(2-(p-nitrophenyl)ethyl)ester (Beispiel 14), jedoch in Pyridin als Lösungsmittel. Ausbeute: 82%. Spektoskopische Daten s. Beispiel 15a.
16) N- Thymin-1-yl-acetyl-N-(4-methoxytriphenylmethoxy)ethylaminomethanphosphonsäure
   Die Synthese erfolgte analog Beispiel 15b. Als Nebenprodukt wird in 18% Ausbeute N-Thymin-1-yl-acetyl-N-(4-methoxytriphenylmethoxy)ethylaminomethanphosphonsäure erhalten.
   MS (ES-): 592.2 (M-H)⁻.
17) 5'-MMTr-T-P(OEthyl)-T-P(OEthyl)₂
   361mg (0.5mMol) N-Thymin-1-yl-acetyl-N-(4-methoxytriphenylmethoxy)-ethylaminomethanphosphonsäuremonoethylester (Triethylammoniumsalz) (IX) und 188.7mg (0.5mMol) N-Thymin-1-yl-acetyl-N-(2-hydroxy)ethylaminomethanphosphonsäurediethylester (X) wurden zusammen zweimal mit absol. Pyridin koevaporiert, dann in 10 ml absol. Pyridin gelöst. Bei 5-10°C wurden 1.5mMol TSNT zugegeben und es wurde 16h bei Raumtemp. gerührt. Das Pyridin wurde im Vakuum abgedampft, der Rückstand in EE gelöst und nacheinander mit gesättigter wässriger NaHCO₃-Lösung, dann mit NaCI-Lösung gewaschen. Es wurde über Na₂SO₄ getrocknet, eingeengt und zur Reinigung wurde über Kieselgel chromatographiert (EE/Methanol/TEA 92/8/2). Die Ausbeute betrug 223mg (46%).
   MS (FAB): 987.5 (M+Li)⁺. ¹H-NMR (200MHz, DMSO, TMS) Characteristische Signale sind: Ar-H & Thymin-H: 6.82-7.43 (m, 16H); CO-CH₂: 4.59-4.78 (m, 4H); Thymin-CH₃: 1.63-1.80 (m, 6H).
18) 5'-HO-T-P(OEthyl)-T-P(OEthyl)₂
   Die Synthese erfolgte analog Beispiel 4 aus 5'-MMTr-T-P(OEthyl)-T-P(OEthyl)₂ (Beispiel 17). Zur Reinigung wurde über Kieselgel chromatographiert (EE/Methanol/TEA 85/15/2, dann 100/50/1.5). Die Ausbeute betrug 95%.
   MS (FAB): 731.2 (M+Na)⁺; 709.1 (M+H)⁺. ¹H-NMR (200MHz, DMSO, TMS) Characteristische Signale sind: Thymin-H: 7.21-7.36 (m, 2H); CO-CH₂: 4.60-4.76 (m, 4H); Thymin-CH₃: 1.63-1.79 (m, 6H).
19) 5'-MMTr-T-P(OPhenyl)-T-P(OEthyl)₂
   Die Synthese erfolgte analog Beispiel 17 aus N-Thymin-1-yl-acetyl-N-(2-hydroxy)ethylaminomethanphosphonsäurediethylester (Beispiel 10) und N-Thymin-1-yl-acetyl-N-(4-methoxytriphenylmethoxy)ethylaminomethanphosphonsäuremonophenylester (Triethylammoniumsalz) (Beispiel 12). Zur Reinigung wurde über Kieselgel chromatographiert (EE/Methanol/TEA 93/7/2). Ausbeute: 58%.
   MS (FAB): 1051.4 (M+Na)⁺; 1029.5 (M+H)⁺. ¹H-NMR (200MHz, DMSO, TMS) Characteristische Signale sind: Ar-H & Thymin-H: 6.82-7.53 (m, 21H); CO-CH₂: 4.52-4.82 (m, 4H); Thymin-CH₃: 1.62-1.80 (m, 6H).
20) N-Thymin-1-yl-acetyl-N-(2-hydroxyethyl)aminomethanphosphonsäuredi(4-nitrophenylethyl)ester
   Die Synthese erfolgte analog Beispiel 4 aus N- Thymin-1-yl-acetyl-N-(4-methoxytriphenylmethoxy)ethylaminomethanphosphonsäuredi(2-(p-nitrophenyl)ethyl)ester (Beispiel 14). Zur Reinigung wurde über Kieselgel chromatographiert (EE/Methanol 90/10). Ausbeute: 85%.
   MS (ES+): 620.3 (M+H)⁺. ¹H-NMR (500MHz, DMSO, TMS): δ = 1.73 (s, 3H, T-CH₃); 2.97 (t, 4H, P-O-CH₂-CH₂-Ar); 3.41 (m, 2H, N-CH₂); 3.59 (m, 2H, CH₂-OH); 3.83 (d, 2H, J=11Hz; P-CH₂); 4.08-4.30 (m, 4H, P-O-CH₂); 4.54 & 4.78 (jew.s, breit, 2H, CO-CH₂); 4.99 (t, 1H, OH); 7.14-8.19 (m, 9H, Ar-H, Thymidinyl-H); 11.30 (s breit, 1H, NH).
21) 5'-MMTr-T-P(ONPE)-T-P(OEthyl)₂
   Die Synthese erfolgte analog Beispiel 17 aus N-Thymin-1-yl-acetyl-N-(2-hydroxy)ethylaminomethanphosphonsäurediethylester (Beispiel 10) und N-Thymin-1-yl-acetyl-N-(4-methoxytriphenylmethoxy)ethylaminomethanphosphonsäure-(4-nitrophenylethyl)monoester (Triethylammoniumsalz) (Beispiel 15). Anstelle von TSNT wurde 3-Nitro-1-(2,4,6 Triisopropylphenyl-sulfonyl)-1H-1,2,4-triazol (TIPSNT) zur Kupplung eingesetzt. Zur Reinigung wurde über Kieselgel chromatographiert (EE/Methanol/TEA 95/5/2, dann 90/10/2). Ausbeute: >90%.
   MS (ES+): 1109.0 (M+Li)⁺. ¹H-NMR (200MHz, DMSO, TMS) Characteristische Signale sind: Ar-H & Thymin-H: 6.82-8.18 (m, 20H); CO-CH₂: 4.51-4.76 (m, 4H); Thymin-CH₃: 1.61-1.78 (m, 6H).
22) 5'-MMTr-T-P(ONPE)-T-P(OEt)₂
   Die Synthese erfolgte analog Beispiel 21 aus N-Thymin-1-yl-acetyl-N-(2-hydroxy)ethylaminomethanphosphonsäurediethylester (Beispiel 10) und N-Thymin-1-yl-acetyl-N-(4-methoxytriphenylmethoxy)ethylaminomethanphosphonsäure-(4-nitrophenylethyl)monoester (Triethylammoniumsalz) (Beispiel 15). Anstelle von TSNT wurde 3-Nitro-1-(2,4,6 Triisopropylphenyl-sulfonyl)-1H-1,2,4-triazol (TIPSNT) zur Kupplung eingesetzt. Ausbeute: >90%. Spektroskopische Daten siehe Beispiel 21.
23) 5'-HO-T-P(ONPE)-T-P(OEt)₂
   Die Synthese erfolgte analog Beispiel 4 aus "5'-MMTr-T-P(ONPE)-T-P(OEt)₂" (Beispiel 22). Zur Reinigung wurde über Kieselgel chromatographiert (EE/Methanol/TEA 90/10/2, dann 80/20/2). Die Ausbeute betrug 75%.
   MS (ES+): 836.3 (M+Li)⁺. ¹H-NMR (200MHz, DMSO, TMS) Characteristische Signale sind: Ar-H & Thymin-H: 7.11-8.22 (m, 6H); CO-CH₂: 4.55-4.77 (m, 4H); Thymin-CH₃: 1.71 (s, breit, 6H).
24) 5'-HO-T-P(OH)-T-P(OEthyl)₂
   10mg (0.012mMol) "5'-HO-T-P(ONPE)-T-P(OEt)₂" (Beispiel 23) wurden in 1ml einer 0.5M Lösung DBU in Pyridin gelöst und zunächst 24 h bei 4°C, dann 24h bei Raumtemperatur gerührt. Das Lösungsmittel wurde im Vakuum abgedampft, der Rückstand zweimal mit Pentan, dann zweimal mit Ether digeriert, dann wurde zur Reinigung über Kieselgel chromatographiert (EE/Methanol/TEA 9/1/0.2, dann 70/30/2, dann 60/40/2). Ausbeute: 10.2 mg.
   MS (FAB): 725.3 (M+2Na-H)⁺; 703.3 (M+Na)⁺. ¹H-NMR (200MHz, DMSO, TMS) Characteristische Signale sind: Thymin-H: 7.15-7.70 (m, 2H); CO-CH₂: 4.67-4.92 (m, 4H); Thymin-CH₃: 1.67-1.81 (m, 6H).
25) 5'-MMTr-T-P(ONP)-T-P(ONPE)-T-P(OEt)₂
   Die Synthese erfolgte analog Beispiel 17 aus "5'-HO-T-P(ONPE)-T-P(OEt)₂" (Beispiel 22) und N-Thymin-1-yl-acetyl-N-(4-methoxytriphenylmethoxy)-ethylaminomethanphosphonsäure-(4-nitrophenylethyl)monoester (Triethylammoniumsalz) (Beispiel 15) unter Zusatz von 1.5 eq (bezogen auf Beispiel 23) 4-Methoxypyridin-N-oxid. Zur Reinigung wurde über Kieselgel chromatographiert (EE/Methanol/TEA 90/10/2, dann 85/15/2). Ausbeute: 61%.
   MS (ES+): 1555.8 (M+H)⁺. ¹H-NMR (200MHz, DMSO, TMS) Characteristische Signale sind: Ar-H & Thymin-H: 6.83-8.20 (m, 25H); CO-CH₂: 4.52-4.75 (m, 6H); Thymin-CH₃: 1.61-1.78 (m, 9H).
26) 5'-HO-T-P(ONPE)-T-P(ONPE)-T-P(OEt)₂
   Die Synthese erfolgte analog Beispiel 4 aus "5'-MMTr-T-P(ONPE)-T-P(ONPE)-T-P(OEt)₂" (Beispiel 25). Zur Reinigung wurde über Kieselgel chromatographiert (EE/Methanol/TEA 70/30/2). Die Ausbeute betrug 89%.
   MS (ES+): 1283.1 (M+H)⁺; 1305.0 (M+Na⁺).
27) 5'-MMTr-T-P(ONPE)-T-P(ONPE)- T-P(ONPE)-T-P(OEt)₂
   Die Synthese erfolgte analog Beispiel 17 aus "5'-HO-T-P(ONPE)-T-P(ONPE)-T-P(OEt)₂" (Beispiel 26) und N-Thymin-1-yl-acetyl-N-(4-methoxytriphenylmethoxy)-ethylaminomethanphosphonsäure-(4-nitrophenylethyl)monoester (Triethylammoniumsalz) (Beispiel 15 ) unter Zusatz von 1.5 eq (bezogen auf Beispiel 23) 4-Methoxypyridin-N-oxid. Zur Reinigung wurde über Kieselgel chromatographiert (EE/Methanol/TEA 90/10/2, dann 80/20/2). Ausbeute: 15%.
   MS (ES+): 2007 (M+H)⁺; 2029 (M+Na)⁺. ¹H-NMR (200MHz, DMSO, TMS) Characteristische Signale sind: Ar-H & Thymin-H: 6.79-8.21 (m, 30H); CO-CH₂: 4.53-4.87 (m, 8H); Thymin-CH₃: 1.58-1.89 (m, 12H).
28) 5'-HO-T-P(ONPE)-T-P(ONPE)- T-P(ONPE)-T-P(OEt)₂
   Die Synthese erfolgte analog Beispiel 4 aus "5'-MMTr-T-P(ONPE)-T-P(ONPE)- T-P(ONPE)-T-P(OEt)₂" (Beispiel 27). Zur Reinigung wurde über Kieselgel chromatographiert (EE/Methanol/ TEA 70/30/2). Die Ausbeute betrug 55%. MS (FAB): 1735 (M+H)⁺; 1757 (M+Na)⁺.
29) 5'-MMTr-T-P(ONPE)-T-P(ONPE)₂
   Die Synthese erfolgte analog Beispiel 17 aus N-Thymin-1-yl-acetyl-N-(2-hydroxyethyl)aminomethanphosphonsäuredi(4-nitrophenylethyl)ester (Beispiel 20) und N-Thymin-1-yl-acetyl-N-(4-methoxytriphenylmethoxy)ethylaminomethanphosphonsäure-(4-nitrophenylethyl)monoester (Triethylammoniumsalz) (Beispiel 15). Zur Reinigung wurde über Kieselgel chromatographiert (EE/Methanol/TEA 100/0/1, dann 90/10/1). Ausbeute: 87%.
   MS (FAB): 1356.2 (M+2Li-H)⁺. ¹H-NMR (200MHz, DMSO, TMS) Characteristische Signale sind: Ar-H & Thymin-H: 6.82-8.18 (m, 28H); CO-CH₂: 4.50-4.71 (m, 4H); Thymin-CH₃: 1.59-1.78 (m, 6H).
30) 5'-HO-T-P(ONPE)-T-P(ONPE)₂
   Die Synthese erfolgte analog Beispiel 4 aus "5'-MMTr-T-P(ONPE)-T-P(ONPE)₂" (Beispiel 29). Zur Reinigung wurde über Kieselgel chromatographiert (EE/Methanol/ TEA 85/15/1, dann 80/20/1). Die Ausbeute betrug 78%.
   MS (ES+): 1072.7 (M+H)⁺. ¹H-NMR (200MHz, DMSO, TMS) Characteristische Signale sind: Ar-H & Thymin-H: 7.08-8.20 (m, 14H); CO-CH₂ 4.52-4.80 (m, 4H); Thymin-CH₃: 1.70 (s, breit, 6H).
31)5'-MMTr-C^{An}-P(ONPE)-C^{An}-P(ONPE)-C^{An}-P(ONPE)₂
   Die Synthese erfolgte analog Beispiel 17 aus N-(N⁶-Anisoyl)cytosin-1-yl-acetyl-N-(4-methoxytriphenylmethoxy)ethylaminomethanphosphonsäure(2-(p-nitrophenyl)ethyl)monoester (Triethylammoniumsalz) (Beispiel 3) und "5'-HO-C^{An}-P(ONPE)-C^{An}-P(ONPE)₂" (Beispiel 6). Zur Reinigung wurde über Kieselgel chromatographiert (EE/Methanol/TEA 80/19/1). Ausbeute: 66%.
   MS (FAB): 2155 (M+H)⁺; 2161 (M+Li)⁺; 2177 (M+Na)⁺.
32) 5'-HO-C^{An}-P(ONPE)-C^{An}-P(ONPE)-C^{An}-P(ONPE)₂
   Die Synthese erfolgte analog Beispiel IV aus "5'-MMTr-C^{An}-P(ONPE)-C^{An}-P(ONPE)-C^{An}-P(ONPE)₂" (Beispiel 31). Zur Reinigung wurde über Kieselgel chromatographiert (EE/Methanol/ TEA 85/15/1, dann 80/20/1). Die Ausbeute betrug 70%.
   MS (FAB): 1882 (M+H)⁺; 1904 (M+Na)⁺.
33) N-(N⁶-Anisoyl)cytosin-1-yl-acetyl-N-(4-methoxytriphenylmethoxy)ethylaminomethanphosphonsäure-allyl-(2-(p-nitrophenyl)ethyl)diester
   Die Synthese erfolgte analog Beispiel 17 aus N-(N⁶-Anisoyl)cytosin-1-yl-acetyl-N-(4-methoxytriphenylmethoxy)ethylaminomethanphosphonsäure(2-(p-nitrophenyl)ethyl)monoester (Triethylammoniumsalz) (Beispiel 3) und Allylalkohol. Zur Reinigung wurde über Kieselgel chromatographiert (EE/Methanol/TEA 95/5/1).
   MS (ES+): 902.1 (M+H)⁺; 924.1 (M+Na)⁺. ¹H-NMR (200MHz, DMSO, TMS): δ= 2.94-3.70 (m, 8H, P-O-CH₂-CH₂-Ar + MMTr-O-CH₂ + N-CH₂ + P-CH₂); 3.75 (s, 3H, OCH₃); 3.86 (s, 3H, OCH₃); 4.10-4.60 (m, 4H, P-O-CH₂); 4.79 & 4.84 (je s, breit, 2H, CO-CH₂); 5.09-5.39 (m, 2H, H₂C=CH-); 5.71-6.00 (m, 1H, H₂C=CH-); 6.83-8.19 (m, 24H, Ar-H, Cytosinyl-H); 11.03 (s breit, 1H, NH).
34) N-(N⁶-Anisoyl)cytosin-1-yl-acetyl-N-(2-hydroxy)ethylaminomethanphosphonsäure-allyl-(2-(p-nitrophenyl)ethyl)diester
   Die Synthese erfolgte analog Beispiel 4 aus N-(N⁶-Anisoyl)cytosin-1-yl-acetyl-N-(4-methoxytriphenylmethoxy)ethylaminomethanphosphonsäure-allyl-(2-(p-nitrophenyl)ethyl)diester (Beispiel 33). Zur Reinigung wurde über Kieselgel chromatographiert (EE/Methanol/ TEA 94/5/1). Die Ausbeute betrug 83%.
   MS (ES+): 630.2 (M+H)⁺. ¹H-NMR (200MHz, DMSO, TMS): δ = 3.02 (t, 2H, P-O-CH₂-CH₂-Ar); 3.37-3.72 (m, 4H, HO-CH₂-CH₂); 3.86 (s, 3H, OCH₃); 3.91 (d, J=11Hz, 2H, P-CH₂); 4.22 (dt, 2H, P-O-CH₂-CH₂-Ar); 4.40 (dd, 2H, O-CH₂-CH=CH₂); 4.78 & 5.01 (m, 2H, CO-CH₂); 5.11-5.33 (m, 2H, H₂C=CH-); 5.71-6.00 (m, 1H, H₂C =CH-); 6.99-8.21 (m, 14H, Ar-H, Cytosinyl-H); 11.03 (s breit, 1H, NH).
35) N- Thymin-1-yl-acetyl-N-(4-methoxytriphenylmethoxy)ethylaminomethanphosphonsäure allyl-(2-(p-nitrophenyl)ethyl)diester
   Synthese analog Beispiel 17 aus N-Thymin-1-yl-acetyl-N-(4-methoxytriphenylmethoxy)ethylaminomethanphosphonsäure-(4-nitrophenylethyl)monoester (Triethylammoniumsalz, Beispiel 15) und Allylalkohol. Zur Reinigung wurde über Kieselgel chromatographiert (EE/Methanol/ TEA 97/3/2). Die Ausbeute betrug 100%.
   MS (FAB): 805.3 (M+Na)⁺.
36) N- Thymin-1-yl-acetyl-N-(2-hydroxy)ethylaminomethanphosphonsäure allyl-(2-(p-nitrophenyl)ethyl)diester
   Synthese analog Beispiel 4 aus N- Thymin-1-yl-acetyl-N-(4-methoxytriphenylmethoxy)ethylaminomethanphosphonsäure allyl-(2-(p-nitrophenyl)ethyl)diester (Beispiel 35). Zur Reinigung wurde über Kieselgel chromatographiert (EE/Methanol/ TEA 90/10/2). Die Ausbeute betrug 86%.
   MS (ES+): 511.1 (M+H)⁺
37) 5'-MMTr-T-P(ONPE)-T-P(ONPE)(OAllyl)
   Synthese analog Beispiel 17 aus N- Thymin-1-yl-acetyl-N-(2-hydroxy)ethylaminomethanphosphonsäure allyl-(2-(p-nitrophenyl)ethyl)diester (Beispiel 36) und N-Thymin-1-yl-acetyl-N-(4-methoxytriphenylmethoxy)ethylaminomethanphosphonsäure-(4-nitrophenylethyl)monoester (Triethylammoniumsalz) (Beispiel 15). Zur Reinigung wurde über Kieselgel chromatographiert (EE/Methanol/TEA 90/10/2). Ausbeute: 90%. MS (FAB): 1257.3 (M+Na)⁺.
38) 5'-MMTr- T-P(ONPE)-T-P(ONPE)-T-P(ONPE)- T-P(ONPE)-T-P(OEt)₂
   Synthese analog Beispiel 17 aus "5'-HO- T-P(ONPE)-T-P(ONPE)-T-P(ONPE)-T-P(OEt)₂" (Beispiel 28) und N-Thymin-1-yl-acetyl-N-(4-methoxytriphenylmethoxy)ethylaminomethanphosphonsäure-(4-nitrophenylethyl)monoester (Triethylammoniumsalz) (Beispiel 15). Zur Reinigung wurde über Kieselgel chromatographiert (EE/Methanol/TEA 80/20/2). Ausbeute: 57%.
   MS (FAB): 2460 (M+H)⁺; 2482 (M+Na)⁺.
39) 5'-HO- T-P(ONPE)-T-P(ONPE)-T-P(ONPE)- T-P(ONPE)-T-P(OEt)₂
   Synthese analog Beispiel 4 aus "5'-MMTr- T-P(ONPE)-T-P(ONPE)-T-P(ONPE)- T-P(ONPE)-T-P(OEt)₂" (Beispiel 38). Zur Reinigung wurde über Kieselgel chromatographiert (EE/Methanol/ TEA 70/30/2). Die Ausbeute betrug 55%.
   MS (FAB): 2209 (M+Na)⁺.
   40) 5'-HO- T-P(OH)-T-P(OH)-T-P(OH)- T-P(OH)-T-P(OEt)₂
   4.0 mg (0.00183mMol) "5'-HO- T-P(ONPE)-T-P(ONPE)-T-P(ONPE)- T-P(ONPE)-T-P(OEt)₂" (Beispiel 39) wurden in 1.1 ml einer 0.5 molaren Lösung von DBU in Pyridin gelöst und 24 h bei Raumtemperatur gerührt. Das Reaktionsgemisch wurde im Vakuum eingedampft, der Rückstand mehrfach mit Toluol verrührt. Das Lösungsmittel wurde mit einer Spritze entfernt, der Rückstand nochmals mit Pentan verrührt, das Lösungsmittel wieder mit einer Spritze entfernt. Das Produkt wurde im Vakuum getrocknet. Die Ausbeute betrug 4mg eines stark hygroskopischen Pulvers. MS (ES-): 1589.7 (M-H⁻)⁻; 1611.8 (M+Na-2H)⁻.
41) 5'-MMTr-T-P(ONPE)-T-P(ONPE)-T-P(ONPE)-T-P(ONPE)- T-P(ONPE)-T-P(OEt)₂
   a) Synthese analog Beispiel 17 aus "5'-HO-T-P(ONPE)-T-P(ONPE)-T-P(ONPE)-T-P(ONPE)-T-P(OEt)₂" (Beispiel 39) und N-Thymin-1-yl-acetyl-N-(4-methoxytriphenylmethoxy)ethylaminomethanphosphonsäure-(4-nitrophenylethyl)monoester (Triethylammoniumsalz) (Beispiel 15). Zur Reinigung wurde über Kieselgel chromatographiert (EE/Methanol/TEA 80/20/2, dann 70/30/2). MS (FAB): 2934 (M+Na)⁺, 2957 (M+2Na-H)⁺, 2978 (M+3Na-2H)⁺.
   b) Synthese analog Beispiel 17 aus "5'-HO-T-P(ONPE)-T-P(ONPE)- T-P(ONPE)-T-P(OEt)₂" (Beispiel 28) und "5'-MMTr-T-P(ONPE)-T-P(ONPE)(OH)" (Beispiel 42). Zur Reinigung wurde über Kieselgel chromatographiert (EE/Methanol/TEA 80/20/2, dann 70/30/2). Die Zielfraktion wurde im Vakuum eingedampft, dann mit Pentan und Ether verrieben. MS wie oben.
42) 5'-MMTr-T-P(ONPE)-T-P(ONPE)(OH)
   24.7 mg (0.02 mmol) "5'-MMTr-T-P(ONPE)-T-P(ONPE)(OAllyl)" (Beispiel 37) wurden zusammen mit 16.2 mg (0.12 mmol) Diethylammoniumhydrogencarbonat in 2 ml absol. DCM gelöst. Bei 15-20 °C wurde eine Lösung von 13.9 mg (0.012 mMol) Tetrakis(triphenylphosphin)-palladium (0) und 2.1 mg (0.008 mMol) Triphenylphosphin in 2 ml absol. DCM während 2 min zugetropft. Es wurde 30 min bei Raumtemp. gerührt. Zur Reinigung wurde das Reaktionsgemisch über Kieselgel chromatographiert (EE/Methanol/TEA 80/20/1, dann 60/40/1). Die Produktfraktion wurde im Vakuum eingedampft, der Rückstand mit Pentan, dann mit EE/Ether, dann wieder mit Pentan verrieben und im Vakuum getrocknet.
   Ausbeute: 57%.
   MS (ES-): 1193.6 (M-H)⁻.
   ¹H-NMR (200MHz, DMSO, TMS): Charakteristische Signale: δ =; 1.67 & 1.72 (je. s, 3H, T-CH₃); 4.60 & 4.82 (jew. s, 2H, CO-CH₂); 6.83-8.19 (m, 24H, Ar-H, T-H);
43) 5'-HO-T-P(ONPE)-T-P(ONPE)-T-P(ONPE)-T-P(ONPE)- T-P(ONPE)-T-P(OEt)₂
   Synthese analog Beispiel 4 aus "5'-MMTr-T-P(ONPE)-T-P(ONPE)-T-P(ONPE)-T-P(ONPE)- T-P(ONPE)-T-P(OEt)₂" (Beispiel 41). Nach erfolgter Reaktion wurde das Reaktionsgemisch eingeengt, der Rückstand dreimal mit Toluol koevaporiert, dann zuerst mit EE/Ether, danach mit Pentan verrührt. Der Rückstand wurde im Vakuum getrocknet.
   MS (FAB): 2662 (M+Na)⁺.
   44) 5'-HO-T-P(ONPE)-T-P(ONPE)(OAllyl)
   Synthese analog Beispiel 4 aus 5'-MMTr-T-P(ONPE)-T-P(ONPE)(OAllyl) (Beispiel 37). Zur Reinigung wurde über Kieselgel chromatographiert (EE/Methanol/ TEA 90/10/1, dann 80/20/1). Die Ausbeute betrug 87%.
   MS (FAB): 963.0 (M+H)⁺; 985.1 (M+Na)⁺.
45) 5'-MMTr-T-P(ONPE)-T-P(ONPE)-T-P(ONPE)(OH)
   a) 5'-MMTr-T-P(ONPE)-T-P(ONPE)-T-P(ONPE)(OAllyl)
      Synthese analog Beispiel 17 aus "5'-HO-T-P(ONPE)-T-P(ONPE)(OAllyl)" (Beispiel 44) und N-Thymin-1-yl-acetyl-N-(4-methoxytriphenylmethoxy)ethylaminomethanphosphonsäure-(4-nitrophenylethyl)monoester (Triethylammoniumsalz) (Beispiel 15). Zur Reinigung wurde über Kieselgel chromatographiert (EE/Methanol/TEA 90/10/1, dann 85/15/1). Ausbeute:55%
   b) 5'-MMTr-T-P(ONPE)-T-P(ONPE)-T-P(ONPE)(OH)

   "5'-MMTr- T-P(ONPE)-T-P(ONPE)-T-P(ONPE)(OAllyl)" (Beispiel 45a) wurde wie in Beispiel 42 beschrieben mit Tetrakis(triphenylphosphin)palladium (O) umgesetzt. Zur Reinigung wurde über Kieselgel chromatographiert (EE/Methanol/TEA 80/20/2, dann 70/30/2). Die Produktfraktion wurde im Vakuum eingedampft, der Rückstand mit Pentan und Ether verrieben. Ausbeute: 98%.
   MS (ES+; LiCl): 1654.1 (M+Li⁺)
46) 5'-MMTr-T-P(ONPE)-T-P(ONPE)- T-P(ONPE)-T-P(OEt)₂
   Synthese analog Beispiel 17 aus N-Thymin-1-yl-acetyl-N-(2-hydroxy)ethylaminomethanphosphonsäurediethylester (Beispiel 10) und "5'-MMTr-T-P(ONPE)-T-P(ONPE)-T-P(ONPE)(OH)" (Beispiel 45b). Zur Reinigung wurde über Kieselgel chromatographiert (EE/Methanol/TEA 90/10/2, dann 80/20/2). Aufarbeitung, Reinigung und Charakterisierung wie in Beispiel 27.
47) 5'-MMTr- T-P(ONPE)-T-P(ONPE)-T-P(ONPE)-T-P(ONPE)- T-P(ONPE)-T-P(OEt)₂
   Synthese analog Beispiel 17 aus "5'-HO-T-P(ONPE)-T-P(ONPE)-T-P(OEt)₂" (Beispiel 26) und "5'-MMTr-T-P(ONPE)-T-P(ONPE)-T-P(ONPE)(OH)" (Beispiel 45b). Zur Reinigung wurde über Kieselgel chromatographiert (EE/Methanol/TEA 80/20/2). Die Produktfraktion wurde im Vakuum eingedampft, mit Toluol koevaporiert und durch präparative HPLC (High Pressure Liquid Chromatography) gereinigt: RP8 LiChrospher60, Wasser/Acetonitril/ : 1/1; 0.1% Ammoniumacetat; 1ml/min) R_{f}= 12.97 min
48) 5'-HO- T-P(OH)-T-P(OH)-T-P(OH)-T-P(OH)- T-P(OH)-T-P(OEt)₂
   a) 5'-HO- T-P(ONPE)-T-P(ONPE)-T-P(ONPE)-T-P(ONPE)- T-P(ONPE)-T-P(OEt)₂
      Synthese analog Beispiel 4 aus "5'-MMTr-T-P(ONPE)-T-P(ONPE)-T-P(ONPE)-T-P(ONPE)- T-P(ONPE)-T-P(OEt)₂" (Beispiel 47). Zur Reinigung wurde über Kieselgel chromatographiert (EE/Methanol/TEA 70/30/2, dann 60/40/2). Die Produktfraktion wurde im Vakuum eingedampft, der Rückstand zuerst mit Pentan, dann mit Ether verrührt und im Vakuum getrocknet. Ausbeute: 100%.
      MS (FAB): 2662 (M+Na)⁺, 2684 (M + 2Na-H) +, 2706 (M + 3Na-2H) +.
   b) 5'-HO- T-P(OH)-T-P(OH)-T-P(OH)-T-P(OH)- T-P(OH)-T-P(OEt)₂

   "5'-HO- T-P(ONPE)-T-P(ONPE)-T-P(ONPE)-T-P(ONPE)- T-P(ONPE)-T-P(OEt)₂" (Beispiel 48a) wurde analog Beispiel 40 mit DBU umgesetzt und aufgearbeitet.
   MS (ES-): 1892 (M-H⁻)⁻; 1915 (M+Na-2H)⁻.
49) 5'-MMTr- T-P(ONPE)-T-P(ONPE)-T-P(ONPE)-T-P(ONPE)-T-P(ONPE)- T-P(ONPE)-T-P(OEt)₂
   Synthese analog Beispiel 17 aus "5'-HO- T-P(ONPE)-T-P(ONPE)-T-P(ONPE)-T-P(OEt)₂" (Beispiel 28) und "5'-MMTr-T-P(ONPE)-T-P(ONPE)-T-P(ONPE)(OH)" (Beispiel 45b). Zur Reinigung wurde über Kieselgel chromatographiert (EE/Methanol/TEA 80/20/2, dann 70/30/2). Die Produktfraktion wurde im Vakuum eingedampft, mit Toluol koevaporiert und durch präparative HPLC gereinigt: RP8 LiChrospher60, Wasser/Acetonitril/ : 1/1; 0.1 % Ammoniumacetat; 1ml/min) R_{f}= 15.24 min
   MS (FAB): 3386 (M+Na)⁺, 3409 (M+2Na-H)⁺,
50) 5'-HO- T-P(ONPE)-T-P(ONPE)-T-P(ONPE)-T-P(ONPE)-T-P(ONPE)- T-P(ONPE)-T-P(OEt)₂
   Synthese analog Beispiel 4 aus "5'-MMTr- T-P(ONPE)-T-P(ONPE)-T-P(ONPE)-T-P(ONPE)-T-P(ONPE)- T-P(ONPE)-T-P(OEt)₂" (Beispiel 49). Zur Reinigung wurde im Vakuum eingedampft, dreimal mit Toluol koevaporiert, der Rückstand zuerst mit Pentan, dann mit Ether verrührt und im Vakuum getrocknet. Ausbeute: > 90%.
   MS (FAB): 3114 (M+Na)⁺.
51) 5'-HO- T-P(OH)-T-P(OH)-T-P(OH)-T-P(OH)-T-P(OH)- T-P(OH)-T-P(OEt)₂
   "5'-HO- T-P(ONPE)-T-P(ONPE)-T-P(ONPE)-T-P(ONPE)-T-P(ONPE)- T-P(ONPE)-T-P(OEt)₂" (Beispiel 50) wurde analog Beispiel 40 mit DBU umgesetzt und aufgearbeitet.
   MS (ES-): 2196 (M-H⁻)⁻; 2218 (M+Na-2H)⁻.
52) 5'-MMTr- T-P(ONPE)-T-P(ONPE)-T-P(ONPE)-T-P(ONPE)-T-P(ONPE)-T-P(ONPE)-T-P(ONPE)- T-P(ONPE)-T-P(OEt)₂
   Synthese analog Beispiel 17 aus "5'-HO- T-P(ONPE)-T-P(ONPE)-T-P(ONPE)-T-P(ONPE)- T-P(ONPE)-T-P(OEt)₂" (Beispiel 48a) und "5'-MMTr-T-P(ONPE)-T-P(ONPE)-T-P(ONPE)(OH)" (Beispiel 45b). Zur Reinigung wurde über Kieselgel chromatographiert (EE/Methanol/TEA 70/30/2, dann 60/40/2). Die
   Produktfraktion wurde im Vakuum eingedampft, mit Toluol koevaporiert und durch präparative HPLC gereinigt: RP8 LiChrospher60, Wasser/Acetonitril/ : 1/1; 0.1% Ammoniumacetat; 1ml/min) R_{f}= 23.95 min
53) 5'-HO- T-P(OH)-T-P(OH)-T-P(OH)-T-P(OH)-T-P(OH)-T-P(OH)-T-P(OH)- T-P(OH)-T-P(OEt)₂
   a) 5'-HO- T-P(ONPE)-T-P(ONPE)-T-P(ONPE)-T-P(ONPE)-T-P(ONPE)-T-P(ONPE)-T-P(ONPE)- T-P(ONPE)-T-P(OEt)₂
      Synthese analog Beispiel 4 aus "5'-MMTr- T-P(ONPE)-T-P(ONPE)-T-P(ONPE)-T-P(ONPE)-T-P(ONPE)-T-P(ONPE)-T-P(ONPE)- T-P(ONPE)-T-P(OEt)₂" (Beispiel 52). Zur Reinigung wurde im Vakuum eingedampft, dreimal mit Toluol koevaporiert, der Rückstand zuerst mit Pentan, dann mit Ether verrührt und im Vakuum getrocknet. Ausbeute: > 90%.
   b) 5'-HO- T-P(OH)-T-P(OH)-T-P(OH)-T-P(OH)-T-P(OH)-T-P(OH)-T-P(OH)- T-P(OH)-T-P(OEt)₂
   "5'-HO- T-P(ONPE)-T-P(ONPE)-T-P(ONPE)-T-P(ONPE)-T-P(ONPE)-T-P(ONPE)-T-P(ONPE)- T-P(ONPE)-T-P(OEt)₂" (Beispiel 53a) wurde analog Beispiel 40 mit DBU umgesetzt und aufgearbeitet.
   MS (ES-): 2802 (M-H-)-; 2825 (M+Na-2H)⁻.
54) 2- ( N'-tert.-8utyloxycarbonylamino)ethylaminomethanphosphonsäuredi(2-(p-nitrophenyl)ethyl)ester
   a) 1-Methylimino-2-(N'- tert.-Butyloxycarbonylamino)ethan (Trimer)
      2.0g (12.5 mmol) 2-amino-1-(N'- tert.-Butyloxycarbonylamino)ethan, gelöst in 8 ml Methanol wurden unter Eiskühlung mit 1.52 ml (18.72 mmol) 37% Formaldehyd versetzt und 1 h bei Raumtemp. gerührt. Der Rückstand wurde in EE aufgenommen, zweimal mit gesättigter NaHC03-Lösung, dann mit NaCl-Lösung gewaschen, getrocknet, filtriert und im Vakuum eingedampft. Zur Reinigung über Kieselgel chromatographiert (EE/TEA 100/0.2, dann EE/Methano/TEA 90/10/0.2). Die Ausbeute betrug 0.8 g.
      MS (FAB/LiCl) 523.4 (M+2Li-H)⁺. ¹H-NMR (200MHz, DMSO, TMS): δ = 1.38 (s, 27H, tBu-H); 2.40 (t, 6H, N-CH₂); 2.99 (t, 6H, N-CH₂); 3.25 (t, 6H, N-CH₂); 6.81 (t, breit, 3H, NH).
   b) 2-( N'-tert.-Butyloxycarbonylamino)ethylaminomethanphosphonsäuredi(2-(p-nitrophenyl)ethyl)ester

   1-Methylimino-2-(N'- tert.-Butyloxycarbonylamino)ethan (Trimer) (Beispiel 54a) wurde analog Beispiel 1f mit Di(2-(4-nitrophenyl)ethyl)phosphit (Beispiel 1e) umgesetzt. Zur Reinigung wurde über Kieselgel chromatographiert (zuerst Toluol/EE/TEA 20/80/0.2; dann EE/TEA 100/0.2, dann EE/Methanol/TEA 0.5/5/0.2). Ausbeute: 25%.
   MS(ES+/LiCl) 553.3 (M+H)⁺, 559.3 (M+Li)⁺. ¹H-NMR (200MHz, DMSO, TMS): δ = 1.37 (s, 9H, tBu-H); 2.83 (d, J=12Hz, 2H, P-CH₂); 2.55 (t, 4H, Ar-CH₂); 2.90-3.09 (m, 4H, N-CH₂- CH₂-N); 4.16 (dt, 4H, PO-CH₂); 7.52&8.15 (je. d, 8H, Ar-H).

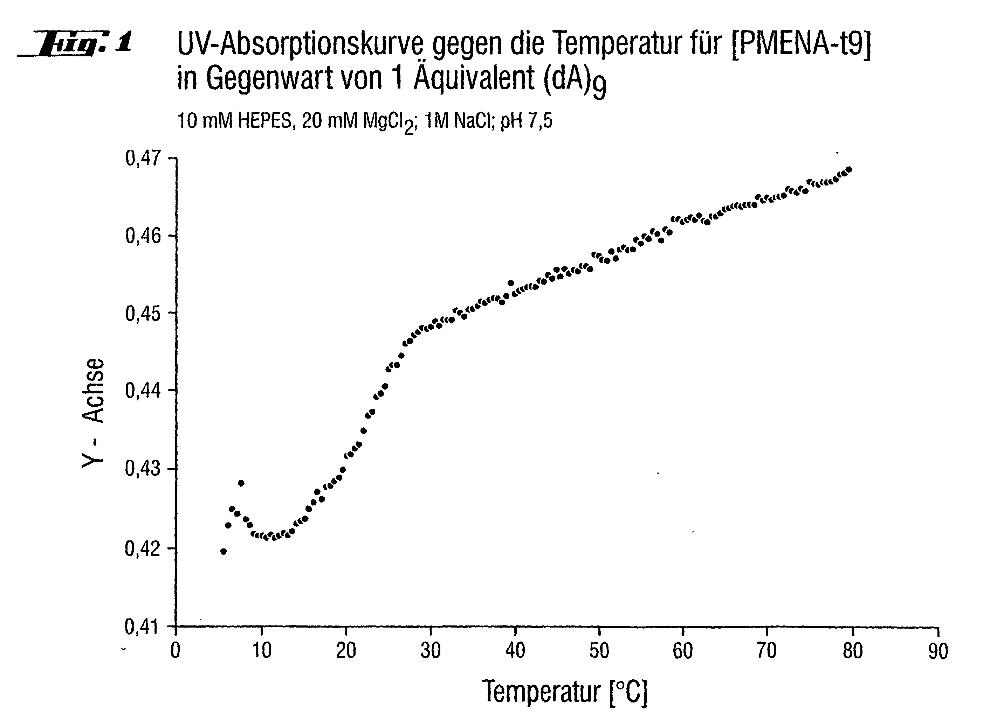

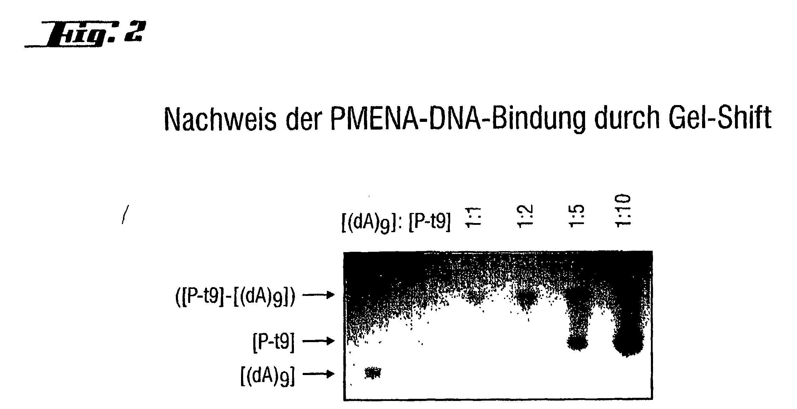

55) N- Thymin-1-yl-acetyl-N- (2-N'-tert.-Butyloxycarbonylamino)ethylaminomethanphosphonsäuredi(2-(p-nitrophenyl)ethyl)ester
   Synthese analog Beispiel 8 aus 2-( N'-tert.-Butyloxycarbonylamino)ethylaminomethanphosphonsäuredi(2-(p-nitrophenyl)ethyl)ester (Beispiel 54b) und Tymidin-1-yl-essigsäure. Ausbeute: 86%
   MS (FAB/LiCl): 725.3 (M+Li)⁺. ¹H-NMR (200MHz, DMSO, TMS): δ = 1.42 (s, 9H, tBu-H); 1.91 (s, 3H, T-CH₃); 2.99-3.58 (m, 8H, P-O-CH₂-CH₂-Ar & N-CH₂-CH₂-N);3.75 (d, J=12Hz, 2H, P-CH₂); 4.06-4.38 (m, 4H, PO-CH₂); 7.37 & 8.15 (je. d, 8H, Ar-H).
56) Wechselwirkung mit DNA: UV-Schmelzkurve
   Die Wechselwirkung der erfindungsgemäßen Verbindungen mit komplementärer Nucleinsäuren wurde beispielhaft durch die Aufzeichnung der UV-Schmelzkurve von "5'-HO- T-P(OH)-T-P(OH)-T-P(OH)-T-P(OH)-T-P(OH)-T-P(OH)-T-P(OH)- T-P(OH)-T-P(OEt)₂" (Beispiel 53b) mit (dA)₉ demonstriert. Dazu wurde eine jeweils 0.3 OD von "5'-HO- T-P(OH)-T-P(OH)-T-P(OH)-T-P(OH)-T-P(OH)-T-P(OH)-T-P(OH)- T-P(OH)-T-P(OEt)₂" und (dA)₉ in 1 ml eines Puffers (1 M NaCI, 20 mM MgCl₂, 10 mM HEPES,.pH 7.5) hergestellt und die Änderung der Extinktion bei 260 nm in Abhängigkeit von der Temperatur (0 bis aufgezeichnet. Das Ergebnis ist in FIG. 1 zu sehen. Aus der erhaltenen Schmelzkurve wurde ein Tₘ-Wert von ca. 23 °C ermittelt.
57) Wechselwirkung mit DNA: Gelshift-Experiment
   Die Wechselwirkung der erfindungsgemäßen Verbindungen mit komplementärer Nucleinsäuren wurde beispielhaft durch die Hybridisierung von "5'-HO- T-P(OH)-T-P(OH)-T-P(OH)-T-P(OH)-T-P(OH)-T-P(OH)-T-P(OH)- T-P(OH)-T-P(OEt)₂" (Beispiel 53b) mit (dA)₉ in einem Gelshift-Experiment demonstriert. Dazu wurden "5'-HO- T-P(OH)-T-P(OH)-T-P(OH)-T-P(OH)-T-P(OH)-T-P(OH)-T-P(OH)- T-P(OH)-T-P(OEt)₂" (Beispiel 53b) und (dA)9 jeweils allein und gemischt im Verhältnis 1:1, 1:2, 1:5 und 1:10 auf ein nicht-denaturierendes Polyacrylamid-Gel (20%, Laufpuffer 1xTBE, 10mM MgCl₂) aufgetragen und das Laufverhalten bestimmt. Das Ergebnis ist in FIG. 2 zu sehen: (dA)₉ läuft schneller als "5'-HO-T-P(OH)-T-P(OH)-T-P(OH)-T-P(OH)-T-P(OH)-T-P(OH)-T-P(OH)- T-P(OH)-T-P(OEt)₂", in der 1:1-Mischung sind beide nur noch schwach zu sehen, dafür ist eine langsamere Bande entstanden, die einem Komplex zwischen den beiden Komponenten entspricht. In der 2:1 Mischung ist von (dA)₉ nichts mehr zu sehen, dafür ist die neue Bande um so deutlicher. Das gleiche gilt für die 5:1 bzw. 10:1 Mischung, in denen zusätzlich der deutliche Überschuß an "5'-HO- T-P(OH)-T-P(OH)-T-P(OH)-T-P(OH)-T-P(OH)-T-P(OH)-T-P(OH)- T-P(OH)-T-P(OEt)₂" zu erkennen ist.
58) 5'-MMTr-T-P(ONPE)-T-P(OEthyl)₂ [s. auch Beispiel 21, alternative Synthesen]
   a) 8.44 mg (10 *µ*Mol) N-Thymin-1-yl-acetyl-N-(4-methoxytriphenylmethoxy)ethylaminomethanphosphonsäure-(4-nitrophenylethyl)monoester (Triethylammoniumsalz) (Beispiel 15), 3.77 mg (10 *µ*Mol) N-Thymin-1-yl-acetyl-N-(2-hydroxy)ethylaminomethanphosphonsäurediethylester (Beispiel 10) und 64.6 mg (500 *µ*Mol) N-Ethyldiisopropylamin (DIPEA) wurden in 0.3 ml absol. DMF gelöst. Dazu wurden 44.2 mg (100 *µ*Mol) Benzotriazol-yloxy)tris(dimethylamino)phosphoniumhexafluorophosphat (BOP) zugesetzt. Es wurde 24h bei Raumtemp. gerührt. Nach DC (EE/Methanol/TEA 100/20/2; R_{f} = 0.5) ca. 70% Ausbeute.
   b) Analog Beispiel 58a, jedoch mit 30*µ*Mol HATU (O-(7-Azabenzotriazol-1yl)-N,N,N',N'-tertamethyluroniumhexafluorophosphat) anstelle 100 *µ*Mol BOP. Nach DC ca. 65% Ausbeute.
59) N- Thymin-1-yl-acetyl-N-(4-methoxytriphenylmethoxy)ethylaminomethanphosphonsäure-[2-(p-nitrophenyl)ethyl]-[5'-(3'-Lävuloyl-thymidin]diester
   Synthese analog Beispiel 17 aus N-Thymin-1-yl-acetyl-N-(4-methoxytriphenyl-methoxy)ethylaminomethanphosphonsäure-(4-nitrophenylethyl)monoester (Triethylammoniumsalz, Beispiel 15) und 3'-Lävuloyl-thymidin. Zur Reinigung wurde über Kieselgel chromatographiert (DCM/Methanol/TEA 98/2/0.25). Die Ausbeute betrug 46%.
   MS (FAB/LiCl): 1071.4 (M+Li)⁺.
60) N- Thymin-1-yl-acetyl-N-(4-methoxytriphenylmethoxy)ethylaminomethanphosphonsäure-[2-(p-nitrophenyl)ethyl]-[5'-thymidin]diester
   64 mg (0.06 mMol) N- Thymin-1-yl-acetyl-N-(4-methoxytriphenylmethoxy)ethylaminomethanphosphonsäure-[2-(p-nitrophenyl)ethyl]-[5'-(3'-Lävuloyl-thymidin]diester (Beispiel 59) wurden in 0.5 ml Dioxan gelöst. 9 mg (0.23 mMol) NaBH₄ in 0.12 ml Wasser wurden zugegeben und es wurde 20 min bei Raumtemperatur gerührt. Das Lösungsmittel wurde im Vakuum eingedampft, der Rückstand in DCM aufgenommen, mit Wasser extrahiert und getrocknet. Zur Reinigung wurde über Kieselgel chromatographiert (DCM/Methanol/TEA 92/8/0.5). Die Ausbeute betrug 72%.
   MS (FAB/LiCl): 973.4 (M+Li)⁺, 979.4 (M+2Li-H)⁺, 985.4 (M+3Li-2H)⁺.
61) N- Thymin-1-yl-acetyl-N-(4-methoxytriphenylmethoxy)ethylaminomethanphosphonsäure-[2-(p-nitrophenyl)ethyl]-[5'-thymidin-(3'-(cyanoethyl-N,N-diisopropylphosphoramidit)ldiester
   31 mg (0.032 mMol) N- Thymin-1-yl-acetyl-N-(4-methoxytriphenylmethoxy)-ethylaminomethanphosphonsäure-[2-(p-nitrophenyl)ethyl]-[5'-thymidin]diester (Beispiel 60) wurden zweimal mit absol. CH₃CN koevaporiert, in 0.4 ml absol. THF gelöst. Dazu wurden zuerst 12.4 mg (0.096 mMol) Diisopropylethylamin, dann 9.8 mg (0.045 mMol) Cyanoethyl-chloro-diisopropylphosphoramidit zugegeben. Es wurde 3h gerührt, abfiltriert und im Vakuum eingedampft. Die Ausbeute betrug 63%.
   MS (FAB/LiCl): 1173.3 (M+Li)⁺, 1180.4 (M+2Li-H)⁺, 1186.4 (M+3Li-2H)⁺.
62) N-[N9-(06-diphenylcarbamoyl-N2-acetylguanin]acetyl-N-(4-methoxytriphenylmethoxy)ethylaminomethanphosphonsäuredi(2-(p-nitrophenyl)ethyl)ester
   Synthese analog Beispiel 2 aus N-(4-methoxytriphenylmethoxy)ethylaminomethanphosphonsäuredi(2-(p-nitrophenyl)ethyl)ester (Beispiel 1f) und 06-diphenylcarbamoyl-N2-acetylguanin-essigsäure. Zur Reinigung wurde über Kieselgel chromatographiert (EE/TEA 98/2). Ausbeute: 87%
   MS (FAB/LiCl): 1154.8 (M+H)⁺, 1160.7 (M+Li)⁺.
63) N-[N9-(N4-Anisoyladenin]acetyl-N-(4-methoxytriphenylmethoxy)ethylaminomethanphosphonsäuredi(2-(p-nitrophenyl)ethyl)ester
   Synthese analog Beispiel 2 aus N-(4-methoxytriphenylmethoxy)ethylaminomethanphosphonsäuredi(2-(p-nitrophenyl)ethyl)ester (Beispiel 1f) und N4-Anisoyladeninessigsäure. Zur Reinigung wurde über Kieselgel chromatographiert (DCM/Methanol/TEA 95/4/1). Ausbeute: 82%; MS (ES+): 1035.7 (M+H)⁺. ¹H-NMR (200MHz, DMSO, TMS): δ = 2.93 (t, 4H, P-O-CH₂-CH₂-Ar); 3.09 (t, 2H, MMTr-O-CH₂); 3.23-3.75 (m, 4H, P-CH₂ + N-CH₂); 3.75 (s, 3H, OCH₃); 3.87 (s, 3H, OCH₃); 4.08 (dt, 4H, P-O-CH₂); 5.28-5.42 (m, 2H, CO-CH₂); 6.81-8.20 (m, 28H, Ar-H, A-H); 11.00 (s breit, 1H, NH).
64) N-[N9-(N4-Anisoyladenin]acetyl-N-(4-methoxytriphenylmethoxy)ethylaminomethanphosphonsäuremono(2-(p-nitrophenyl)ethyl)ester
   Synthese analog Beispiel 3 aus N-[N9-(N4-Anisoyladenin]acetyl-N-(4-methoxytriphenylmethoxy)ethylaminomethanphosphonsäuredi(2-(p-nitrophenyl)ethyl)ester (Beispiel 63). Zur Reinigung wurde über Kieselgel chromatographiert (EE/Methanol/TEA 65/35/2). Die Ausbeute betrug 52%.
   MS (FAB/LiCl): 874.3 (M+2Li-H)⁺.
65) N- Thymin-1-yl-acetyl-N-(2-methoxy)ethylaminomethanphosphonsäuredi(2-(p-nitrophenyl)ethyl)ester
   Synthese analog Beispiel 2 aus N-(2-methoxy)ethylaminomethanphosphonsäuredi(2-(p-nitrophenyl)ethyl)ester (hergestellt analog Beispiel I ausgehend von 2-Methoxyethylamin durch Umsetzung mit Formaldehyd und Di(2-(4-nitrophenyl)ethyl)phosphit) und Tymidin-1-yl-essigsäure. Zur Reinigung wurde über Kieselgel chromatographiert (EE/Methanol 90/10). Ausbeute: 64%
   MS (FAB/LiCl): 640.3 (M+Li)⁺.
66) 5'-MMTr-C^{An}-P(ONPE)- C^{A}n-P(ONPE)(OAllyl)
   Synthese analog Beispiel 17 aus N-(N⁶-Anisoyl)cytosin-1-yl-acetyl-N-(4-methoxytriphenylmethoxy)ethylaminomethanphosphonsäure (2-(p-nitrophenyl)ethyl)monoester (Triethylammoniumsalz) (Beispiel 3) und N-(N⁶-Anisoyl)cytosin-1-yl-acetyl-N-(2-hydroxy)ethylaminomethanphosphonsäure-allyl-(2-(p-nitrophenyl)ethyl)diester (Beispiel 34). Zur Reinigung wurde über Kieselgel chromatographiert (EE/Methanol/TEA 85/14/1). Ausbeute: 36%.
   MS (FAB): 1474 (M+H)⁺; 1496 (M+Na)⁺.
67) 5'-HO-C^{An}-P(ONP_{E)-C}^{An}-P(ONPE)(_{OAllyl)}
   Synthese analog Beispiel 4 aus "5'-MMTr-C^{An}-P(ONPE)-C^{A}n-P(ONPE)(OAllyl)" (Beispiel 66). Zur Reinigung wurde über Kieselgel chromatographiert (EE/Methanol/ TEA 80/19/1). Die Ausbeute betrug 55%.
   MS (FAB): 1201.3 (M+H)⁺, 1223.3 (M+Na)⁺.
68) 5'-MMTr- C^{An}-P(ONPE)-C^{A}n-P(ONPE)-C^{A}n-P(ONPE)(OAllyl)
   Synthese analog Beispiel 17 aus N-(N⁶-Anisoyl)cytosin-_{1-yl-acetyl-N-(4-} methoxytriphenylmethoxy)ethylaminomethanphosphonsäure (2-(p-nitrophenyl)ethyl)monoester (Triethylammoniumsalz) (Beispiel 3) und 5'-HO-C^{An}-P(ONPE)- C^{A}n-P(ONPE)(OAllyl) (Beispiel 67). Zur Reinigung wurde über Kieselgel chromatographiert (EE/Methanol/TEA 80/20/1). Ausbeute: 58%.
   MS (FAB): 2046 (M+H)⁺; 2068 (M+Na)⁺.
69) 5'-MMTr- C^{An}-P(ONPE)-C^{A}n-P(ONPE)-C^{A}n-P(ONPE)(OH)
   Synthese analog Beispiel 42 aus 5'-MMTr- C^{An}-P(ONPE)-C^{A}n-P(ONPE)-C^{A}n-P(ONPE)(OAllyl) (Beispiel 68). Zur Reinigung wurde über Kieselgel chromatographiert (EE/Methanol/TEA 60/38/2). Ausbeute: 66%.
   MS (FAB): 2027 (M+Na)⁺; 2049 (M+2Na-H)⁺.
70) 5'-MMTr- C^{An}-P(ONPE)-C^{A}n-P(ONPE)-C^{A}n-P(ONPE)-C^{A}n-P(ONPE)-C^{A}n-P(ONPE)-C^{An}-P(ONPE)₂
   Synthese analog Beispiel 17 aus "5'-MMTr- C^{An}-P(ONPE)-C^{A}n-P(ONPE)-C^{A}n-P(ONPE)(OH)" (Beispiel 69) und "5'-HO-C^{An}-P(ONPE)-C^{A}n-P(ONPE)-C^{A}n-P(ONPE)₂" (Beispiel 32). Zur Reinigung wurde über Kieselgel chromatographiert (EE/Methanol/TEA 70/30/2). Ausbeute: 58%.
   MS (FAB): 3892 (M+Na)⁺; 3914 (M+2Na-H)⁺.
71) 5'-MMTr-T-P(ONPE)-T-P(ONPE)-T-P(ONPE)-C^{An}-P(ONPE)-C^{A}n-P(ONPE)-C^{An}-P(ONPE)₂
   Synthese analog Beispiel 17 aus "5'-MMTr-T-P(ONPE)-T-P(ONPE)-T-P(ONPE)(OH)" (Beispiel 45) und "5'-HO-C^{An}-P(ONPE)-C^{A}n-P(ONPE)-C^{A}n-P(ONPE)₂" (Beispiel 32). Zur Reinigung wurde über Kieselgel chromatographiert (EE/Methanol/TEA 60/40/2). Die Produktfraktion wurde im Vakuum eingedampft und durch präparative HPLC (High Pressure Liquid Chromatography) gereinigt: RP8 LiChrospher60, Wasser/Acetonitril/ : 1/1; 0.1% Ammoniumacetat; 1 ml/min) R_{f}= 16.6 min
   MS (FAB): 3534 (M+Na)⁺; 3556 (M+2Na-H)⁺.
72) 5'-MMTr-T-P(OH)-T-P(OH)-T-P(OH)-C^{An}-P(OH)-C^{A}n-P(OH)-C^{A}n-P(OH)₂
   Synthese analog Beispiel 40 aus "5'-MMTr-T-P(ONPE)-T-P(ONPE)-T-P(ONPE)-CAn-P(ONPE)-CAn-P(ONPE)-CAn-P(ONPE)₂" (2 mg) (Beispiel 71).
   MS (ES-): 2466.4 (M-H).
73) 5'-MMTr-T-P(OH)-T-P(OH)-T-P(OH)-C-P(OH)-C-P(OH)-C-P(OH)₂
   Ca. 1mg "5'-MMTr-T-P(OH)-T-P(OH)-T-P(OH)-CAn-P(OH)-CAn-P(OH)-CAn-P(OH)₂" (Beispiel 72) wurden mit 3 ml 33% wässrigem NH₄OH versetzt und 24 h bei Raumtemperatur gerührt. Das Reaktionsgemisch wurde im Vakuum eingedampft. Ausbeute: ca. 0.6 mg (19 OD)
   MS (ES-): 2064.5 (M-H)⁻.
74) 5'-HO-T-P(OH)-T-P(OH)-T-P(OH)-C-P(OH)-C-P(OH)-C-P(OH)₂
   8 OD von "5'-MMTr-T-P(OH)-T-P(OH)-T-P(OH)-C-P(OH)-C-P(OH)-C-P(OH)₂" (Beispiel 73) wurden in 0.5 ml Wasser gelöst und auf eine PolyPak™ (Glen Research, #60-1100-10) aufgegeben. Die MMTr-Gruppe wurde anhand der Angaben des Herstellers (Glen Research User Guide) abgespalten. Ausbeute: ca. 0.35 mg (11 OD).
   MS (ES-): 1792.6 ((M-H)-.
75) 5'-MMTr- C^{An}-P(ONPE)-C^{A}n-P(ONPE)-C^{A}n- P(ONPE)- T-P(ONPE)-T-P(ONPE)-T-P(ONPE)₂
   Synthese analog Beispiel 17 aus "5'-MMTr- C^{An}-P(ONP_{E)-C}^{An}-P(ONP_{E)-C}^{An}-P(ONPE)(OH)" (Beispiel 69) und "5'-HO-T-P(ONPE)-T-P(ONPE)-T-P(OEt)₂" (Beispiel 26). Zur Reinigung wurde über Kieselgel chromatographiert (EE/Methanol/TEA 80/20/2, dann 70/30/2). Die Produktfraktion wurde mit Toluol koevaporiert, im Vakuum eingedampft und mit Pentan verrieben. Ausbeute: 48%.
   MS (FAB): 3744 (M+Na)⁺; 3766 (M+2Na-H)⁺.

## Patentansprüche

1. Verbindungen der Formel I worin 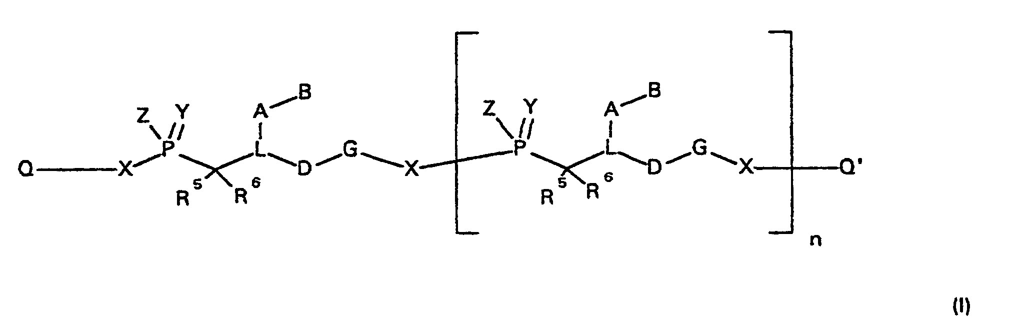
n eine Zahl von Null bis 100 bedeutet;
B unabhängig voneinander für eine natürliche Nucleobasen, eine unnatürliche Nucleobase oder einen Reporter Liganden steht;
A-B kann auch für eine über die Carboxylgruppe aufkondensierte D- oder L-Aminosäure oder für Peptide bestehend aus diesen Aminosäuren mit bis zu einer Länge von 5 Aminosäureresten stehen,
L unabhängig voneinander N oder R¹N⁺, und
R¹ für Wasserstoff oder (C₁-C₆)-Alkyl steht, das mit Hydroxy, (C₁-C₆)-Alkoxy, (C₁-C₆)-Alkylthio oder Amino substituiert sein kann, bevorzugt Wasserstoff oder Methyl bedeutet;
A unabhängig voneinander eine Einfachbindung, eine Methylengruppe oder eine Gruppe der Formel IIa oder IIb bedeutet; 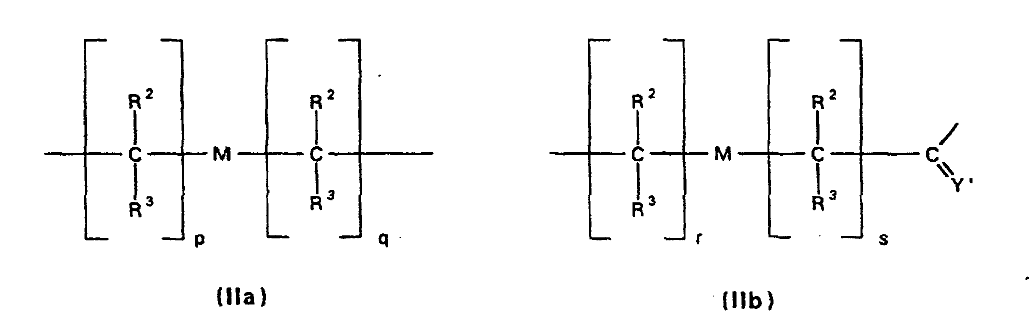
Y' für =O, =S, =CH₂, =C(CH₃)₂ oder =NR¹ steht, wobei R¹ wie oben definiert ist;
M für eine Einfachbindung, -O-, -S- oder -NR¹- steht, wobei R¹ wie oben definiert ist;
R² und R³ unabhängig voneinander für Wasserstoff, Hydroxy, (C₁-C₆)-Alkoxy, (C₁-C₆)-Alkylthio, Amino, Halogen, wie F, Cl, Br oder (C₁-C₆)-Alkyl steht, welches gegebenenfalls mit Hydroxy, (C₁-C₆)-Alkoxy oder (C₁-C₆)-Alkylthio substituiert sein kann;
p und q unabhängig voneinander für Null bis 5 stehen;
r und s unabhängig voneinander für Null bis 5 stehen;
D und G unabhängig voneinander für CR⁵R⁶ stehen;
R⁵ und R⁶ unabhängig voneinander Wasserstoff, (C₁-C₆)-Alkyl, (C₆-C₂₀)-Aryl, (C₆-C₂₀)-Aryl-(C₁-C₆)-alkyl, Hydroxy, (C₁-C₆)-Alkoxy, (C₁-C₆)-Alkylthio bedeuten, und Alkyl und Aryl gegebenenfalls mit SR¹ oder NR¹R^{1'} substituiert sein kann, wobei R¹ wie oben definiert ist und R^{1'} unabhängig von R¹ die gleiche Bedeutung wie R¹ hat;
X für -O-, -S- oder -NR¹-, worin R¹ wie oben definiert ist, steht;
Y für =O oder =S steht;
Z für -OR⁸, -NR⁹R¹⁰ oder X'Q" steht, wobei X' wie X und Q" wie Q definiert sind;
R⁸ Wasserstoff, (C₁-C₁₈)-Alkyl, (C₂-C₁₈)-Alkenyl, (C₃-C₁₈)-Alkinyl, (C₆-C₁₂)-Aryl, (C₆-C₁₂)-Aryl-(C₁-C₆)-alkyl bedeutet, wobei Alkyl ein oder mehrfach mit Hydroxy, (C₁-C₄)-Alkoxy, F, Cl, Br substituiert sein kann und Aryl 1-3-fach mit Hydroxy, (C₁-C₄)-Alkoxy, (C₁-C₄)-Alkyl, F, Cl, Br, NO₂,-NR⁹R¹⁰, -C(O)OH, -C(O)O-(C₁-C₆)-Alkyl, -C(O)NR⁹R¹⁰, substituiert sein kann, bevorzugt jedoch für Wasserstoff, (C₁-C₆)-Alkyl, (C₆-C₁₂)-Aryl oder (C₆-C₁₂)-Aryl-(C₁-C₆)-alkyl steht, wobei Aryl einfach mit (C₁-C₄)-Alkoxy, (C₁-C₄)-Alkyl, F, Cl, Br, NO₂, substituiert sein kann, besonders bevorzugt Wasserstoff, (C₁-C₆)-Alkyl, Phenyl oder 2-(4-Nitrophenyl)ethyl bedeutet;
R⁹ und R¹⁰ unabhängig voneinander für Wasserstoff, (C₁-C₁₈)-Alkyl, (C₁-C₁₈)-Alkenyl, (C₁-C₁₈)-Alkinyl, (C₆-C₁₂)-Aryl, (C₆-C₁₂)-Aryl-(C₁-C₆)-alkyl stehen, wobei Alkyl ein oder mehrfach mit Hydroxy, (C₁-C₄)-Alkoxy, F, Cl, Br substituiert sein kann, oder R⁹ und R¹⁰ können zusammen mit dem sie tragenden N-Atom einen 4-7-gliedrigen Ring bilden;
Q und Q' unabhängig voneinander Wasserstoff oder R⁸ bedeuten, für Konjugate stehen, welche die Eigenschaften von Antisense-Oligonucleotiden oder von Tripelhelix bildenden Oligonucleotiden günstig beeinflußen oder als Markierung einer DNA Sonde dienen oder bei der Hybridisierung des Oligonucleotidanalogons an die Target-Nucleinsäure diese unter Bindung oder Quervernetzung angreift, oder Oligonucleotide bedeuten, die unmodifiziert oder modifiziert sein können.

2. Verbindungen der Formel I gemäß Anspruch 1, worin
n eine Zahl von Null bis 50 bedeutet;
B unabhängig voneinander für eine natürliche Nucleobase oder eine unnatürliche Nucleobase steht;
L N bedeutet;
A eine Gruppe der Formel IIb bedeutet, worin r = 1 und s Null, und R², R³ = H und Y' =O und M eine Einfachbindung bedeuten;
D und G unabhängig voneinander CHR⁵ bedeuten;
R⁵ für Wasserstoff steht;
X -O- bedeutet;
Y =O bedeutet;
Z für Hydroxy, Methoxy, Ethoxy, (4-Nitrophenol)ethoxy, Propoxy, isoPropoxy, Butoxy, Pentoxy, Phenoxy oder Allyloxy steht;
Q und Q' unabhängig voneinander für Wasserstoff, R⁸ oder für Oligonucleotide stehen, die unmodifiziert und modifiziert sein können, wobei
a) die 3'- und/oder 5'-Phosphorsäurediesterbrücken vollständig oder teilweise durch Phosphorothioat-, Phoshorodithioat-, NR⁴R^{4'-}Phosphoramidat-, Phosphat-O-Methylester-, Phosphat-O-ethylester-, Phosphat-O-isopropylester-, Methylphosphonat- oder Phenylphosphonat-Brücken ersetzt sind;
b) ein, zwei oder drei 3'- oder 5-Phosphorsäurediesterbrücken an den Pyrimidin-Positionen und am 5'-Ende und/oder am 3'-Ende durch Formacetale und/oder 3'-Thioformacetale ersetzt sind;
c) das Zuckerphosphat-Rückgrats vollständiger oder teilweise durch "PNAs" oder PNA-DNA-Hybride ersetzt ist;
d) die β-D-2'-Desoxyriboseeinheiten vollständig oder teilweise durch 2'-F-2'-Desoxyribose, 2'-O-(C₁-C₆)Alkyl-Ribose, 2'-O-(C₂-C₆)Alkenyl-Ribose, 2'-NH₂-2'-desoxyribose ersetzt sind;
e) die natürlichen Nucleosid-Basen vollständig oder teilweise durch 5-(C₁-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5-Bromcytosin, 7-Deaza-7-(C₂-C₇)-alkinylguanin, 7-Deaza-7-(C₂-C₇)-alkinyladenin, 7-Deaza-7-(C₂-C₇)-alkenylguanin, 7-Deaza-7-(C₂-C₇)-alkenyladenin, 7-Deaza-7-(C₁₋C₇)-alkylguanin, 7-Deaza-7-(C₁-C₇)-alkyladenin, 7-Deaza-7-bromguanin, 7-Deaza-7-bromadenin ersetzt sind.

3. Verbindungen der Formel I gemäß den Ansprüchen 1 oder 2, **dadurch gekennzeichnet, daß**
n eine Zahl von 0 bis 30 bedeutet;
Q und Q' unabhängig voneinander für Wasserstoff, R⁸, worin R⁸ für H, (C₁-C₆)-Alkyl, Phenyl oder 2-(4-Nitrophenylethyl) steht,oder für Oligonucleotide stehen, die unmodifiziert und modifiziert sein können, wobei
a) die 3'- und/oder 5'-Phosphorsäurediesterbrücken vollständig oder teilweise durch Phosphorothioat-, Phosphorodithioat- oder Methylphosphonat-Brücken ersetzt sind;
b) ein, zwei oder drei 3'- oder 5-Phosphorsäurediesterbrücken am 5'- und am 3'-Ende ersetzt sind;
c) das Zuckerphosphat-Rückgrats vollständiger oder teilweise durch "PNAs" oder PNA-DNA-Hybride ersetzt ist;
d) die β-D-2'-Desoxyriboseeinheiten vollständig oder teilweise durch 2'-F-2'-Desoxyribose, 2'-O-(C₁-C₄)Alkyl-Ribose, 2'-O-(C₂-C₄)Alkenyl-Ribose, 2'-NH₂-2'-desoxyribose ersetzt sind;
e) die natürlichen Nucleosid-Basen vollständig oder teilweise durch 5-(C₃-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 7-Deaza-7-(C₂-C₇)-alkinylguanin, 7-Deaza-7-(C₂-C₇)-alkinyladenin, 7-Deaza-7-(C₂-C₇)-alkenylguanin, 7-Deaza-7-(C₂-C₇)-alkenyladenin, 7-Deaza-7-(C₁₋C₇)-alkylguanin, 7-Deaza-7-(C₁-C₇)-alkyladenin, 7-Deaza-7-bromguanin, 7-Deaza-7-bromadenin ersetzt sind.

4. Verbindungen der Formel I gemäß den Ansprüchen 1 bis 3, **dadurch gekennzeichnet, daß**
n eine Zahl von 0 bis 25 bedeutet;
B unabhängig voneinander für eine natürliche Nucleobase steht;
Z für Hydroxy, Ethoxy, (4-Nitrophenol)ethoxy oder Phenoxy steht;
Q und Q' unabhängig voneinander für Wasserstoff, R⁸, worin R⁸ für H, (C₁-C₆)-Alkyl, Phenyl oder 2-(4-Nitrophenylethyl) steht, oder für Oligonucleotide stehen, die unmodifiziert und modifiziert sein können, wobei
a) die 3'- und/oder 5'-Phosphorsäurediesterbrücken vollständig oder teilweise durch Phosphorothioat-Brücken ersetzt sind;
c) das Zuckerphosphat-Rückgrats vollständiger oder teilweise durch "PNAs" oder PNA-DNA-Hybride ersetzt ist;
d) die β-D-2'-Desoxyriboseeinheiten vollständig oder teilweise durch 2'-O-Methyl-, 2'-O-Allyl-, 2'-O-Butylribose ersetzt sind;
e) die natürlichen Nucleosid-Basen vollständig oder teilweise durch 5-Hexinylcytosin, 5-Hexinyluracil, 5-Hexinylcytosin, 7-Deaza-7-propinylguanin, 7-Deaza-7-propinyladenin, 7-Deaza-7-methylguanin, 7-Deaza-7-methyladenin, Deaza-7-bromguanin, 7-Deaza-7-bromadenin ersetzt sind.

5. Verfahren zur Herstellung von Verbindungen der Formel I, gemäß den Ansprüchen 1 - 4, **dadurch gekennzeichnet, daß** man
a₁) Verbindungen der Formel III 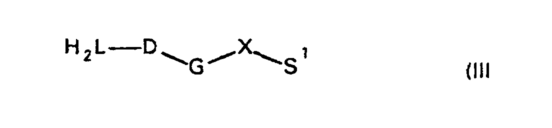 worin
D, G, L und X wie in den Ansprüchen 1 - 4 definiert sind und
S¹ für eine geeignete Schutzgruppe steht, wie beispielsweise Dimethoxytrityl, Monomethoxytrityl, Trityl, Pixyl, tert.-Butyloxycarbonyl oder Fluorenylmethyloxycarbonyl,
mit Verbindungen der Formel IV 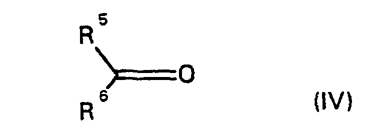 worin
R⁵ und R⁶ wie in den Ansprüchen 1 - 4 definiert sind,
in einem geeigneten organischen Lösungsmittel, bei Temperaturen von 0 °C bis 100 °C, umsetzt zu Verbindungen der Formel Va oder Vb 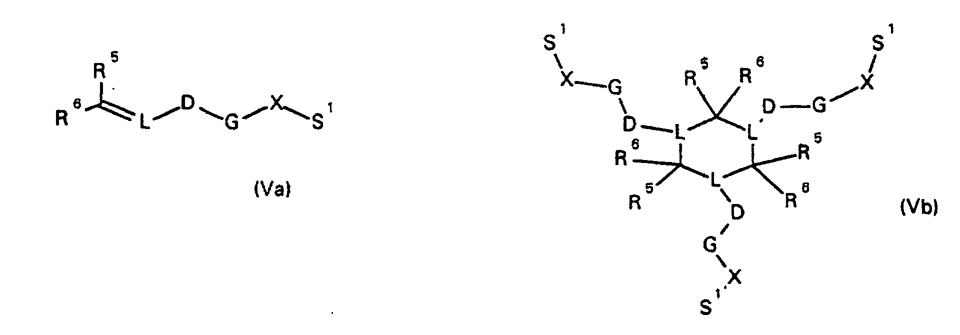
b₁) Verbindungen der Formel Va oder Vb mit Verbindungen der Formel Vla oder VIb 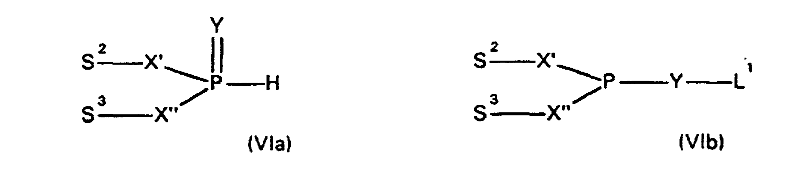 worin
Y wie in den Ansprüchen 1 - 4 definiert ist,
X' und X" unabhängig voneinander wie X definiert sind,
S² und S³ unabhängig voneinander Schutzgruppen bedeuten, und
L¹ für eine Abgangsgruppe, steht,
in einem geeigneten organischen Lösungsmittel, bei Temperaturen von 0 °C bis 100 °C, gegebenenfalls unter Zusatz von Basen, komplexen Basen oder ungeladenen, peralkylierten Polyamino-Phosphazen-Basen umsetzt zu **Verbindungen der Formel VII** 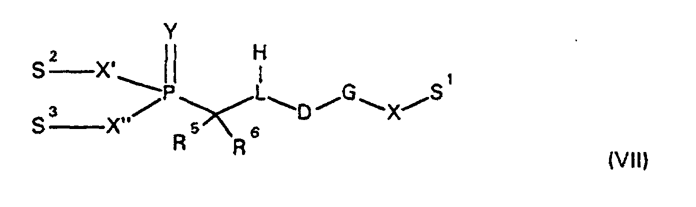 worin D, G, L, R⁵, R⁶, S¹, S², S³, X, X', X" und Y wie in den Ansprüchen 1 - 4 definiert sind;
c₁) Verbindungen der Formel VII mit Verbindungen der Formel VIII, 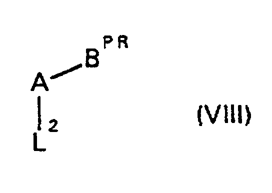 worin
A wie in den Ansprüchen 1 - 4 definiert ist,
B^{PR} die gleiche Bedeutung wie B hat, gegebenenfalls jedoch in geschützter Form vorliegt, und
L² für eine dem Fachmann bekannte Abgangsgruppe steht oder, falls A die Bedeutung von Formel IIb hat auch für OH stehen kann;
in einem geeigneten organischen Lösungsmittel, bei Temperaturen von -20°C bis 100 °C, gegebenenfalls unter Zusatz von Basen, komplexen Basen oder ungeladenen, peralkylierten Polyamino-Phosphazen-Basen oder ohne Basenzusatz und unter Zusatz eines zur Knüpfung von Peptid-Bindungen üblichen Kopplungsreagenzes, umsetzt zu Verbindungen der Formel IX 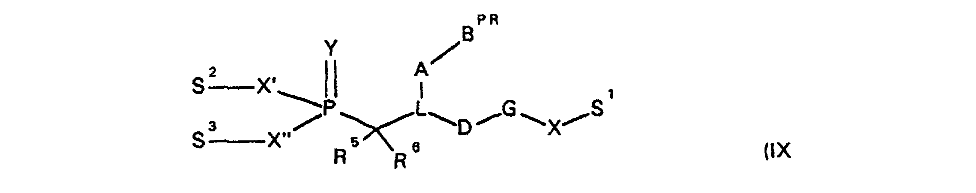 worin
A, B^{PR}, D, G, L, R⁵, R⁶, S¹, S², S³, X, X', X" und Y wie oben definiert sind;
d₁) aus Verbindungen der Formel IX die Schutzgruppe S³ nach bekannten Verfahren abspaltet, wobei man Verbindungen der Formel X erhält 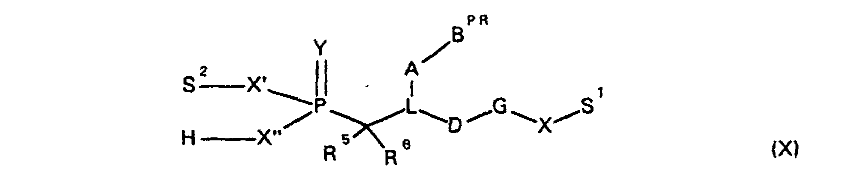 worin
A, B^{PR}, D, G, L, R⁵, R⁶, S¹, S², X, X', X" und Y wie oben definiert sind;
e₁) aus Verbindungen der Formel IX die Schutzgruppe S¹ nach bekannten Verfahren, wobei man Verbindungen der Formel Xl erhält 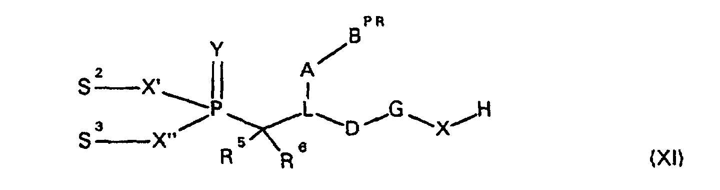 worin A, B^{PR}, D, G, L, R⁵, R⁶, S², S³, X, X', X" und Y wie oben definiert sind;
f₁) Verbindungen der Formel XI mit Verbindungen der Formel X gemäß dem aus der Oligonucleotid-Chemie bekannten "Phosphotriester-Verfahren" in einem geeigneten organischen Lösungsmittel, bei Temperaturen von -20°C bis 100 °C, unter Zusatz eines Kopplungsreagenzes oder einer Verbindung der Formel XII 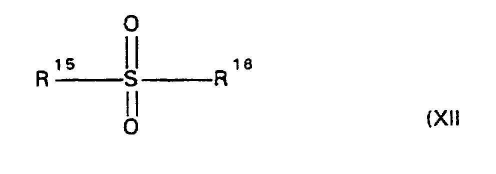 worin
R¹⁵ für (C₆-C₁₂)-Aryl, gegebenenfalls ein bis vierfach substituiert durch (C₁-C₆)-Alkyl, (C₁-C₆)-Alkoxy, Nitro, Chlor, Brom und wobei gegebenenfalls ein bis 3 C-Atome durch Heteroatome substituiert sind, und
R¹⁶ für eine Abgangsgruppe steht,
gegebenenfalls unter Zusatz eines Katalysators, wobei die Herstellung der Kopplungsreagenzien in situ erfolgen kann, oder aber separat erfolgen und die Lösung der aktivierten Spezies in einem geeigneten Lösungsmittel zugegeben werden kann,
zu Verbindungen der Formel XIII umsetzt, 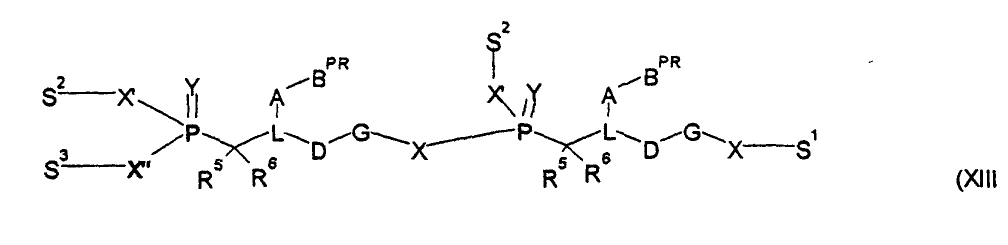 worin A, B^{PR}, D, G, L, R⁵, R⁶, S¹, S², S³, X, X', X" und Y wie oben definiert sind;
g₁) ausgehend von Verbindungen der Formel XIII die Schritte e₁) und f₁) bis zur gewünschten Kettenlänge wiederholt wobei Verbindungen der Formel XIV resultieren, 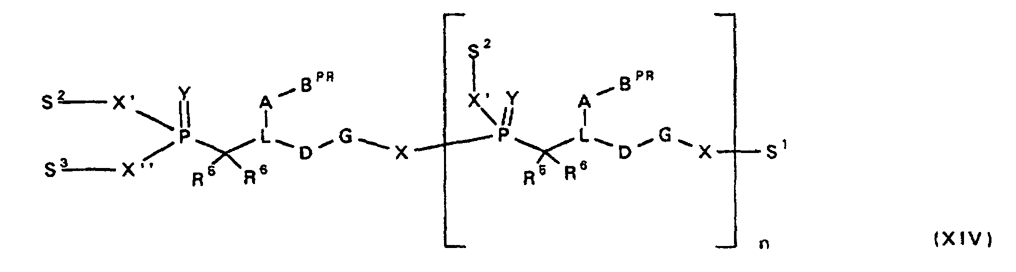 worin
A, B^{PR}, D, G, L, R⁵, R⁶, S¹, S², S³, X, X', X" und Y wie oben definiert sind und a wie in den Ansprüchen 1 - 4 definiert ist;
h₁) die Schutzgruppen S¹, S², und S³ und die Schutzgruppen an B^{PR} nach bekannten Verfahren abspaltet; und gegebenenfalls die Gruppen Q und Q' nach dem Fachmann bekannten Verfahren einführt, und gegebenenfalls die erhaltenen Verbindungen cyclisiert, wodurch Verbindungen der Formel I resultieren.

6. Verfahren zur Herstellung der Verbindungen der Formel I gemäß den Ansprüchen 1 bis 4, worin n 1 bis 100 bedeutet, **dadurch gekennzeichnet ist, daß** man in den Verbindungen der Formeln XV und XVI, 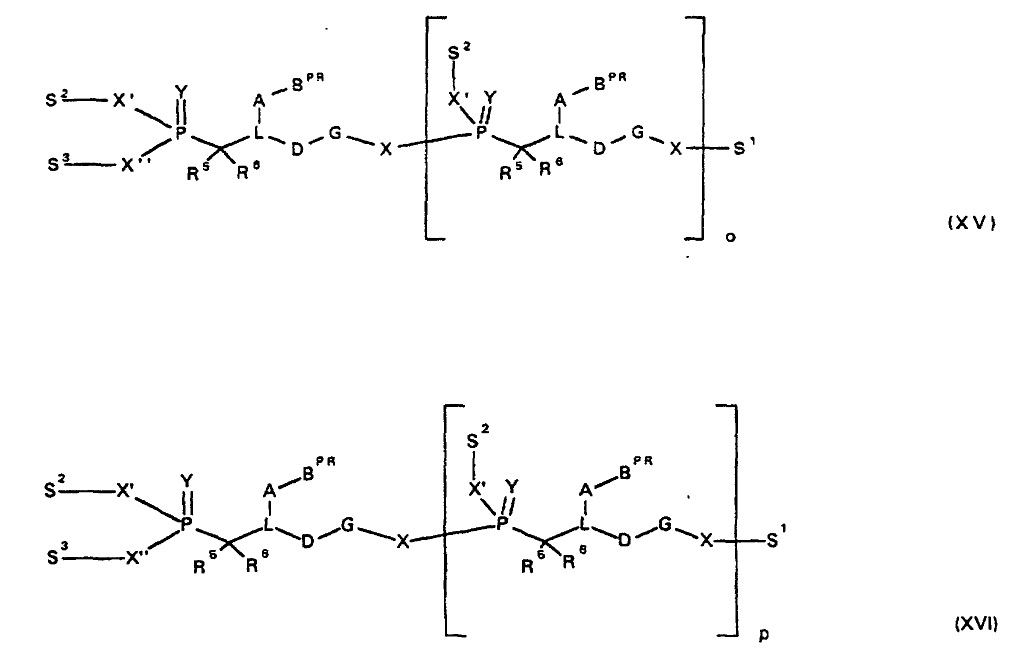 worin
A, B^{PR}, D, G, L, R⁵, R⁶, S¹, S², S³, X, X', X" und Y wie in den Ansprüchen 1-4 definiert sind,
o und p unabhängig voneinander Null bis 50 und o+p+1=n bedeuten;
a₂) in den Verbindungen der Formel XV die Schutzgruppe S¹ wie in Anspruch 5 unter e₁) beschrieben abspaltet,
b₂) in den Verbindungen der Formel XVI die Schutzgruppe S³ wie in Anspruch 5 unter d₁) beschrieben abspaltet und
c₂) die entstehenden Verbindungen wie in Anspruch 5 unter f₁) beschrieben miteinander koppelt, wobei Verbindungen der Formel XIV resultieren, 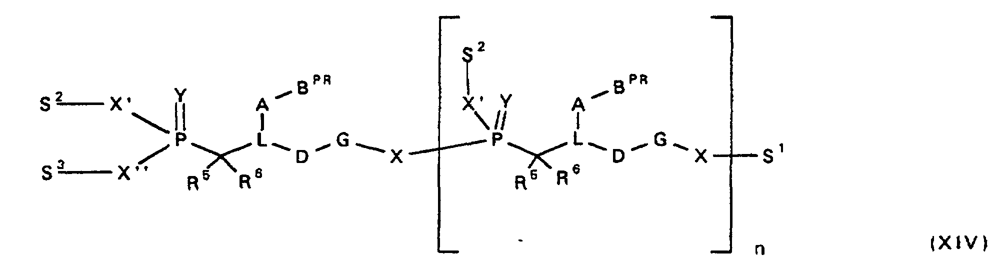 worin
A, B^{PR}, D, G, L, R⁵, R⁶, S¹, S², S³, X, X', X", Y und n wie oben definiert sind,
d₂) und diese wie in Anspruch 5 unter h₁) beschrieben zu Verbindungen der Formel I umsetzt.

7. Verfahren zur Herstellung der Verbindungen der Formel I gemäß den Ansprüchen 1 bis 4, **dadurch gekennzeichnet, daß** man
a₃) Verbindungen der Formel X, 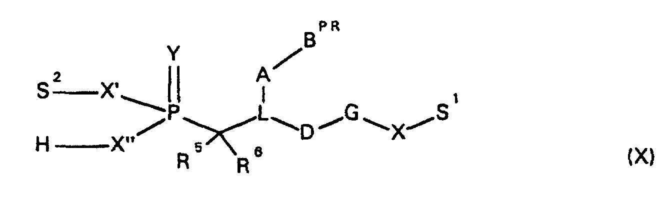 worin
A, B^{PR}, D, G, L, R⁵, R⁶, S¹, S², X, X', X" und Y wie in den Ansprüchen 1 - 4 definiert sind, nach bekannten Verfahren über einen SPACER an einen festen Träger koppelt, zu Verbindungen der Formel XVII, 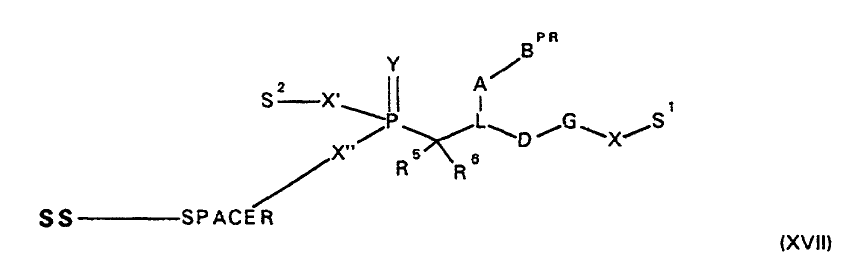 worin
A, B^{PR}, D, G, L, R⁵, R⁶, S¹, S², X, X', X" und Y wie oben definiert sind,
SS für einen zur Festphasensynthese geeigneten festen Träger steht, und
SPACER für eine vom Träger nach erfolgter Synthese abspaltbare Gruppe steht, wie sie dem Fachmann bekannt sind, oder SPACER für bisfunktionelle Konjugatmoleküle Q, die über bekannte abspaltbare Gruppen an den festen Träger geknüpft werden, steht;
b₃) aus Verbindungen der Formel XVII, 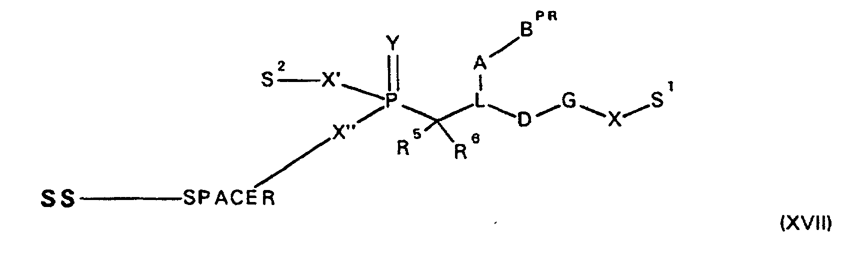 worin
A, B^{PR}, D, G, L, R⁵, R⁶, S¹, S², SS, SPACER, X, X', X" und Y wie oben definiert sind,
die Schutzgruppe S¹ wie in Anspruch 5 unter e₁) beschrieben abspaltet;
c₃) die resultierende Verbindung mit Verbindungen der Formel X, 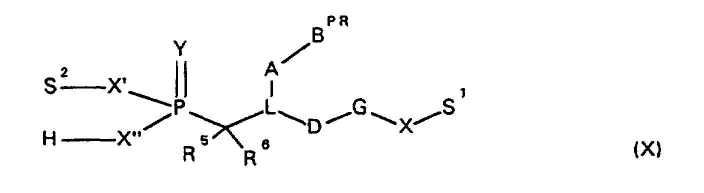 worin
A, B^{PR}, D, G, L, R⁵, R⁶, S¹, S², X, X', X" und Y wie oben definiert sind, wie in Anspruch 5 unter f₁) beschrieben umsetzt;
d₃) die Schritte b₃) und c₃) bis zur gewünschten Kettenlänge wiederholt;
e₃) gegebenenfalls Konjugate Q' durch bekannte Verfahren aufkoppelt;
f₃) die so erzeugten Verbindungen nach bekannten Verfahren vom festen Träger abspaltet, und die Schutzgruppen wie in Schritt h₁) beschrieben, wobei die Abspaltung der Schutzgruppen auch vor der Spaltung vom Träger erfolgen kann, und gegebenenfalls Konjugate Q durch bekannte Verfahren aufkoppelt, wobei die Reihenfolge der Aufkopplung von Q bis Q' (e₃), f₃)) auch geändert werden kann, und gegebenenfalls die erhaltenen Verbindung cyclisiert.

8. Verwendung der Verbindungen der Formel I gemäß den Ansprüchen 1 bis 4 als Inhibitoren der Genexpression.

9. Verwendung der Verbindungen der Formel I gemäß den Ansprüchen 1 bis 4 als Diagnostikum, zur Behandlung von Erkrankungen, die durch Viren hervorgerufen, durch Integrine oder Zell-Zell-Adhäsionsrezeptoren beinflußt oder durch Faktoren wie TNF alpha ausgelöst werden, zur Behandlung von Krebs oder der Restenose.

10. Eine Verbindung folgender Formeln
5'-OLIGO-PMENA;
5'-PMENA-OLIGO;
5'-OLIGO-PMENA-OLIGO;
5'-OLIGO-(PMENA-OLIGO)ₐ;
5'-PMENA-OLIGO-PMENA; oder
5'-PMENA-(OLIGO-PMENA)ₐ
worin a 1-20 ist, OLIGO für ein gegebenenfalls modifiziertes Oligonucleotid steht und PMENA eine Verbindung der Formel I gemäß den Ansprüchen 1-4, worin Q und Q' Wasserstoff ist, bedeutet.

## Claims

1. A compound of the formula I 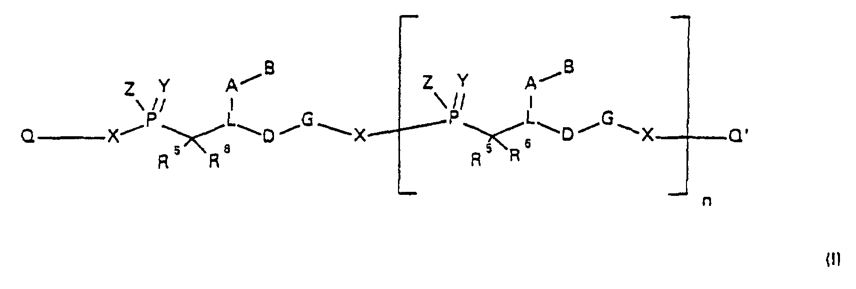 in which n is a number from zero to 100;
B independently of one another is a natural nucleobase, an unnatural nucleobase or a reporter ligand;
A-B can also be a D- or L-amino acid condensed on via the carboxyl group or peptides consisting of these amino acids having a length of up to 5 amino acid esters,
L independently of one another is N or R¹N⁺, and
R¹ is hydrogen or (C₁-C₆)-alkyl which can be substituted by hydroxyl, (C₁-C₆)-alkoxy, (C₁-C₆)-alkylthio or amino, preferably hydrogen or methyl;
A independently of one another is a single bond, a methylene group or a group of the formula IIa or IIb; 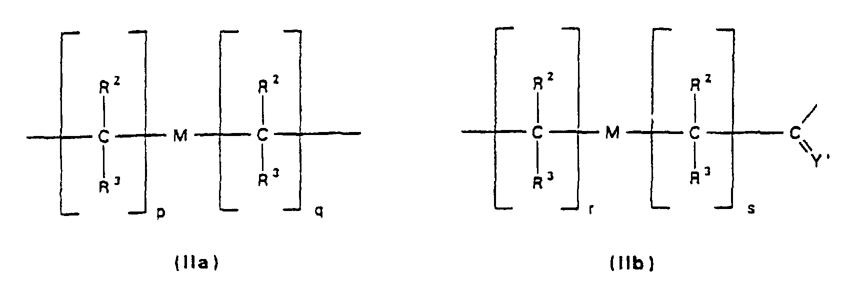
Y' is =O, =S, =CH₂; =C(CH₃)₂ or =NR¹, where R¹ is as defined above;
M is a single bond, -O-, -S- or -NR¹-, where R¹ is as defined above;
R² and R³ independently of one another are hydrogen, hydroxyl, (C₁-C₆)-alkoxy, (C₁-C₆)-alkylthio, amino, halogen, such as F, Cl or Br, or (C₁-C₆)-alkyl which can optionally be substituted by hydroxyl, (C₁-C₆)-alkoxy or (C₁-C₆)-alkylthio;
p and q independently of one another are zero to 5;
r and s independently of one another are zero to 5;
D and G independently of one another are CR⁵R⁶;
R⁵ and R⁶ independently of one another are hydrogen, (C₁-C₆)-alkyl, (C₆-C₂₀)-aryl, (C₆-C₂₀)-aryl-(C₁-C₆)-alkyl, hydroxyl, (C₁-C₆)-alkoxy, (C₁-C₆)-alkylthio, and alkyl and aryl can optionally be substituted by SR¹ or NR¹R^{1'}, where R¹ is as defined above and R^{1'} independently of R¹ has the same meaning as R¹;
X is -O-, -S- or -NR¹-, in which R¹ is as defined above;
Y is =O or =S;
Z is -OR⁸, -NR⁹R¹⁰ or X'Q", where X' is defined as X and Q" is defined as Q;
R⁸ is hydrogen, (C₁-C₁₈)-alkyl, (C₂-C₁₈)-alkenyl, (C₃-C₁₈)-alkynyl, (C₆-C₁₂)-aryl, (C₆-C₁₂)-aryl-(C₁-C₆)-alkyl, where alkyl can be substituted one or more times by hydroxyl, (C₁-C₄)-alkoxy, F, Cl or Br and aryl can be substituted 1-3 times by hydroxyl, (C₁-C₄)-alkoxy, (C₁-C₄)-alkyl, F, Cl, Br, NO₂, -NR⁹R¹⁰, -C(O)OH, -C(O)O-(C₁-C₆)-alkyl or -C(O)NR⁹R¹⁰, but preferably is hydrogen, (C₁-C₆)-alkyl, (C₆-C₁₂)-aryl or (C₆-C₁₂)-aryl-(C₁-C₆)-alkyl, where aryl can be monosubstituted by (C₁-C₄)-alkoxy, (C₁-C₄)-alkyl, F, Cl, Br or NO₂, and is particularly preferably hydrogen, (C₁-C₆)-alkyl, phenyl or 2-(4-nitrophenyl)ethyl;
R⁹ and R¹⁰ independently of one another are hydrogen, (C₁-C₁₈)-alkyl, (C₁-C₁₈) -alkenyl, (C₁-C₁₈)-alkynyl, (C₆-C₁₂) -aryl, (C₆-C₁₂) -aryl- (C₁-C₆) -alkyl, where alkyl can be substituted one or more times by hydroxyl, (C₁-C₄) -alkoxy, F, Cl or Br, or R⁹ and R¹⁰ can together form a 4 to 7-membered ring together with the N atom carrying them;
Q and Q' independently of one another are hydrogen or R⁸, or conjugates which favorably affect the properties of antisense oligonucleotides or of triple helix-forming oligonucleotides or serve as a label of a DNA probe or in the hybridization of the oligonucleotide analog to the target nucleic acid attack this with binding or crosslinking, or are oligonucleotides which can be unmodified or modified.

2. A compound of the formula I as claimed in claim 1, in which
n is a number from zero to 50;
B independently of one another is a natural nucleobase or an unnatural nucleobase;
L is N;
A is a group of the formula IIb, in which r = 1 and s is zero, and R², R³ = H and Y' = O and M is a single bond;
D and G independently of one another are CHR⁵;
R⁵ is hydrogen;
X is -O-;
Y is =O;
Z is hydroxyl, methoxy, ethoxy, (4-nitrophenyl)ethoxy, propoxy, isopropoxy, butoxy, pentoxy, phenoxy or allyloxy;
Q and Q' independently of one another are hydrogen, R³ or oligonucleotides which can be unmodified or modified, where
a) the 3'- and/or 5'-phosphoric acid diester bridges are completely or partially replaced by phosphorothioate, phosphorodithioate, NR⁴R^{4'}-phosphoramidate, phosphate O-methyl ester, phosphate O-ethyl ester, phosphate O-isopropyl ester, methylphosphonate or phenylphosphonate bridges;
b) one, two or three 3'- or 5'-phosphoric acid diester bridges in the pyrimidine positions and at the 5' end and/or at the 3' end are replaced by formacetals and/or 3'-thioformacetals;
c) the sugar phosphate backbone is completely or partially replaced by "PNAs" or PNA-DNA hybrids;
d) the β-D-2'-deoxyribose units are completely or partially replaced by 2'-F-2'-deoxyribose, 2'-0-(C₁-C₆) alkylribose, 2'-O-(C₂-C₆) alkenylribose or 2'-NH₂-2'-deoxyribose;
e) the natural nucleoside bases are completely or partially replaced by 5-(C₁-C₆)-alkyluracil, 5- (C₂-C₆)-alkenyluracil, 5- (C₂-C₆)-alkynyluracil, 5-(C₁-C₆)-alkylcytosine, 5-(C₂-C₆)-alkenylcytosine, 5-(C₂-C₆)-alkynylcytosine, 5-fluorouracil, 5-fluorocytosine, 5-chlorouracil, 5-chlorocytosine, 5-bromouracil, 5-bromocytosine, 7-deaza-7-(C₂-C₇)-alkynylguanine, 7-deaza-7-(C₂-C₇)-alkynyladenine, 7-deaza-7- (C₂-C₇)-alkenylguanine, 7-deaza-7- (C₂-C₇)-alkenyladenine, 7-deaza-7-(C₁-C₇)-alkylguanine, 7-deaza-7-(C₁-C₇)-alkyladenine, 7-deaza-7-bromoguanine, 7-deaza-7-bromoadenine.

3. A compound of the formula I as claimed in claim 1 or 2, wherein
n is a number from 0 to 30;
Q and Q' independently of one another are hydrogen, R⁸, in which R⁸ is H, (C₁-C₆)-alkyl, phenyl or 2-(4-nitrophenyl)ethyl, or are oligonucleotides which can be unmodified or modified, where
a) the 3'- and/or 5'-phosphoric acid diester bridges are completely or partially replaced by phosphorothioate, phosphorodithioate or methylphosphonate bridges;
b) one, two or three 3'- or 5-phosphoric acid diester bridges are replaced at the 5'- and at the 3'-end;
c) the sugar phosphate backbone is completely or partially replaced by "PNAs" or PNA-DNA hybrids;
d) the β-D-2'-deoxyribose units are completely or partially replaced by 2'-F-2'-deoxyribose, 2'-O-(C₁-C₄)alkylribose, 2' -O-(C₂-C₄)alkenylribose or 2'-NH₂-2'-deoxyribose;
e) the natural nucleoside bases are completely or partially replaced by 5-(C₃-C₆)-alkyluracil, 5-(C₂-C₆)-alkenyluracil, 5-(C₂-C₆)-alkynyluracil, 5-(C₁-C₆)-alkylcytosine, 5-(C₂-C₆)-alkenylcytosine, 5-(C₂-C₆)-alkynylcytosine, 7-deaza-7- (C₂-C₇) -alkynyl-guanine, 7-deaza-7- (C₂-C₇) -alkynyladenine, 7-deaza-7- (C₂-C₇) -alkenylguanine, 7-deaza-7- (C₂-C₇) -alkenyladenine, 7-deaza-7- (C₁-C₇) -alkylguanine, 7-deaza-7-(C₁-C₇) -alkyladenine, 7-deaza-7-bromoguanine, 7-deaza-7-bromoadenine.

4. A compound of the formula I as claimed in claims 1 to 3, wherein
n is a number from 0 to 25;
B independently of one another is a natural nucleobase;
z is hydroxyl, ethoxy, (4-nitrophenyl)ethoxy or phenoxy;
Q and Q' independently of one another are hydrogen, R⁸, in which R⁸ is H, (C₁-C₆)-alkyl, phenyl or 2-(4-nitrophenyl)ethyl, or are Oligonucleotides which can be unmodified or modified, where
a) the 3'- and/or 5'-phosphoric acid diester bridges are completely or partially replaced by phosphorothioate bridges;
c) the sugar phosphate backbone is completely or partially replaced by "PNAs" or PNA-DNA hybrids;
d) the β-D-2'-deoxyribose units are completely or partially replaced by 2'-O-methyl-, 2'-O-allyl- or 2'-O-butylribose;
e) the natural nucleoside bases are completely or partially replaced by 5-hexynylcytosine, 5-hexynyluracil, 5-hexynylcytosine, 7-deaza-7-propynylguanine, 7-deaza-7-propynyladenine, 7-deaza-7-methylguanine, 7-deaza-7-methyladenine, 7-deaza-7-bromoguanine, 7-deaza-7-bromoadenine.

5. A process for the preparation of compounds of the formula I as claimed in claims 1 - 4, which comprises
a₁) reacting compounds of the formula III 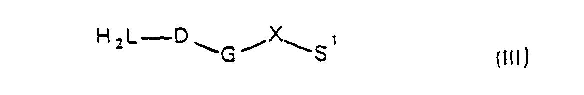 in which
D, G, L and X are as defined in claims 1 - 4 and
S¹ is a suitable protective group, such as dimethoxytrityl, monomethoxytrityl, trityl, pixyl, tert-butyloxycarbonyl or fluorenylmethyloxycarbonyl,
with compounds of the formula IV 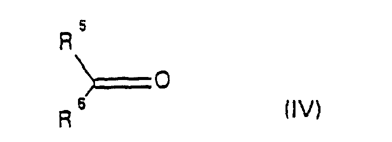 in which
R⁵ and R⁶ are as defined in claims 1 - 4, in a suitable organic solvent, at temperatures from 0°C to 100°C, to give compounds of the formula Va or Vb 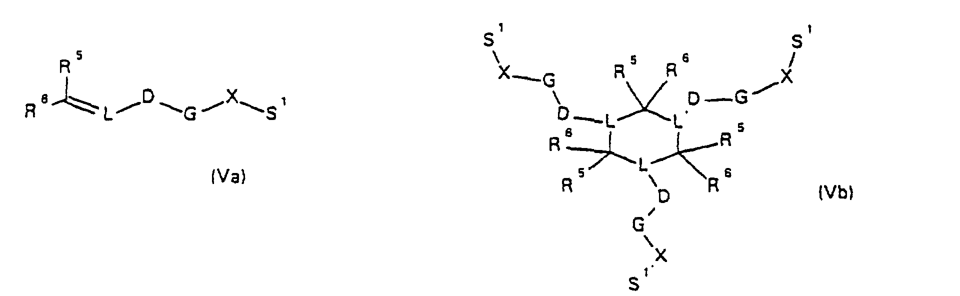
b₁) reacting compounds of the formula Va or Vb with compounds of the formula VIa or VIb 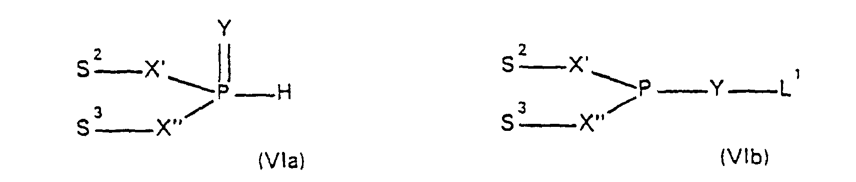 in which
Y is as defined in claims 1 - 4,
X' and X" independently of one another are defined as X,
S² and S³ independently of one another are protective groups, and
L¹ is a leaving group,
in a suitable organic solvent, at temperatures from 0°C to 100°C, if appropriate with addition of bases, complex bases or uncharged, peralkylated polyamino-phosphazene bases, to give compounds of the formula VII 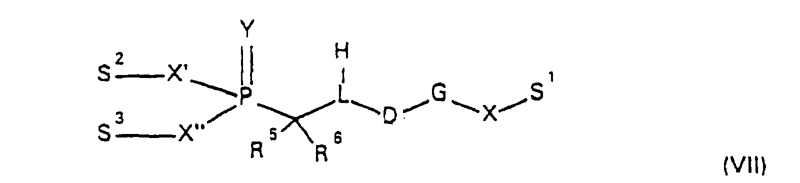 in which
D, G, L, R⁵, R⁶, S¹, S², S³, X, X', X" and Y are as defined in claims 1 - 4;
c₁) reacting compounds of the formula VII with compounds of the formula VIII 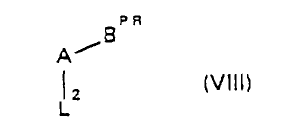 in which
A is as defined in claims 1 - 4,
B^{PR} has the same meaning as B, but is optionally
L² present in protected form, and is a leaving group known to the person skilled in the art, or, if A has the meaning of formula IIb, can also be OH;
in a suitable organic solvent, at temperatures from -20°C to 100°C, if appropriate with addition of bases, complex bases or uncharged, peralkylated polyaminophosphazene bases, or without addition of base and with addition of a coupling reagent customary for the coupling of peptide bonds, to give compounds of the formula IX 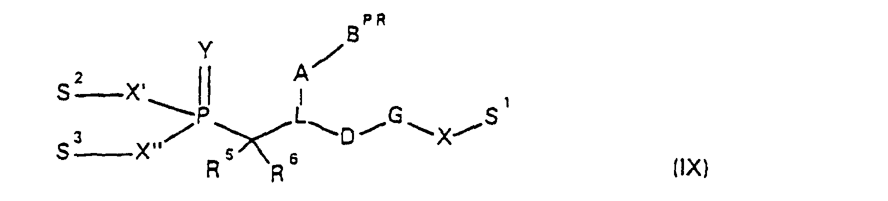 in which
A, B^{PR}, D, G, L, R⁵, R⁶, S¹, S², S³, X, X' , X" and Y are as defined above;
d₁) removing the protective group S³ from compounds of the formula IX by known processes compounds of the formula X being obtained 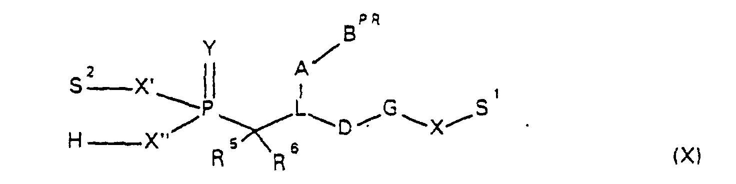 in which
A, B^{PR}, D, G, L, R⁵, R⁶, S¹, S², X', X" and Y are as defined above;
e₁) removing the protective group S¹ from compounds of the formula IX by known processes compounds of the formula XI being obtained 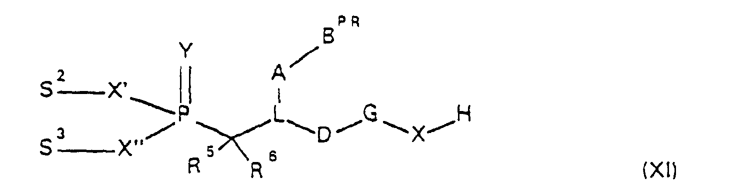 in which
A, B^{PR}, D, G, L, R⁵, R⁶, S², S³, X, X', X" and Y are as defined above;
f₁) reacting compounds of the formula XI with compounds of the formula X according to the "phosphotriester process" known from oligonucleotide chemistry in a suitable organic solvent, at temperatures from -20°C to 100°C, with addition of a coupling reagent or a compound of the formula XII 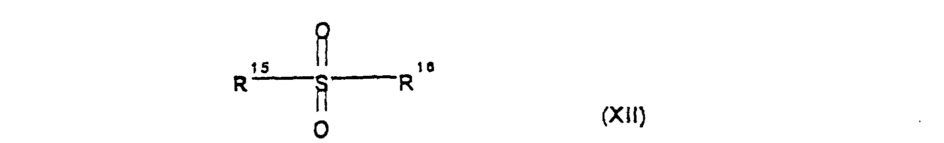 in which
R¹⁵ is (C₆-C₁₂)-aryl, optionally substituted one to four times by (C₁-C₆)-alkyl, (C₁-C₆)-alkoxy, nitro, chlorine or bromine and where one to 3 carbon atoms are optionally substituted by heteroatoms, and
R¹⁶ is a leaving group,
optionally with addition of a catalyst, where the preparation of the coupling reagents can be carried out in situ, or else carried out separately and the solution of the activated species can be added in a suitable solvent,
to give compounds of the formula XIII 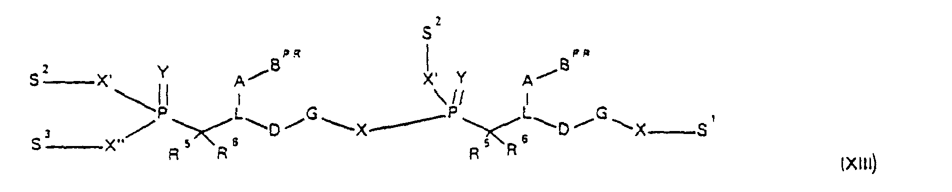 in which
A, B^{PR}, D, G, L, R⁵, R⁶, S¹, S², S³, X, X', X" and Y are as defined above;
g₁) starting from compounds of the formula XIII, repeating the steps e₁) and f₁) up to the desired chain length, compounds of the formula XIV resulting 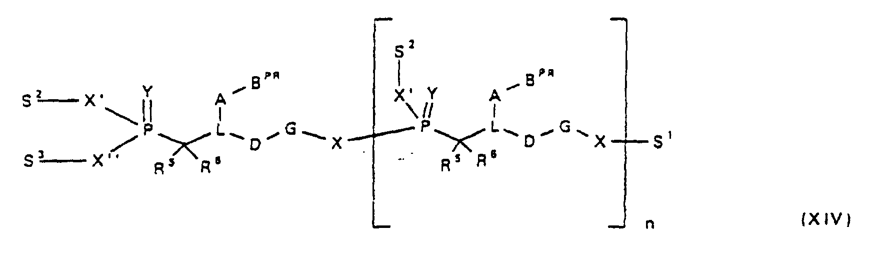 in which
A, B^{PR}, D, G, L, R⁵, R⁶, S¹, S², S³, X, X', X" and Y are as defined above and a is as defined in claims 1 - 4;
h₁) removing the protective groups S¹, S² and S³ and the protective groups on B^{PR} according to known processes;
and optionally introducing the groups Q and Q' according to processes known to the person skilled in the art, and optionally cyclizing the compounds obtained, whereby compounds of the formula I result.

6. A process for the preparation of the compounds of the formula I as claimed in claims 1 to 4, in which n is 1 to 100, which comprises, in the compounds of the formulae XV and XVI, 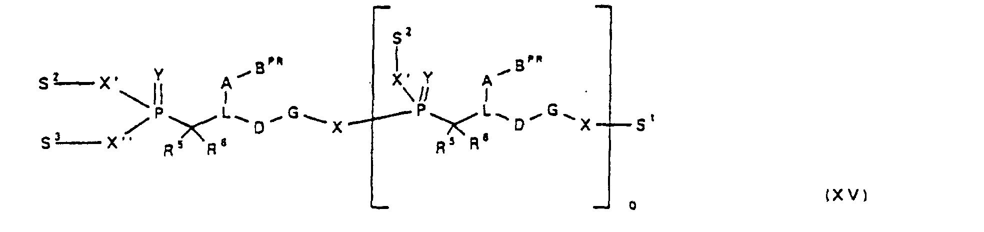 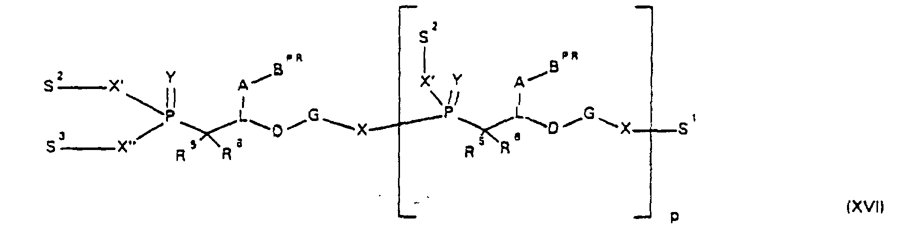 in which
A, B^{PR}, D, G, L, R⁵, R⁶, S¹, S², S³, X, X', X" and Y are as defined in claims 1 - 4,
o and p independently of one another are zero to 50 and o + p + 1 = n;
a₂) in the compounds of the formula XV removing the protective group S¹ as described in claim 5 under e₁),
b₂) in the compounds of the formula XVI removing the protective group S³ as described in claim 5 under d₁) and
c₂) coupling the resulting compounds with one another as described in claim 5 under f₁), compounds of the formula XIV resulting 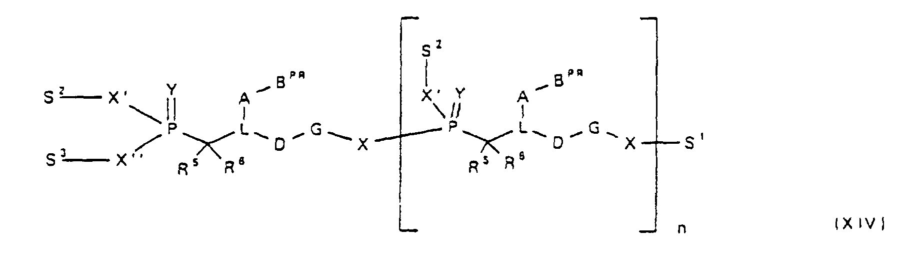 in which
A, B^{PR}, D, G, L, R⁵, R⁶, S¹, S², S³, X, X', X", Y and n are as defined above,
d₂) and reacting these as described in claim 5 under h₁) to give compounds of the formula I.

7. A process for the preparation of the compounds of the formula I as claimed in claims 1 to 4, which comprises
a₃) coupling compounds of the formula X 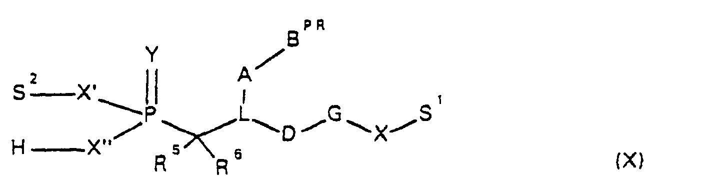 in which
A, B^{PR}, D, G, L, R⁵, R⁶, S¹, S², X, X', X" and Y are as defined in claims 1 - 4,
to a solid support via a SPACER according to known processes, to give compounds of the formula XVII, 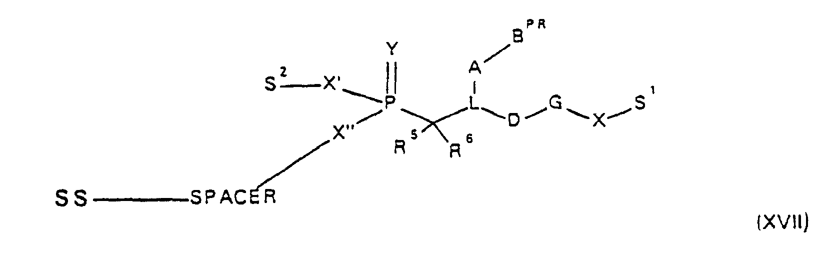 in which
A, B^{PR}, D, G, L, R⁵, R⁶, S¹, S², X, X', X" and Y are as defined above,
SS is a solid support suitable for solid-phase synthesis, and
SPACER is a group removable from the support after synthesis has taken place, such as is known to the person skilled in the art, or SPACER is bifunctional conjugate molecules Q, which are linked to the solid support via known removable groups;
b₃) removing the protective group S¹ from compounds of the formula XVII 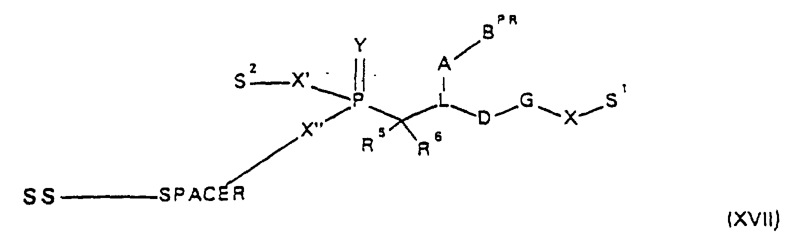 in which
A, B^{PR}, D, G, L, R⁵, R⁶, S¹, S², SS, SPACER, X, X',
X" and Y are as defined above,
as described in claim 5 under e₁);
c₃) reacting the resulting compound with compounds of the formula X 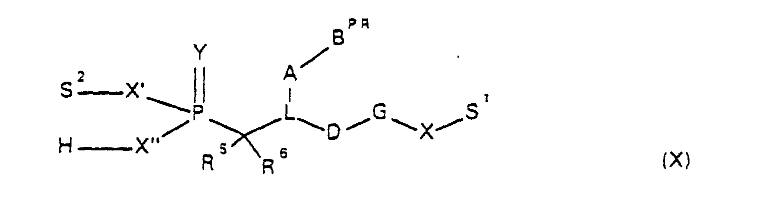 in which
A, B^{PR}, D, G, L, R⁵, R⁶, S¹, S², X, X', X" and Y are as defined above,
as described in claim 5 under f₁);
d₃) repeating the steps b₃) and C₃) up to the desired chain length;
e₃) optionally coupling conjugates Q' by known processes;
f₃) removing the compounds produced in this way from the solid support by known processes, and the protective groups as described in step h₁), where the removal of the protective groups can also be carried out before the cleavage from the support, and optionally coupling by conjugates Q by known processes, where the sequence of the coupling of Q to Q' (e₃), f₃)) can also be changed, and optionally cyclizing the compound obtained.

8. The use of the compounds of the formula I as claimed in claims 1 to 4 as inhibitors of gene expression.

9. The use of the compounds of the formula I as claimed in claims 1 to 4 as a diagnostic, for the treatment of illnesses which are caused by viruses, affected by integrins or cell-cell adhesion receptors or induced by factors such as TNF alpha, for the treatment of cancer or restenosis.

10. A compound of the following formulae:
5'-OLIGO-PMENA;
5'-PMENA-OLIGO;
5'-OLIGO-PMENA-OLIGO;
5'-OLIGO-(PMENA-OLIGO)ₐ;
5'-PMENA-OLIGO-PMENA; or
5'-PMENA-(OLIGO-PMENA)ₐ
in which a is 1 - 20, CLIGO is an optionally modified oligonucleotide, and PMENA is a compound of the formula I as claimed in claims 1 - 4, in which Q and Q' are hydrogen.

## Revendications

1. Composés de formule I 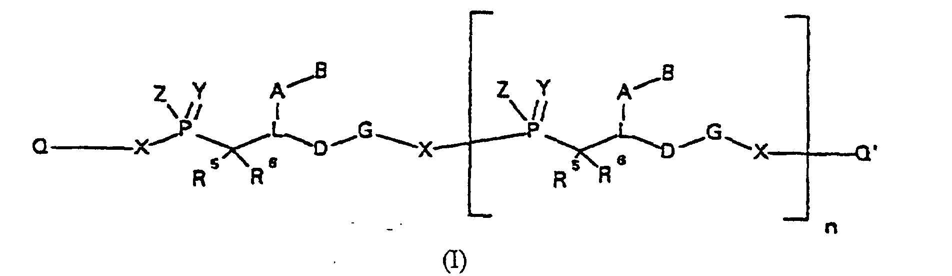 dans laquelle
n représente un nombre de 0 à 100;
les B représentent, indépendamment les uns des autres, une base nucléique naturelle, une base nucléique non naturelle ou un ligand reporter;
A-B peut aussi représenter un D- ou L-aminoacide ou un peptide constitué de ces aminoacides ayant une longueur allant jusqu'à 5 aminoacides, condensé par l'intermédiaire du groupe carboxyle,
les L sont indépendamment les uns des autres N ou R¹N⁺, et
R¹ représente un atome d'hydrogène ou un groupe alkyle en C₁-C₆ pouvant être substitué par hydroxy, alcoxy en C₁-C₆, alkylthio en C₁-C₆ ou amino, de préférence un atome d'hydrogène ou un groupe méthyle;
les A représentent, indépendamment les uns des autres, une liaison simple, un groupe méthylène ou un groupe de formule IIa ou IIb 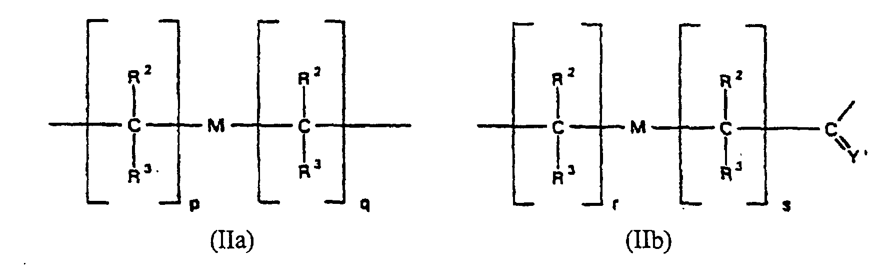
Y' représente =O, =S, =CH₂, =C(CH₃)₂ ou =NR¹, R¹ ayant la définition donnée ci-dessus;
M représente une liaison simple, -O-, -S- ou -NR¹-, R¹ ayant la définition donnée ci-dessus;
R² et R³ représentent, indépendamment l'un de l'autre, un atome d'hydrogène ou un groupe hydroxy, alcoxy en C₁-C₆, alkylthio en C₁-C₆, amino, halogéno comme F, Cl ou Br, ou alkyle en C₁-C₆ qui peut éventuellement étre substitué par hydroxy, alcoxy en C₁-C₆ ou alkylthio en C₁-C₆;
p et q, , indépendamment l'un de l'autre, représentent un nombre de 0 à 5;
r et s, indépendamment l'un de l'autre, représentent un nombre de 0 à 5;
D et G, indépendamment l'un de l'autre, représentent un groupe CR⁵R⁶;
R⁵ et R⁶, indépendamment l'un de l'autre, représentent un atome d'hydrogène ou un groupe alkyle en C₁-C₆, aryle en C₆-C₂₀, (aryl en C₆-C₂₀)(alkyle en C₁-C₆), hydroxy, alcoxy en C₁-C₆, alkylthio en C₁-C₆, et les groupes alkyle et aryle peuvent éventuellement être substitués par SR¹ ou NR¹R^{1'}, R¹ ayant la définition donnée ci-dessus et R^{1'} ayant, indépendamment de R¹, la même signification que R¹;
X représente -O-, -S- ou -NR¹-, R¹ ayant la définition donnée ci-dessus;
Y représente =O ou =S;
Z représente un groupe -OR⁸, -NR⁹R¹⁰ ou X'Q", X' ayant la même définition que X et Q" la même définition que Q;
R⁸ représente un atome d'hydrogène ou un groupe alkyle en C₁-C₁₈, alcényle en C₂-C₁₈, alcynyle en C₃-C₁₈, aryle en C₆-C₁₂, (aryl en C₆-C₁₂)(alkyle en C₁-C₆), le groupe alkyle pouvant être substitué une ou plusieurs fois par hydroxy, alcoxy en C₁-C₄, F, Cl, Br, et le groupe aryle pouvant être substitué 1 à 3 fois par hydroxy, alcoxy en C₁-C₄, alkyle en C₁-C₄, F, Cl, Br, NO₂, -NR⁹R¹⁰, -C(O)OH, -C(O)O-(alkyle en C₁-C₆), -C(O)NR⁹R¹⁰, mais représente de préférence un atome d'hydrogène ou un groupe alkyle en C₁-C₆, aryle en C₆-C₁₂ ou (aryl en C₆-C₁₂)(alkyle en C₁-C₆), le groupe aryle pouvant être monosubstitué par un groupe alcoxy en C₁-C₄, alkyle en C₁-C₄, F, Cl, Br ou NO₂, et représente de façon particulièrement préférée un atome d'hydrogène ou un groupe alkyle en C₁-C₆, phényle ou 2-(4-nitrophényl)éthyle;
R⁹ et R¹⁰ représentent, indépendamment l'un de l'autre, un atome d'hydrogène ou un groupe alkyle en C₁-C₁₈, alcényle en C₁-C₁₈, alcynyle en C₁-C₁₈, aryle en C₆-C₁₂, (aryl en C₆-C₁₂)(alkyle en C₁-C₆), le groupe alkyle pouvant être substitué une ou plusieurs fois par hydroxy, alcoxy en C₁-C₄, F, Cl, Br, ou R⁹ et R¹⁰ peuvent former ensemble, avec l'atome d'azote qui les porte, un cycle de 4 à 7 chaînons;
Q et Q' représentent, indépendamment l'un de l'autre, un atome d'hydrogène ou R⁸, ou représentent des conjugats qui ont un effet favorable sur les propriétés d'oligonucléotides antisens ou d'oligonucléotides formant une triple hélice, ou qui servent de marqueur d'une sonde ADN ou qui, lors de l'hybridation de l'analogue d'oligonucléotide à l'acide nucléique cible, forment avec celui-ci une liaison ou une réticulation transversale, ou représentent des oligonucléotides qui peuvent être non modifiés ou modifiés.

2. Composés de formule 1 selon la revendication 1, dans lesquels
n représente un nombre de 0 à 50;
les B représentent, indépendamment les uns des autres, une base nucléique naturelle ou une base nucléique non naturelle;
L représente N;
A représente un groupe de formule IIb dans laquelle r = 1 et s = 0, R² et R³ = H; Y' est =O et M représente une liaison simple;
D et G, indépendamment l'un de l'autre, représentent un groupe CHR⁵;
R⁵ représente un atome d'hydrogène;
X représente -O-;
Y représente =O;
Z représente un groupe hydroxy, méthoxy, éthoxy, (4-nitrophényl)éthoxy, propoxy, isopropoxy, butoxy, pentyloxy, phénoxy ou allyloxy;
Q et Q' représentent, indépendamment l'un de l'autre, un atome d'hydrogène ou R⁸, ou représentent des oligonucléotides qui peuvent être non modifiés ou modifiés, dans lesquels
a) les ponts 3'- et/ou 5'-phosphodiester sont entièrement ou partiellement remplacés par des ponts phosphorothioate, phosphorodithioate, NR⁴R^{4'}-phosphoramidate, phosphate de O-méthyle, phosphate de O-éthyle, phosphate de O-isopropyle, méthylphosphonate ou phénylphosphonate;
b) un, deux ou trois ponts 3'- ou 5'-phosphodiester aux positions de la pyrimidine et à l'extrémité 5' et/ou à l'extrémité 3' sont remplacés par des formacétals et/ou des 3'-thioformacétals;
c) le squelette de phosphates de sucres est remplacé entièrement ou partiellement par des "PNA" ou des hybrides PNA-ADN;
d) les unités de β-D-2'-désoxyribose sont entièrement ou partiellement remplacées par du 2'-F-2'-désoxyribose, du 2'-O-(alkyl en C₁-C₆)-ribose, du 2'-O-(alcényl en C₂-C₆)-ribose, du 2'-NH₂-2'-désoxyribose;
e) les bases de nucléosides naturelles sont entièrement ou partiellement remplacées par un 5-(alkyl en C₁-C₆)uracile, un 5-(alcényl en C₂-C₆)uracile, un 5-(alcynyl en C₂-C₆)uracile, une 5-(alkyl en C₁-C₆)cytosine, une 5-(alcényl en C₂-C₆)cytosine, une 5-(alcynyl en C₂-C₆)cytosine, du 5-fluorouracile, de la 5-fluorocytosine, du 5-chlorouracile, de la 5-chlorocytosine, du 5-bromouracile, de la 5-bromocytosine, une 7-déaza-7-(alcynyl en C₂-C₇)guanine, une 7-déaza-7-(alcynyl en C₂-C₇)adénine, une 7-déaza-7-(alcényl en C₂-C₇)guanine, une 7-déaza-7-(alcényl en C₂-C₇)adénine, une 7-déaza-7-(alkyl en C₁-C₇)guanine, une 7-déaza-7-(alkyl en C₁-C₇)adénine, une 7-déaza-7-bromoguanine, une 7-déaza-7-bromoadénine.

3. Composés de formule 1 selon les revendications 1 ou 2, dans lesquels
n représente un nombre de 0 à 30;
Q et Q' représentent, indépendamment l'un de l'autre, un atome d'hydrogène, R⁸, R⁸ étant un atome d'hydrogène ou un groupe alkyle en C₁-C₆, phényle ou 2-(4-nitrophényl)éthyle, ou des oligonucléotides qui peuvent être non modifiés ou modifiés, dans lesquels
a) les ponts 3'- et/ou 5'-phosphodiester sont entièrement ou partiellement remplacés par des ponts phosphorothioate, phosphorodithioate ou méthylphosphonate;
b) un, deux ou trois ponts 3'- ou 5'-phosphodiester sont remplacés à l'extrémité 5' et à l'extrémité 3';
c) le squelette de phosphates de sucres est remplacé entièrement ou partiellement par des "PNA" ou des hybrides PNA-ADN;
d) les unités de β-D-2'-désoxyribose sont entièrement ou partiellement remplacées par du 2'-F-2'-désoxyribose, du 2'-O-(alkyl en C₁-C₄)-ribose, du 2'-O-(alcényl en C₂-C₄)-ribose, du 2'-NH₂-2'-désoxyribose;
e) les bases de nucléosides naturelles sont entièrement ou partiellement remplacées par un 5-(alkyl en C₃-C₆)uracile, un 5-(alcényl en C₂-C₆)uracile, un 5-(alcynyl en C₂-C₆)uracile, une 5-(alkyl en C₁-C₆)cytosine, une 5-(alcényl en C₂-C₆)cytosine, une 5-(alcynyl en C₂-C₆)cytosine, une 7-déaza-7-(alcynyl en C₂-C₇)guanine, une 7-déaza-7-(alcynyl en C₂-C₇)adénine, une 7-déaza-7-(alcényl en C₂-C₇)guanine, une 7-déaza-7-(alcényl en C₂-C₇)adénine, une 7-déaza-7-(alkyl en C₁-C₇)guanine, une 7-déaza-7-(alkyl en C₁-C₇)adénine, une 7-déaza-7-bromoguanine, une 7-déaza-7-bromoadénine.

4. Composés de formule 1 selon les revendications 1 à 3, dans lesquels
n représente un nombre de 0 à 25;
les B₁ représentent indépendamment les uns des autres une base nucléique naturelle;
Z représente un groupe hydroxy, éthoxy, (4-nitrophényl)éthoxy ou phénoxy;
Q et Q' représentent, indépendamment l'un de l'autre, un atome d'hydrogène, R⁸, R⁸ étant un atome d'hydrogène ou un groupe alkyle en C₁-C₆, phényle ou 2-(4-nitrophényl)éthyle, ou des oligonucléotides qui peuvent être non modifiés ou modifiés, dans lesquels
a) les ponts 3'- et/ou 5'-phosphodiester sont entièrement ou partiellement remplacés par des ponts phosphorothioate;
c) le squelette de phosphates de sucres est remplacé entièrement ou partiellement par des "PNA" ou des hybrides PNA-ADN;
d) les unités de β-D-2'-désoxyribose sont entièrement ou partiellement remplacées par du 2'-O-méthylribose, du 2'-O-allylribose ou du 2'-O-butylribose;
e) les bases de nucléosides naturelles sont entièrement ou partiellement remplacées par une 5-hexynylcytosine, un 5-hexynyluracile, une 7-déaza-7-propynylguanine, une 7-déaza-7-propynyladénine, une 7-déaza-7-méthylguanine, une 7-déaza-7-méthyladénine, une 7-déaza-7-bromoguanine, une 7-déaza-7-bromoadénine.

5. Procédé de préparation de composés de formule I selon les revendications 1 à 4, **caractérisé en ce que**
a₁) on fait réagir des composés de formule III 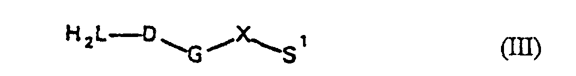 dans laquelle
D, G, L et X ont la définition donnée dans les revendications 1 à 4, et
S¹ représente un groupe protecteur approprié comme, par exemple, un groupe diméthoxytrityle, monométhoxytrityle, trityle, pixyle, tert-butoxycarbonyle ou fluorénylméthyloxycarboxyle,
avec des composés de formule IV 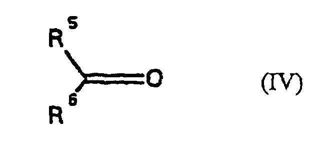 dans laquelle
R⁵ et R⁶ ont la définition donnée dans les revendications 1 à 4, dans un solvant organique approprié, à des températures de 0°C à 100°C, pour obtenir des composés de formule Va ou Vb 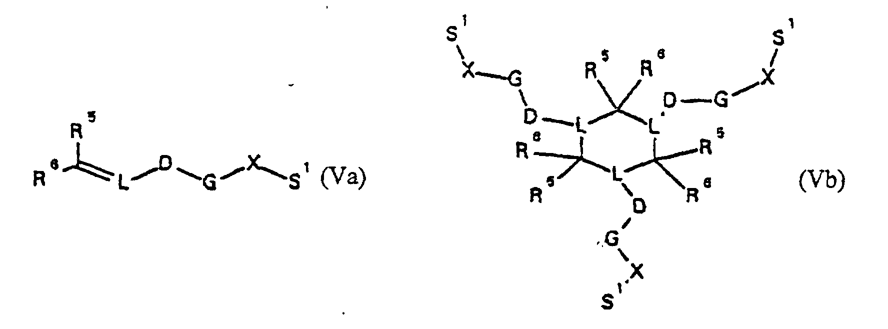
b₁) on fait réagir des composés de formule Va ou Vb avec des composés de formule VIa ou VIb 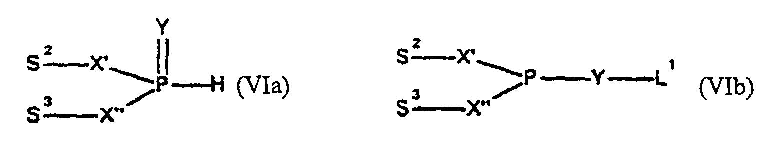 dans lesquelles
Y a la définition donnée dans les revendications 1 à 4,
X' et X" ont, indépendamment l'un de l'autre la signification donnée pour X,
S² et S³ représentent indépendamment l'un de l'autre des groupes protecteurs, et
L¹ représente un groupe partant,
dans un solvant organique approprié, à des températures de 0°C à 100°C, éventuellement en présence de bases, de bases complexes ou de bases de type polyamino-phosphazène peralkylées non chargées pour obtenir des composés de formule VII 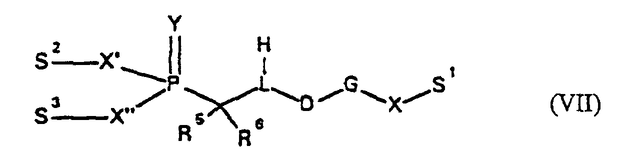 dans laquelle D, G, L, R⁵, R⁶, S¹, S², S³, X, X', X" et Y ont la définition donnée dans les revendications 1 à 4;
c₁) on fait réagir des composés de formule VII avec des composés de formule VIII 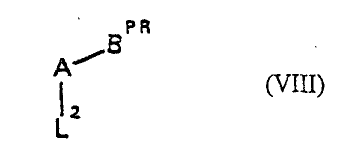 dans laquelle
A a la définition donnée dans les revendications 1 à 4,
B^{PR} a la même signification que B, mais est éventuellement sous forme protégée, et
L² représente un groupe partant connu de l'homme de l'art, ou peut aussi être un
groupe OH lorsque A représente un groupe de formule IIb; dans un solvant organique approprié, à des températures de -20°C à 100°C, éventuellement en présence de bases, de bases complexes ou de bases de type polyamino-phosphazène peralkylées non chargées, ou sans addition de base et en présence d'un réactif de couplage classique pour la formation de liaisons peptidiques, pour obtenir des composés de formule IX 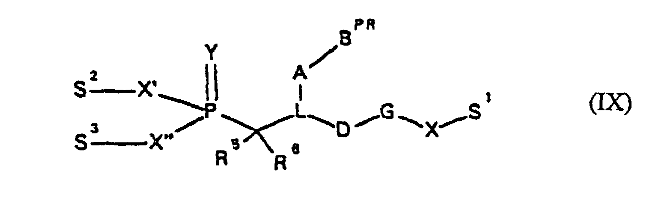 dans laquelle
A, B^{PR}, D, G, L, R⁵, R⁶, S¹, S², S³, X, X', X" et Y ont la définition donnée ci-dessus; d₁) on sépare par des procédés connus le groupe protecteur S³ des composés de formule IX, pour obtenir des composés de formule X 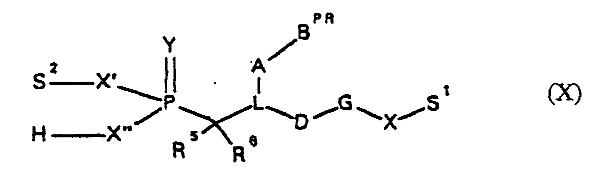 dans laquelle
A, B^{PR}, D, G, L, R⁵, R⁶, S¹, S², X, X', X" et Y ont la définition donnée ci-dessus;
e₁) on sépare par des procédés connus le groupe protecteur S¹ des composés de formule IX, pour obtenir des composés de formule XI 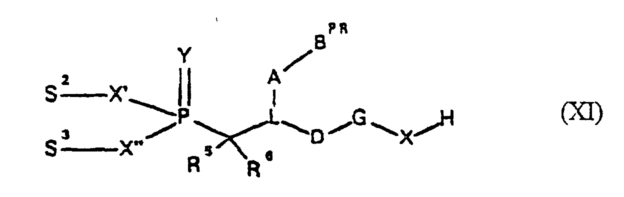 dans laquelle
A, B^{PR}, D, G, L, R⁵, R⁶, S², S³, X, X', X" et Y ont la définition donnée ci-dessus;
f₁) on fait réagir des composés de formule XI avec des composés de formule X selon
le procédé des phosphotriesters connu dans la chimie des oligonucléotides dans un solvant organique approprié, à des températures de -20°C à 100°C, en présence d'un réactif de couplage ou d'un composé de formule XII 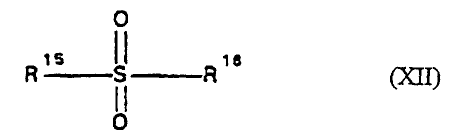 dans laquelle
R¹⁵ représente un groupe aryle en C₆-C₁₂, éventuellement substitué 1 à 4 fois par alkyle en C₁-C₆, alcoxy en C₁-C₆, nitro, chloro, bromo et dans lequel 1 à 3 atomes de carbone sont éventuellement remplacés par des hétéroatomes, et
R¹⁶ représente un groupe partant; éventuellement en présence d'un catalyseur, la préparation des réactifs de couplage pouvant se faire *in situ*, mais aussi séparément, et on peut ajouter la solution des espèces activées dans un solvant approprié, pour obtenir des composés de formule XIII 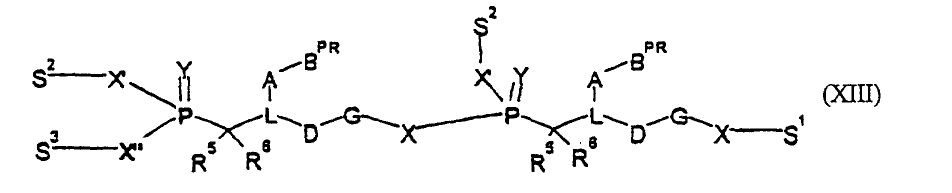 dans laquelle
A, B^{PR}, D, G, L, R⁵, R⁶, S¹, S², S³, X, X', X" et Y ont la définition donnée ci-dessus;
g₁) en partant de composés de formule (XIII), on répète les étapes e₁) et f₁) jusqu'à la longueur de chaîne désirée, en obtenant des composés de formule (XIV) 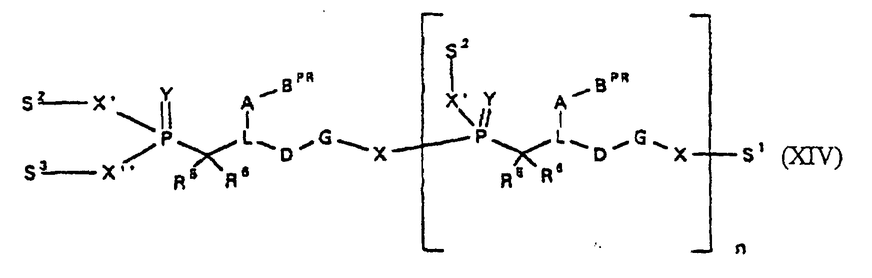 dans laquelle
A, B^{PR}, D, G, L, R⁵, R⁶, S¹, S², S³, X, X', X" et Y ont la définition donnée ci-dessus; et n a la définition donnée dans les revendications 1 à 4;
h₁) on sépare les groupes protecteurs S¹, S² et S³ et les groupes protecteurs de B^{PR} par des procédés connus;
et on introduit éventuellement les groupes Q et Q' par des procédés connus de l'homme de l'art, et on cyclise éventuellement les composés obtenus, grâce à quoi on obtient des composés de formule I.

6. Procédé de préparation de composés de formule I selon les revendications 1 à 4, dans lesquels n est un nombre de 1 à 100, **caractérisé en ce que**, dans les composés de formule XV et XVI 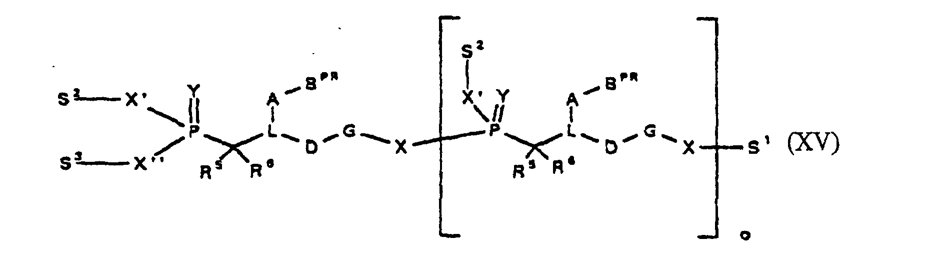 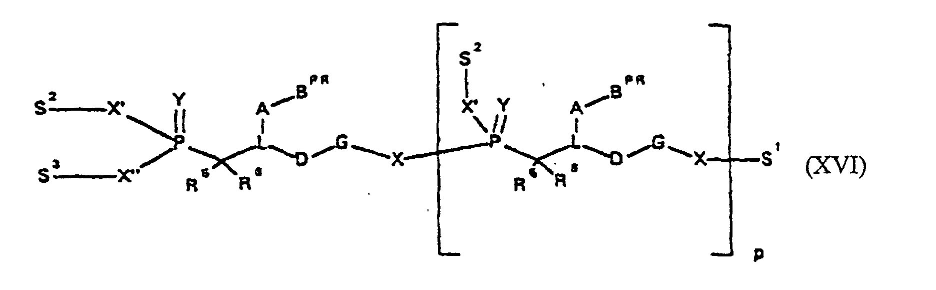 où
A, B^{PR}, D, G, L, R⁵, R⁶, S¹, S², S³, X, X', X" et Y ont la définition donnée dans les revendications 1 à 4,
o et p représentent indépendamment l'un de l'autre un nombre de 1 à 50 et o+p+1 = n;
a₁) on sépare le groupe protecteur S¹ dans les composés de formule XV de la manière décrite dans la revendication 5 sous e₁),
b₂) on sépare le groupe protecteur S³ dans les composés de formule XVI de la manière décrite dans la revendication 5 sous d₁), et
c₂) on couple ensemble les composés formés de la manière décrite dans la revendication 5 sous f₁) pour obtenir des composés de formule XIV 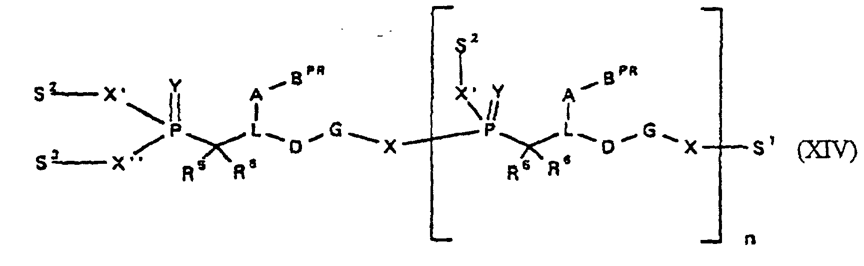 dans laquelle
A, B^{PR}, D, G, L, R⁵, R⁶, S¹, S², S³, X, X', X", Y et n ont la définition donnée ci-dessus,
d₂) et on transforme ces derniers en composés de formule I de la manière décrite dans la revendication 5 sous h₁).

7. Procédé de préparation de composés de formule I selon les revendications 1 à 4, **caractérisé en ce que**
a₃) on couple par des procédés connus des composés de formule X 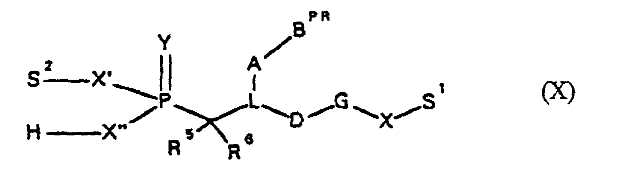 dans laquelle
A, B^{PR}, D, G, L, R⁵, R⁶, S¹, S², X, X', X" et Y ont la définition donnée dans les revendications 1 à 4, à un support solide par l'intermédiaire d'un ESPACEUR, pour obtenir des composés de formule XVII 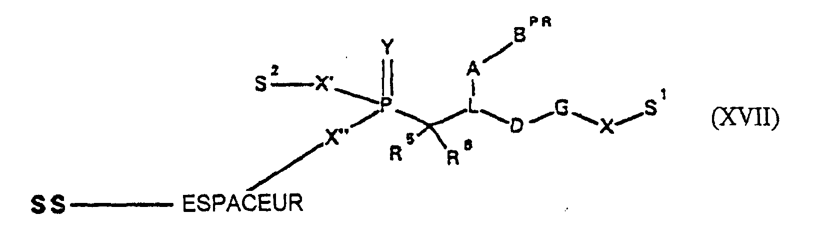 dans laquelle
A, B^{PR}, D, G, L, R⁵, R⁶, S¹, S², X, X', X" et Y ont la définition donnée ci-dessus,
SS représente un support solide approprié pour une synthèse en phase solide, et
ESPACEUR représente un groupe dissociable du support après la fin de la synthèse, de manière connue de l'homme de l'art, ou ESPACEUR représente une molécule de conjugat Q bifonctionnelle qui est liée au support solide par l'intermédiaire d'un groupe dissociable connu;
b₃) on sépare de la manière décrite dans la revendication 5 sous e₁) le groupe protecteur S¹ de composés de formule XVII 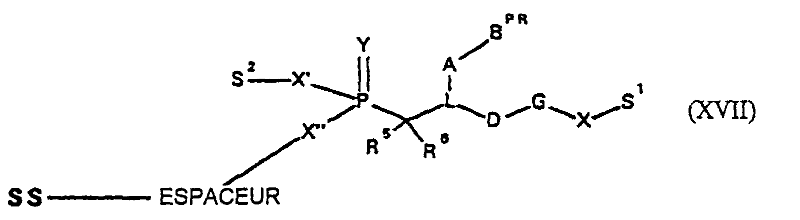 dans laquelle
A, B^{PR}, D, G, L, R⁵, R⁶, S¹, S², SS, ESPACEUR, X, X', X" et Y ont la définition donnée ci-dessus;
c₃) on fait réagir de la manière décrite dans la revendication 5 sous f₁) le composé obtenu avec des composés de formule X 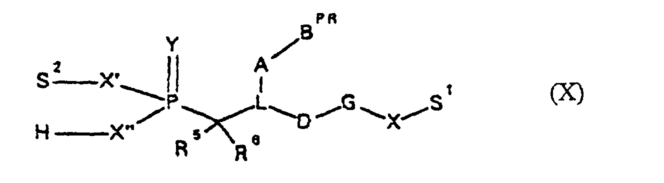 dans laquelle
A, B^{PR}, D, G, L, R⁵, R⁶, S¹, S², X, X', X" et Y ont la définition donnée ci-dessus;
d₃) on répète les étapes b₃) et c₃) jusqu'à la longueur de chaîne désirée;
e₃) on couple éventuellement des conjugats Q' par des procédés connus;
f₃) on sépare par des procédés connus les composés ainsi obtenus du support solide, et on sépare les groupes protecteurs de la manière décrite dans l'étape h₁), la séparation des groupes protecteurs pouvant aussi s'effectuer avant la séparation du support, et on couple éventuellement des conjugats Q par des procédés connus, l'ordre du couplage de Q et Q' [e₃), f₃)] pouvant aussi être modifié, et on cyclise éventuellement le composé obtenu.

8. Utilisation des composés de formule I selon les revendications 1 à 4 comme inhibiteurs de l'expression de gènes.

9. Utilisation des composés de formule I selon les revendications 1 à 4 comme agents de diagnostic, pour le traitement de maladies qui sont provoquées par des virus, influencées par des intégrines ou des récepteurs d'adhésion cellule-cellule, ou déclenchées par des facteurs comme le TNF-α, ou pour le traitement du cancer ou de la resténose.

10. Composé ayant l'une des formules suivantes:
5'-OLIGO-PMENA;
5'-PMENA-OLIGO;
5'-OLIGO-PMENA-OLIGO;
5'-OLIGO-(PEENA-OLIGO)ₐ;
5'-PMENA-OLIGO-PMENA; ou
5'-PEENA-(OLIGO-PEENA)ₐ
dans lesquelles a est un nombre de 1 à 20, OLIGO représente un oligonucléotide éventuellement modifié et PMENA représente un composé de formule I selon les revendications 1 à 4, dans laquelle Q et Q' sont des atomes d'hydrogène.
